(19) Europäisches Patentamt
European Patent Office
Office européen des brevets

(11) EP 4 252 846 A1

(12) EUROPEAN PATENT APPLICATION
published in accordance with Art. 153(4) EPC

(43) Date of publication:
**04.10.2023 Bulletin 2023/40**

(21) Application number: **21889279.2**

(22) Date of filing: **05.11.2021**

(51) International Patent Classification (IPC):
***A61P 21/04*** (2006.01) ***A61P 25/00*** (2006.01)
***A61P 43/00*** (2006.01) ***C12N 15/113*** (2010.01)
***A61K 31/712*** (2006.01) ***A61K 31/7125*** (2006.01)
***A61K 31/713*** (2006.01)

(52) Cooperative Patent Classification (CPC):
**A61K 31/712; A61K 31/7125; A61K 31/713; A61P 21/04; A61P 25/00; A61P 43/00; C12N 15/113**

(86) International application number:
**PCT/JP2021/040853**

(87) International publication number:
**WO 2022/097727 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.11.2020 JP 2020185944**

(71) Applicants:
- **Sumitomo Pharma Co., Ltd.**
  **Osaka 541-0045 (JP)**
- **Luxna Biotech Co., Ltd.**
  **Suita-shi, Osaka 565-0871 (JP)**

(72) Inventors:
- **SUZUKI, Rika**
  **Osaka-shi, Osaka 554-0022 (JP)**
- **ASANO, Shigehiro**
  **Osaka-shi, Osaka 554-0022 (JP)**
- **KURITA, Mitsumasa**
  **Osaka-shi, Osaka 554-0022 (JP)**
- **SUZUKI, Takao**
  **Suita-shi, Osaka 565-0871 (JP)**
- **YAMAGAMI, Masaki**
  **Suita-shi, Osaka 565-0871 (JP)**
- **SHRESTHA, Ajaya Ram**
  **Suita-shi, Osaka 565-0871 (JP)**
- **KAWANOBE, Takaaki**
  **Suita-shi, Osaka 565-0871 (JP)**
- **UMEMOTO, Tadashi**
  **Suita-shi, Osaka 565-0871 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) REGULATOR FOR EXPRESSION AND/OR FUNCTION OF RPS25 GENE

(57) A single-stranded antisense oligonucleotide, or a pharmaceutically acceptable salt thereof, capable of modulating expression and/or function of RPS25 gene, wherein nucleotides of the single-stranded antisense oligonucleotide are bonded to each other via a phosphate group and/or a modified phosphate group, the single-stranded antisense oligonucleotide includes a gap region, a 3' wing region bonded to a 3' end of the gap region, and a 5' wing region bonded to a 5' end of the gap region, the gap region is a deoxyribose-based nucleic acid optionally including a nucleic acid having a modified sugar moiety, each of the 3' wing region and the 5' wing region is a modified nucleotide, the single-stranded antisense oligonucleotide has a base length of 12- to 30-mer, and a base sequence of the antisense oligonucleotide is: a base sequence with a sequence identity of 90% to 100% to a base sequence complementary to at least one target region of the same base length as the antisense oligonucleotide present in the base sequence as set forth in SEQ ID NO: 1 or SEQ ID NO: 2; a base sequence complementary to a base sequence of the target region with deletion, substitution, insertion, or addition of one or several bases; or a base sequence capable of hybridizing under stringent conditions with an



Processed by Luminess, 75001 PARIS (FR)

(Cont. next page)

oligonucleotide having the target region.

FIG.1

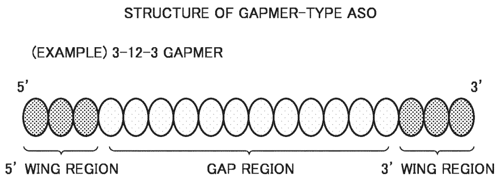

## Description

### TECHNICAL FIELD

The present invention relates to an antisense oligonucleotide capable of modulating expression and/or function of RPS25 gene, as well as to an agent comprising the same for modulating expression and/or function of RPS25 gene.

### BACKGROUND ART

Ribosomal Protein S25 (RPS25) gene is a gene that codes for one of the constituent proteins of ribosomal 40S subunit. The constituent protein coded for by RPS25 gene (RPS25 protein) has already been determined in terms of its conformation when it is present in the ribosome 40S subunit. (NPL 1)

RPS25 protein plays a role in protein synthesis process by controlling translation by means of binding to an RNA element that is capable of initiating cap independent translation. The RNA element to which RPS25 protein binds is called Internal Ribosome Entry Site (IRES). IRES is one of the cap-independent translation mechanisms often found in viruses in particular. (NPL 2)

Repeat associated non-ATG translation (RAN translation) was first reported in a patient with spinocerebellar ataxia type 8 in 2011 (NPL 3). RAN translation refers to a mechanism where repeat expansion of a particular sequence brings about ATG-independent translation into a protein (such as a dipeptide repeat (DPR)). After this, more reports followed, suggesting the involvement of RAN translation in a plurality of repeat diseases (diseases caused by repeat expansion of a particular gene sequence) including amyotrophic lateral sclerosis (ALS) with mutation in C9orf72 gene (which may also be expressed as "C9orf72 ALS" hereinafter), Huntington's disease, and myotonic dystrophy. Researches were conducted to investigate the correlation between the DPR produced by RAN translation and the pathology, and it was reported that DPR removal was effective for alleviating the pathology. (NPL 4, NPL 5)

In 2019, a report identified RPS25 protein as a molecule that significantly contributes to the dipeptide repeat production attributable to RAN translation. RPS25 gene knockdown reduced RAN-translation-dependent DPR production attributable to GGGGCC repeats or CAG repeats. The GGGGCC repeat is known as abnormal expansion mutation of C9orf72 gene, a familial mutation in amyotrophic lateral sclerosis. The CAG repeat is known as abnormal expansion mutation of huntingtin gene and ATXN2 gene. In motor neurons derived from induced pluripotent stem cells (iPSC) established from a patient with C9orf72 gene mutation, it was demonstrated that RPS25 gene knockdown reduced DPR production attributable to GGGGCC repeats and also reduced death of the motor neurons. (NPL 6)

The antisense oligonucleotide for RPS25 gene disclosed by NPL 6 is a gapmer that has its wing moiety modified with 2'-O-methylated RNA (2'-OMe nucleic acid) and also has phosphorothioate bonds between its nucleosides. The base sequence of the antisense oligonucleotide includes a partial base sequence of the sense strand of the RPS25 gene.

### CITATION LIST

#### PATENT LITERATURES

PTL 1: International Patent Laying-Open No. WO 2019/084068
PTL 2: International Patent Laying-Open No. WO 2011/052436
PTL 3: International Patent Laying-Open No. WO 2014/046212
PTL 4: International Patent Laying-Open No. WO 2015/125783
PTL 5: International Patent Laying-Open No. WO 2020/158910
PTL 6: International Patent Laying-Open No. WO 99/14226


#### NON PATENT LITERATURES

NPL 1: Science (2011) 331 (6018):730-736.
NPL 2: Genes Dev. (2009) 23 (23):2753-2764.
NPL 3: Proc. Natl. Acad. Sci. USA (2011) 108 (1):260-265
NPL 4: Neuron (2015) 88:667-677
NPL 5: Neuron (2020) 105:645-662
NPL 6: Nat. Neurosci. (2019) 22 (9):1383-1388

NPL 7: Mol. Gen. Genet. (1979) 169:1-6
NPL 8: Curr. Opin. Struct. Biol. (2014) 24:165-169
NPL 9: J. Med. Chem. (2016) 59:9645-9667
NPL 10: Nature (2015) 518 (7539):409-412

## SUMMARY OF INVENTION

## TECHNICAL PROBLEM

Although PTL 1 recites that RPS25 gene inhibition reduces DPR production, it is unclear whether the same effect can be exhibited by an approach which uses a nucleic acid such as an antisense nucleic acid. In addition, the RPS25 gene expression-reducing action of the antisense oligonucleotide recited by NPL 5 is limited, and, therefore, for pharmaceutical application, more effective RPS25 gene antisense oligonucleotides need to be developed.

The present invention has been devised in light of the above-described circumstances, and an object of the present invention is to provide a single-stranded antisense oligonucleotide capable of modulating expression and/or function of RPS25 gene, as well as an agent comprising the same for modulating expression and/or function of RPS25 gene.

## SOLUTION TO PROBLEM

The inventors of the present invention have conducted intensive research to achieve the above object and, as a result, have found a single-stranded antisense oligonucleotide or a pharmaceutically acceptable salt thereof (which may also be called "the antisense oligonucleotide according to the present invention" hereinafter) capable of binding to RPS25 gene and effectively modulating the expression of the RPS25 gene. Thus, the present invention has now been completed. In the following, more specific description of the present invention will be given.

[1] An antisense oligonucleotide according to the present invention is a single-stranded antisense oligonucleotide, or a pharmaceutically acceptable salt thereof, capable of modulating expression and/or function of RPS25 gene, wherein

nucleotides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid optionally including a nucleic acid having a modified sugar moiety,
each of the 3' wing region and the 5' wing region is a modified nucleic acid,
the single-stranded antisense oligonucleotide has a base length of 12- to 30-mer, and
a base sequence of the single-stranded antisense oligonucleotide is:

a base sequence with a sequence identity of 90% to 100% to a base sequence complementary to at least one target region of the same base length as the single-stranded antisense oligonucleotide present in a base sequence as set forth in SEQ ID NO: 1 or SEQ ID NO: 2;
a base sequence complementary to a base sequence of the target region with deletion, substitution, insertion, or addition of one or several bases; or
a base sequence capable of hybridizing under stringent conditions with an oligonucleotide having the target region.

The antisense oligonucleotide according to the present invention has modified nucleic acids in the gap region, the 5' wing region, and the 3' wing region. When the gap region has a deoxyribose including a nucleic acid having a modified sugar moiety, the modified nucleic acid is preferably 5'-CP nucleic acid (5'-cyclopropyl nucleic acid). As a modified nucleic acid which may be included in the 5' wing region and the 3' wing region, if it is a 2'-modified nucleic acid, then it is 2'-MOE nucleic acid (2'-O-methoxyethyl nucleic acid), 2'-OMe nucleic acid (2'-O-methyl nucleic acid), and/or MCE (2'-O-(2-N-methylcarbamoyl) ethyl nucleic acid), and if it is a bridged nucleic acid, then it is 2',4'-BNA (Locked Nucleic Acid, which may also be called "LNA" hereinafter), AmNA (Amido-bridged nucleic acid), GuNA (Guanidino-bridged nucleic acid), and/or scpBNA (2'-O,4'-C-Spirocyclopropylene bridged nucleic acid); preferably, at least one of these modified nucleic acids is included. With this configuration, the antisense oligonucleotide according to the present invention is expected to have a high binding affinity for RPS25 mRNA or mRNA precursor. In addition, the antisense oligonucleotide according to the present invention, which is a so-called gapmer-type single-stranded antisense oligonucleotide, functions as a catalyst in RNase-driven RPS25 gene degradation reaction, which is described below. Because of this, administration of only a small amount is expected to exhibit a certain level of sustained effect.

[2] Preferably, the base sequence of the single-stranded antisense oligonucleotide is:
a base sequence with a sequence identity of 95% to 100% to a base sequence complementary to at least one target region of the same base length as the single-stranded antisense oligonucleotide present in the base sequence as set forth in SEQ ID NO: 1 or SEQ ID NO: 2.

[3] Preferably, the base sequence of the single-stranded antisense oligonucleotide is:
a base sequence complementary to at least one target region of the same base length as the single-stranded antisense oligonucleotide present in the base sequence as set forth in SEQ ID NO: 1 or SEQ ID NO: 2.

[4] Preferably, the gap region has a base count of 5- to 20-mer,

the 3' wing region is a 1- to 5-mer modified nucleic acid, and
the 5' wing region is a 1- to 5-mer modified nucleic acid.

[5] Preferably, the single-stranded antisense oligonucleotide has a base length of 14- to 22-mer.

[6] Preferably, the modified nucleic acid of the 3' wing region includes at least one selected from the group consisting of 2'-MOE nucleic acid, LNA, AmNA, GuNA, and scpBNA, and
the modified nucleic acid of the 5' wing region includes at least one selected from the group consisting of 2'-MOE nucleic acid, LNA, AmNA, GuNA, and scpBNA.

[7] Preferably, at least one bond between nucleotides of the single-stranded antisense oligonucleotide is a phosphorothioate bond.

[8] Preferably, at least one bond between nucleotides of the single-stranded antisense oligonucleotide is a phosphodiester bond.

[9] Preferably, the base sequence of the single-stranded antisense oligonucleotide is:

a base sequence with a sequence identity of 90% to 100% to a base sequence complementary to a target region of 14- to 22-mer present in the base sequence as set forth in SEQ ID NO: 1 following a base located at one of position 8 to position 10, position 27 to position 29, position 34 to position 40, position 79, position 98, position 101 to position 106, position 123 to position 129, position 140, position 160 to position 161, position 180 to position 191, position 208 to position 221, position 242 to position 243, position 255 to position 268, position 285 to position 286, position 292 to position 304, position 321 to position 328, position 340 to position 344, position 365, and position 429 to position 454 counted from a 5' end of the base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region.

[10] Preferably, the base sequence of the single-stranded antisense oligonucleotide is a base sequence with a sequence identity of 90% to 100% to a base sequence complementary to a target region of 14- to 22-mer present in the base sequence as set forth in SEQ ID NO: 1 following a base located at one of position 8, position 10, position 28 to position 29, position 35 to position 37, position 101 to position 104, position 123 to position 126, position 129, position 160, position 180 to position 187, position 209 to position 220, position 258 to position 267, position 285, position 295 to position 297, position 300 to position 304, position 321 to position 327, position 341, position 344, position 365, and position 429 to position 454 counted from a 5' end of the base sequence as set forth in SEQ ID NO: 1,

the 3' wing region is a 2- to 5-mer, and
the 5' wing region is a 2- to 5-mer.

[11] Preferably, the base sequence of the single-stranded antisense oligonucleotide is a base sequence complementary to a target region of 14- to 22-mer present in the base sequence as set forth in SEQ ID NO: 1 following a base located at one of position 36, position 102 to position 103, position 123 to position 126, position 185 to position 187, position 213 to position 214, position 220, position 259 to position 260, position 263 to position 265, position 295 to position 296, position 300, position 302 to position 303, position 322 to position 327, position 429 to position 431, position 435, and position 438 to position 454 counted from a 5' end of the base sequence as set forth in SEQ

ID NO: 1.

[12] Preferably, the base sequence of the single-stranded antisense oligonucleotide is a base sequence selected from the group consisting of base sequences as set forth in SEQ ID NOs: 18, 24 to 25, 28 to 29, 38, 48 to 49, 53, 58 to 59, 63 to 64, 66 to 68, 79 to 80, 84, 86 to 91, 93 to 95, 97, 99 to 105, 113 to 119, 121 to 123, 125, 127 to 130, 140, 162, 169, 171 to 173, 183, 188, 190, 304 to 306, 309, 310, 312, 313, 317, 321 to 323, 326 to 327, 331 to 332, 334, 337, 340 to 344, 346, 348, 349, 351, 353, 355 to 364, 366 to 367, 371 to 382, 385, 386, 388, 389, 391, 394, 396, 397, 407, 408, 410, 418 to 424, 426 to 427, and 431 to 432.

[13] Preferably, the base sequence of the single-stranded antisense oligonucleotide is:

a base sequence with a sequence identity of 90% to 100% to a base sequence complementary to a target region of 14- to 22-mer present in the base sequence as set forth in SEQ ID NO: 2 following a base located at one of position 1, position 75, position 233, position 261, position 278 to position 280, position 390 to position 392, position 417 to position 423, position 445 to position 447, position 460 to position 461, position 510, position 561 to position 562, position 589, position 605, position 626 to position 628, position 632 to position 634, position 696 to position 697, position 1034 to position 1035, position 1103 to position 1107, position 1128 to position 1129, position 1196 to position 1197, position 1398, position 1408 to position 1412, position 1478 to position 1480, position 1715, position 1749 to position 1751, position 2047 to position 2049, position 2121 to position 2123, position 2260 to position 2268, position 2342, position 2406, and position 2585 to position 2587 counted from a 5' end of the base sequence as set forth in SEQ ID NO: 2;

a base sequence complementary to a base sequence of the target region with deletion, substitution, insertion, or addition of one or several bases; or

a base sequence capable of hybridizing under stringent conditions with an oligonucleotide having the target region.

[14] Preferably, the base sequence of the single-stranded antisense oligonucleotide is a base sequence with a sequence identity of 90% to 100% to a base sequence complementary to a target region of 14- to 22-mer present in the base sequence as set forth in SEQ ID NO: 2 following a base located at one of position 1, position 278 to position 279, position 417 to position 420, position 561, position 605, position 627, position 632 to position 634, position 697, position 1035, position 1128, position 1196 to position 1197, position 1409 to position 1410, position 1478, position 1715, position 1750, position 2047 to position 2049, position 2342, position 2406, and position 2585 to position 2587 counted from a 5' end of the base sequence as set forth in SEQ ID NO: 2,

the 3' wing region is a 2- to 5-mer, and
the 5' wing region is a 2- to 5-mer.

[15] A double-stranded antisense oligonucleotide according to the present invention is a double-stranded antisense oligonucleotide or a pharmaceutically acceptable salt thereof comprising:

the above-described single-stranded antisense oligonucleotide; and

a second oligonucleotide hybridized to the single-stranded antisense oligonucleotide, wherein

a base sequence of the second oligonucleotide is a base sequence with a sequence identity of 90% to 100% to a base sequence complementary to the base sequence of the single-stranded antisense oligonucleotide.

[16] An antisense oligonucleotide complex according to the present invention is an antisense oligonucleotide complex or a pharmaceutically acceptable salt thereof comprising:

the above-described single-stranded antisense oligonucleotide or a pharmaceutically acceptable salt thereof, or the above-described double-stranded antisense oligonucleotide or a pharmaceutically acceptable salt thereof; and

an additional substance bonded to the single-stranded antisense oligonucleotide or to the second oligonucleotide, wherein

the additional substance is selected from the group consisting of polyethylene glycol, peptide, alkyl chain, ligand compound, antibody, protein, and sugar chain.

[17] A pharmaceutical according to the present invention comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient.

[18] An agent for modulating expression and/or function of RPS25 gene according to the present invention comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient.

[19] An agent for inhibiting dipeptide repeat production attributable to RAN translation according to the present invention comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient.

[20] An agent for treating a repeat disease according to the present invention comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient.

[21] An agent for preventing a repeat disease according to the present invention comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient.

[22] Preferably, with regard to the treating agent and the preventing agent described above, the repeat disease is at least one selected from the group consisting of C9orf72 ALS, frontotemporal lobar degeneration (FTLD) with mutation in C9orf72 gene (which may also be expressed as "C9orf72 FTLD" hereinafter), Huntington’s disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

[23] A method of modulating expression of RPS25 gene according to the present invention comprises administering the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient, to a cell, a tissue, or an individual expressing the RPS25 gene.

[24] A method of treating or preventing a repeat disease according to the present invention comprises administering the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient, to an individual suffering from the repeat disease. Preferably, the repeat disease is at least one selected from the group consisting of C9orf72 ALS, C9orf72 FTLD, Huntington’s disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

[25] The present invention provides a method of treating or preventing C9orf72 ALS comprising administering the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, to an individual.

[26] The present invention provides the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, for use for treating or preventing C9orf72 ALS.

[27] The present invention provides the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, for use for producing an agent for treating or preventing C9orf72 ALS.

## ADVANTAGEOUS EFFECTS OF INVENTION

The present invention makes it possible to provide a single-stranded antisense oligonucleotide capable of modulating expression and/or function of RPS25 gene, as well as an agent comprising the same for modulating expression and/or function of RPS25 gene.

## BRIEF DESCRIPTION OF DRAWINGS

Fig. 1 is a schematic view of an example configuration of a single-stranded antisense oligonucleotide according to the present embodiment.

Fig. 2 is a schematic view describing the mechanism of reduction of RPS25 gene expression when the single-stranded antisense oligonucleotide according to the present embodiment is used.

Fig. 3 is a schematic view of the chemical structure of the single-stranded antisense oligonucleotide used in Example 412.

Fig. 4 is a schematic view of the chemical structure of the single-stranded antisense oligonucleotide used in Example 413.

Fig. 5 is a schematic view of the chemical structure of the single-stranded antisense oligonucleotide used in Example 414.

Fig. 6 is a schematic view of the chemical structure of the single-stranded antisense oligonucleotide used in Example 415.

Fig. 7 is a schematic view of the chemical structure of the single-stranded antisense oligonucleotide used in Example 416.

## DESCRIPTION OF EMBODIMENTS

In the following, a description will be given of an embodiment of the present invention (which may also be expressed as "the present embodiment" hereinafter). It should be noted that the present embodiment is not limited to the description below. Herein, the expression "from I to J" means the upper limit and the lower limit of a range (that is, it means "not less than I and not more than J"). When I is not accompanied by unit expression and J is accompanied by unit expression, the unit of I is the same as the unit of J.

### <<Single-Stranded Antisense Oligonucleotide Capable of Modulating Expression and/or Function of RPS25 Gene>>

A single-stranded antisense oligonucleotide according to the present embodiment is a single-stranded antisense oligonucleotide, or a pharmaceutically acceptable salt thereof, capable of modulating expression and/or function of RPS25 gene, wherein

nucleotides of the single-stranded antisense oligonucleotide are bonded to each other via a phosphate group and/or a modified phosphate group,

the single-stranded antisense oligonucleotide includes a gap region, a 3' wing region bonded to a 3' end of the gap region, and a 5' wing region bonded to a 5' end of the gap region,

the gap region is a deoxyribose-based nucleic acid optionally including a nucleic acid having a modified sugar moiety,

each of the 3' wing region and the 5' wing region is a modified nucleic acid,

the antisense oligonucleotide has a base length of 12- to 30-mer, and

a base sequence of the antisense oligonucleotide is:

> a base sequence with a sequence identity of 90% to 100% to a base sequence complementary to at least one target region of the same base length as the antisense oligonucleotide present in a base sequence as set forth in SEQ ID NO: 1 or SEQ ID NO: 2;
>
> a base sequence complementary to a base sequence of the target region with deletion, substitution, insertion, or addition of one or several bases; or
>
> a base sequence capable of hybridizing under stringent conditions with an oligonucleotide having the target region. In the following, a detailed description will be given.

### <Definitions of Terms, etc.>

First, definitions and the like of terms used in the present specification are given below.

### (RPS25 Gene)

Definition of "RPS25 gene" herein can be found in Mol. Gen. Genet. (1979) 169:1-6 (NPL 7) and Curr. Opin. Struct. Biol. (2014) 24:165-169 (NPL 8). Examples of synonyms of "RPS25" include 40S ribosomal protein S25, ribosomal protein S25, Small ribosomal subunit protein eS25, Rps25, 2810009D21Rik, ribosomal protein s25, Ribosomal Protein S25, S25, eS25, and ribosomal protein.

(Single-Stranded Antisense Oligonucleotide)

Herein, "single-stranded antisense oligonucleotide" or "antisense oligonucleotide" (which may also be called "ASO" hereinafter) means an oligonucleotide that is complementary to mRNA, mRNA precursor, or ncRNA (noncoding RNA) of the target gene (hereinafter, these three may also be collectively called "target RNA"), or a pharmacologically acceptable salt of the oligonucleotide. The antisense oligonucleotide is composed of DNA, RNA, and/or an analog thereof. The antisense oligonucleotide forms a double strand with the target mRNA, mRNA precursor, or ncRNA to reduce the action of the target mRNA, mRNA precursor, or ncRNA. The antisense oligonucleotide includes the following: one having a base sequence fully complementary to the base sequence of the target mRNA, mRNA precursor, or ncRNA; one having this complementary base sequence with deletion, substitution, insertion, or addition of one or several bases; and one including, in its base sequence, a base that is capable of forming a wobble base pair.

Moreover, the antisense oligonucleotide according to the present invention may further include, in addition to "a modified nucleic acid whose sugar moiety is a modified sugar" (a modified nucleotide having sugar modification) described below, other modified nucleotides that are known in the field. Examples of the modified nucleotides that are known in the field, other than the modified nucleotides having sugar modification, include modified nucleotides having phosphate modification, modified nucleotides having nucleobase modification (both of which are described below), and the like.

The structure of either terminal of the antisense oligonucleotide according to the present embodiment is not particularly limited, and may be either -OH or -OR (where R represents an alkyl chain, a phosphate, or an additional substance described below), for example.

Moreover, the single-stranded antisense oligonucleotide according to the present embodiment may be in the form of a single strand, or may be hybridized with a second oligonucleotide (which is described below) to form a double strand. A double-stranded oligonucleotide that is composed of the single-stranded antisense oligonucleotide and the second oligonucleotide hybridized with the single-stranded antisense oligonucleotide may also be called "a double-stranded antisense oligonucleotide".

(Oligonucleotide)

Herein, "oligonucleotide" means a polymer of 2 to 30 nucleotides (which are the same as or different from each other) bonded to each other via phosphodiester bonding or other bondings. Another way of understanding it is that the oligonucleotide is composed of a nucleobase moiety, a phosphate moiety, and a sugar moiety, as shown by a structural formula below.

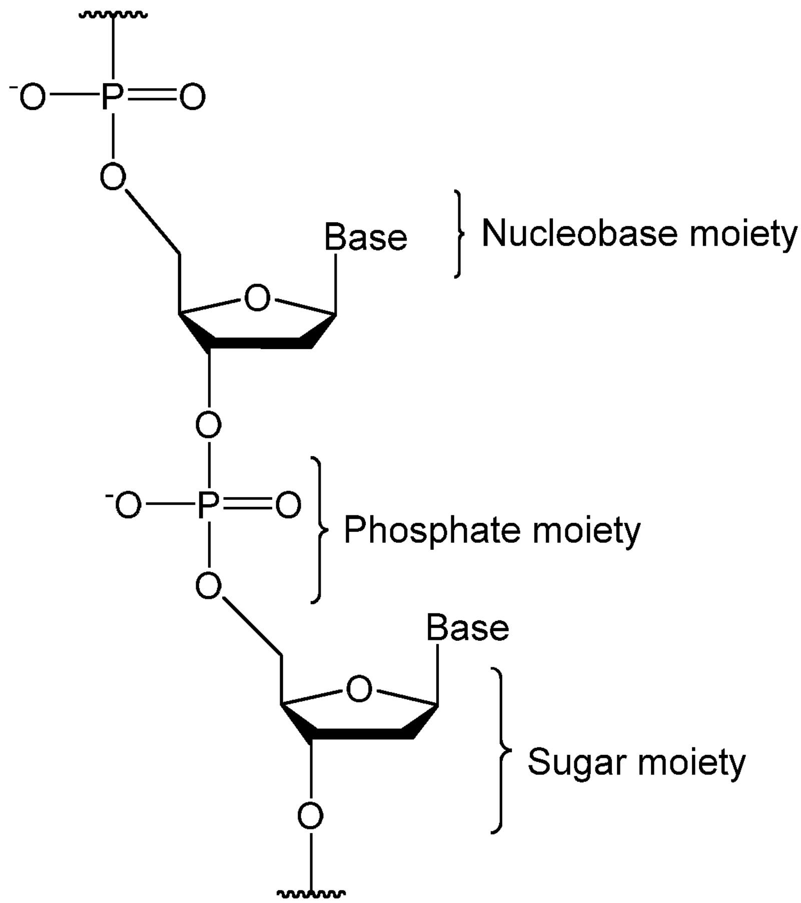


The oligonucleotide is broadly classified into natural oligonucleotide and non-natural oligonucleotide. "Natural oligonucleotide" means an oligonucleotide formed of naturally-occurring nucleotides. "Non-natural oligonucleotide" means an oligonucleotide that includes at least one modified nucleotide (which is described below) as a structural unit. Preferable examples of the "non-natural oligonucleotide" include sugar-modified derivatives in which the sugar moiety is modified; phosphorothioate derivatives in which one non-bridging oxygen atom of the phosphodiester bond is substituted with a sulfur atom; phosphorodithioate derivatives in which two non-bridging oxygen atoms of the phosphodiester bond are substituted with sulfur atoms; ester derivatives in which the phosphodiester bond is converted into the triester form; phosphoamide derivatives in which the phosphodiester bond is amidated; boranophosphate derivatives in which the phosphodiester bond is converted into the boronate ester form; alkylphosphonate (such as methylphosphonate or methoxypropylphosphonate, for example) derivatives in which a non-bridging oxygen atom of the phosphodiester bond is substituted with an alkyl group; amide derivatives in which the phosphodiester bond is substituted with an amide bond; and modified base derivatives in which the nucleobase is modified. Further preferable examples of the non-natural oligonucleotide include bridged sugar-modified derivatives in which the sugar moiety is modified; phosphorothioate derivatives in which one non-bridging oxygen atom of the phosphodiester bond is substituted with a sulfur atom; ester derivatives in which the phosphodiester bond is converted into the ester form; alkylphosphonated derivatives in which the sugar moiety is modified with a modified sugar (which is described below) (such as a bridged sugar, for example), and in which one non-bridging oxygen atom of the phosphodiester bond is substituted with a sulfur atom or a non-bridging oxygen atom of the phosphodiester bond is substituted with an alkyl group; and the like.

(Nucleoside)

Herein, "nucleoside" means a compound that consists of a purine base or a pyrimidine base and a sugar which are bonded to each other. A naturally-occurring nucleoside may also be called "a natural nucleoside". A non-naturally-occurring modified nucleoside may also be called "a modified nucleoside". A modified nucleoside whose sugar moiety is modified, in particular, may also be called "a sugar-modified nucleoside".

(Nucleotide)

Herein, "nucleotide" means a compound that consists of an above-defined nucleoside whose sugar is bonded to a phosphate group. A naturally-occurring nucleotide may also be called "a natural nucleotide". A non-naturally-occurring modified nucleotide may also be called "a modified nucleotide" or "a modified nucleic acid". Examples of the "modified nucleotide" or the "modified nucleic acid" include compounds that consist of an above-defined modified nucleoside whose sugar moiety is bonded to a phosphate group, compounds that consist of an above-defined modified nucleoside whose sugar moiety is bonded to a modified phosphate group (which is described below), compounds that consist of a natural nucleoside whose sugar moiety is bonded to a modified phosphate group (which is described below), and the like.

(Sugar Modification, Modified Sugar)

Herein, "sugar modification" means modification to the sugar moiety of an above-defined nucleotide. A modified sugar moiety, in particular, may also be called "a modified sugar". A modified nucleotide with sugar modification can be used as a modified nucleic acid, and examples thereof include AmNA, GuNA, scpBNA, 2'-O-alkyl (such as 2'-O-methyl nucleic acid and 2'-MOE nucleic acid, for example), 2'-F, 5'-methyl-DNA, LNA, ENA (2'-O,4'-C-Ethylene-bridged Nucleic Acid), S-cEt (2',4'-constrained Ethyl nucleic acid), 5'-CP nucleic acid (5'-CycloPropyl nucleic acid), and the like.

Examples of LNA include those that include structures represented by symbols "A (L)", "5 (L)", "G (L)", "T (L)" which are described below. Examples of AmNA include those that include structures represented by symbols "A (Y)", "5 (Y)", "G (Y)", "T (Y)" which are described below. Examples of GuNA include those that include structures represented by symbols "A (Gx)", "5 (Gx)", "G (Gx)", "T (Gx)" which are described below. Examples of scpBNA include those that include structures represented by symbols "A (S)", "5 (S)", "G (S)", "T (S)" which are described below. Examples of 2'-MOE nucleic acid include those that include structures represented by symbols "A (m)", "5 (m)", "G (m)", "T (m)" which are described below. Examples of 5'-CP nucleic acid include those that include structures represented by symbols "A (5'-CP)", "5 (5'-CP)", "G (5'-CP)", "T (5'-CP)" which are described below. Examples of 2'-OMe nucleic acid include those that include structures represented by symbols "A (M)", "C (M)", "G (M)", "U (M)" which are described below. Examples of MCE nucleic acid include those that include structures represented by symbols "A (Mx)", "C (Mx)", "G (Mx)", "U (Mx)" which are described below.

(Nucleotide Modification Known in Field Other Than Sugar Modification)

A nucleotide modification that is known in the field other than the above-defined sugar modification can be used for a modified nucleic acid that is to be used for producing the single-stranded antisense oligonucleotide according to the present invention. As nucleotide modifications of this type, phosphate modification and nucleobase modification are known, which are described below. Examples of the nucleotide modifications of this type include nucleotide modification described in W. Brad Wan et. Al. J. Med. Chem. (2016) 59:9645-9667. (NPL 9), for example. Such nucleotide modifications can be carried out based on a method known in the field described in a document cited by the above document.

(Phosphate Group)

Herein, "phosphate group" means a phosphate moiety of an above-defined nucleotide whose bonding is a naturally-occurring phosphodiester bond (which is a bond represented by symbol "-" described below).

(Phosphate Modification, Modified Phosphate Group)

Herein, "phosphate modification" means modification to the phosphate moiety of an above-defined nucleotide. A modified phosphate moiety, in particular, may also be called "a modified phosphate group". Examples of the bonding that includes this modified phosphate group include a phosphorothioate bond (a bond represented by symbol "^" described below), a phosphorodithioate bond, a phosphoamidate bond (a bond represented by symbol "=" described below), a boranophosphate bond (a bond represented by symbol "×" described below), alkylphosphonate, and the like.

(Nucleobase Modification, Modified Nucleobase)

Herein, "nucleobase modification" means modification to the nucleobase moiety of an above-defined nucleotide. A modified nucleobase moiety, in particular, may also be called "a modified nucleobase". Examples of the modified nucleobase include 5-methylcytosine, 5-hydroxymethylcytosine, 5-propynylcytosine, and the like.

(DNA or RNA Analog)

The above-mentioned DNA or RNA analog means a molecule whose structure is similar to that of DNA or RNA. Examples thereof include peptide nucleic acids (PNA), morpholino nucleic acids, and the like.

(ncRNA)

Herein, "ncRNA" is a generic name for RNAs that are not involved in protein translation. Examples of this ncRNA include ribosomal RNA, transfer RNA, miRNA, Natural Antisense Transcript (NAT), and the like.

(Nucleobase Moiety of Oligonucleotide)

Examples of the nucleobase moiety of an above-defined oligonucleotide include thyminyl group, cytosinyl group, adeninyl group, guaninyl group, 5-methylcytosinyl group, uracilyl group, 2-oxo-4-hydroxy-5-methyl-1,2-dihydropyrimidin-1-yl group, 2-oxo-4-amino-1,2-dihydropyrimidin-1-yl group, 4-amino-5-methyl-2-oxo-1,2-dihydropyrimidin-1-yl group, 2-oxo-4-hydroxy-1,2-dihydropyrimidin-1-yl group, and the like. Preferable examples of the nucleobase moiety include thyminyl group, cytosinyl group, adeninyl group, guaninyl group, 5-methylcytosinyl group, uracilyl group, and the like. Among these nucleobases, uracil (U) and thymine (T) are interchangeable. Both uracil (U) and thymine (T) are capable of forming a base pair with adenine (A) in the complementary strand. The same is true for the nucleobase moiety of an antisense oligonucleotide.

(Target RNA)

Herein, "target RNA" means an RNA whose function is reduced by an above-defined single-stranded antisense oligonucleotide binding thereto. In other words, the target RNA according to the present embodiment means RPS25 mRNA and mRNA precursor. Examples of the target RNA include human RPS25 mRNA (which may also be called "hRPS25" hereinafter) having the base sequence as set forth in SEQ ID NO: 1, human RPS25 mRNA precursor (which may also be called "hpRPS25" hereinafter) having the base sequence as set forth in SEQ ID NO: 2, monkey RPS25 mRNA (which may also be called "cRPS25" hereinafter) having the base sequence as set forth in SEQ ID NO: 3, monkey RPS25 mRNA precursor having the base sequence as set forth in SEQ ID NO: 4, mouse RPS25 mRNA (which may also be called "mRPS25" hereinafter) having the base sequence as set forth in SEQ ID NO: 5, mouse RPS25 mRNA precursor having the base sequence as set forth in SEQ ID NO: 6, and the like.

(Binding to Target RNA)

Herein, "binding to target RNA" means that the nucleobase of the single-stranded antisense oligonucleotide forms a double-stranded nucleic acid with the nucleobase of target RNA due to complementation to the target RNA. The double-stranded nucleic acid needs to be formed only with at least part of the target RNA. The strength of the bonding to the target RNA can be measured with the use of a thermal stability index, for example. Examples of the thermal stability index include the melting temperature (Tm value) of the double-stranded nucleic acid, and the like. The Tm value is preferably from 40 to 90°C, more preferably from 50 to 70°C.

(Target Region)

The above-mentioned target region means a region in RPS25 mRNA and mRNA precursor capable of binding to the single-stranded antisense oligonucleotide. The target region includes a target region consisting of the above-described base sequence as well as a region of RPS25 mRNA precursor.

(mRNA Precursor)

The mRNA precursor means a primary RNA transcript transcribed from DNA. That is, the mRNA precursor is an RNA that includes an exon region, an intron region, and an untranslated region (UTR). Another way of understanding it is that the mRNA precursor is an RNA prior to post-transcriptional splicing. The mRNA precursor is spliced to become an mRNA.

(Binding to Target Region)

Binding to the target region means that the single-stranded antisense oligonucleotide according to the present

invention forms a double strand with the target region. The single-stranded antisense oligonucleotide according to the present invention does not necessarily need to form a double strand with the entire target region; it only needs to form a double strand with a part of the target region. In other words, the single-stranded antisense oligonucleotide according to the present invention is preferably fully complementary to the target region, but, as long as it is capable of binding to RPS25 target RNA, it is only necessary to be complementary to at least part of the target region.

(Part of Target Region)

The above-mentioned part of the target region means a region of the target region consisting of 10 to 15 nucleotide bases.

(Complementary to At Least Part of Target Region)

The expression "complementary to at least part of the target region" means being complementary to the base(s) of at least part of the target region of the target RNA. This expression also includes being complementary to the base(s) of mRNA or mRNA precursor corresponding to the at least part of the region.

<Base Sequence of Single-Stranded Antisense Oligonucleotide>

The base sequence of the single-stranded antisense oligonucleotide according to the present embodiment is:

(A) a base sequence with a sequence identity of 90% to 100% to a base sequence complementary to a target region of 12- to 30-mer (preferably 14- to 22-mer) present in the base sequence as set forth in SEQ ID NO: 1 following a base located at one of position 8 to position 10, position 27 to position 29, position 34 to position 40, position 79, position 98, position 101 to position 106, position 123 to position 129, position 140, position 160 to position 161, position 180 to position 191, position 208 to position 221, position 242 to position 243, position 255 to position 268, position 285 to position 286, position 292 to position 304, position 321 to position 328, position 340 to position 344, position 365, and position 429 to position 454 counted from the 5' end of the base sequence as set forth in SEQ ID NO: 1; or
a base sequence with a sequence identity of 90% to 100% to a base sequence complementary to a target region of 12- to 30-mer (preferably 14- to 22-mer) present in the base sequence as set forth in SEQ ID NO: 2 following a base located at one of position 1, position 75, position 233, position 261, position 278 to position 280, position 390 to position 392, position 417 to position 423, position 445 to position 447, position 460 to position 461, position 510, position 561 to position 562, position 589, position 605, position 626 to position 628, position 632 to position 634, position 696 to position 697, position 1034 to position 1035, position 1103 to position 1107, position 1128 to position 1129, position 1196 to position 1197, position 1398, position 1408 to position 1412, position 1478 to position 1480, position 1715, position 1749 to position 1751, position 2047 to position 2049, position 2121 to position 2123, position 2260 to position 2268, position 2342, position 2406, and position 2585 to position 2587 counted from the 5' end of the base sequence as set forth in SEQ ID NO: 2;
(B) a base sequence complementary to a base sequence of the target region with deletion, substitution, insertion, or addition of one or several bases; or
(C) a base sequence capable of hybridizing under stringent conditions with an oligonucleotide having the target region.

With regard to the present embodiment, each base sequence provided in the sequence listing identifies the information of the sequence of the nucleobase moiety. The information of the structure of the oligonucleotide including the nucleobase moiety as well as the sugar moiety and the phosphate moiety is provided in the form shown in Table 3-1 to Table 3-17 and Table 4-1 to Table 4-5 provided below.

Herein, "sequence identity" means the following: two base sequences are aligned by a mathematical algorithm known in the technical field (preferably, this algorithm tolerates gap introduction to either one of the sequences or both the sequences for the sake of optimum alignment), and, in the resulting optimum alignment, the proportion (%) of matching bases across the entire base sequences overlapping each other is referred to as "sequence identity". A person skilled in the art can easily check the "sequence identity" between base sequences. For example, NCBI BLAST (National Center for Biotechnology Information Basic Local Alignment Search Tool) can be used.

The base sequence of the single-stranded antisense oligonucleotide according to the present embodiment preferably has a sequence identity of 95% to 100%, more preferably has a sequence identity of 98% to 100%, further preferably has a sequence identity of 100%, to a base sequence complementary to the above-described particular target region present in the base sequence as set forth in SEQ ID NO: 1 or SEQ ID NO: 2.

In the present embodiment, examples of the "base sequence with deletion, substitution, insertion, or addition of one or several bases" include a base sequence after the deletion, substitution, insertion, or addition that has a sequence identity of 80% or more, 85% or more, 90% or more, 95% or more, 97% or more, 98% or more, or 99% or more to a base sequence before the deletion, substitution, insertion, or addition. As for the specific number meant by the "one or several bases", the number of bases for each of the deletion, substitution, insertion, or addition may be independently one, two, three, four, or five, and a combination of more than one of these may be present.

Herein, "stringent conditions" refers to conditions for carrying out incubation at room temperature for 12 hours in a solution containing 6×SSC (the composition of 1×SSC is 0.15-M NaCl, 0.015-M sodium citrate, pH7.0), 0.5% SDS, 5×Denhardt's solution, 100 μg/mL denatured salmon sperm DNA, and 50% (v/v) formamide, followed by rinsing with 0.5×SSC at a temperature of 50°C or more. This expression also includes more stringent conditions, such as, for example, incubation at 45°C or 60°C for 12 hours and rinsing with 0.2×SSC or 0.1×SSC wherein the rinsing is carried out at a temperature of 60°C or 65°C or more.

In an aspect of the present embodiment, preferably, the target region is a base sequence of 12- to 30-mer or 14- to 22-mer present in the base sequence as set forth in SEQ ID NO: 1 following a base located at one of position 8, position 10, position 28 to position 29, position 35 to position 37, position 101 to position 104, position 123 to position 126, position 129, position 160, position 180 to position 187, position 209 to position 220, position 258 to position 267, position 285, position 295 to position 297, position 300 to position 304, position 321 to position 327, position 341, position 344, position 365, and position 429 to position 454 counted from the 5' end of the base sequence as set forth in SEQ ID NO: 1.

In an aspect of the present embodiment, preferably, the target region is a base sequence of 12- to 30-mer or 14- to 22-mer present in the base sequence as set forth in SEQ ID NO: 2 following a base located at one of position 1, position 278 to position 279, position 417 to position 420, position 561, position 605, position 627, position 632 to position 634, position 697, position 1035, position 1128, position 1196 to position 1197, position 1409 to position 1410, position 1478, position 1715, position 1750, position 2047 to position 2049, position 2342, position 2406, and position 2585 to position 2587 counted from the 5' end of the base sequence as set forth in SEQ ID NO: 2, the 3' wing region is a 2- to 5-mer, and the 5' wing region is a 2- to 5-mer.

In another aspect of the present embodiment, preferably, the target region is a base sequence of 12- to 30-mer, 14- to 22-mer, or 14- to 20-mer present in the base sequence as set forth in SEQ ID NO: 1 following a base located at one of position 36, position 102 to position 103, position 123 to position 126, position 185 to position 187, position 213 to position 214, position 220, position 259 to position 260, position 263 to position 265, position 295 to position 296, position 300, position 302 to position 303, position 322 to position 327, position 429 to position 431, position 435, and position 438 to position 454 counted from the 5' end of the base sequence as set forth in SEQ ID NO: 1.

The single-stranded antisense oligonucleotide is capable of binding to the target region in RPS25. Herein, the single-stranded antisense oligonucleotide according to the present invention "binding to the target region in RPS25" encompasses direct binding of the single-stranded antisense oligonucleotide according to the present invention to RPS25 mRNA, as well as direct binding thereof to RPS25 mRNA precursor.

An aspect of the single-stranded antisense oligonucleotide according to the present invention is a single-stranded oligonucleotide that is capable of modulating expression of RPS25 gene and that has one of the base sequences shown in Table 1-1 to Table 1-9, where the single-stranded oligonucleotide is complementary to a target region in human RPS25 mRNA shown in Table 1-1 to Table 1-9. As long as it includes a base sequence shown in Table 1-1 to Table 1-9, the single-stranded oligonucleotide may be longer by one to five bases toward the 3' end and/or the 5' end. It can be understood that the target region is a region of human RPS25 mRNA that is associated with modulation of expression of human RPS25 gene in particular (for example, a region that has an mRNA secondary structure to which the antisense nucleotide can bind easily). For example, in Table 1-1, the 5' end position being "8" and the 3' end position being "22" mean that a base sequence from position 8 to position 22 of the base sequence as set forth in SEQ ID NO: 1 counted from the 5' end thereof is the target region in human RPS25 mRNA targeted by the corresponding single-stranded antisense oligonucleotide (sequence name: "h8-22").

[Table 1-1]

| Sequence name | Antisense oligonucleotide sequence (5'-3') | hRPS25 target region (SEQ ID NO: 1) | |
|---|---|---|---|
| | | 5' end position | 3' end position |
| h8-22 | C'G'T'C'A'A'G'A'T'G'T'C'G'G'A' | 8 | 22 |
| h9-23 | T'C'G'T'C'A'A'G'A'T'G'T'C'G'G' | 9 | 23 |
| h10-24 | C'T'C'G'T'C'A'A'G'A'T'G'T'C'G' | 10 | 24 |
| h27-41 | G'C'A'G'C'A'G'A'C'A'C'C'G'C'A' | 27 | 41 |

(continued)

| Sequence name | Antisense oligonucleotide sequence (5'-3') | hRPS25 target region (SEQ ID NO: 1) | |
|---|---|---|---|
| | | 5' end position | 3' end position |
| h28-42 | A'G'C'A'G'C'A'G'A'C'A'C'C'G'C' | 28 | 42 |
| h29-43 | T'A'G'C'A'G'C'A'G'A'C'A'C'C'G' | 29 | 43 |
| h30-44 | A'T'A'G'C'A'G'C'A'G'A'C'A'C'C' | 30 | 44 |
| h32-46 | G'A'A'T'A'G'C'A'G'C'A'G'A'C'A' | 32 | 46 |
| h33-47 | A'G'A'A'T'A'G'C'A'G'C'A'G'A'C' | 33 | 47 |
| h34-48 | G'A'G'A'A'T'A'G'C'A'G'C'A'G'A' | 34 | 48 |
| h35-49 | G'G'A'G'A'A'T'A'G'C'A'G'C'A'G' | 35 | 49 |
| h36-50 | C'G'G'A'G'A'A'T'A'G'C'A'G'C'A' | 36 | 50 |
| h37-51 | T'C'G'G'A'G'A'A'T'A'G'C'A'G'C' | 37 | 51 |
| h38-52 | C'T'C'G'G'A'G'A'A'T'A'G'C'A'G' | 38 | 52 |
| h39-53 | G'C'T'C'G'G'A'G'A'A'T'A'G'C'A' | 39 | 53 |
| h40-54 | A'G'C'T'C'G'G'A'G'A'A'T'A'G'C' | 40 | 54 |
| h72-86 | C'T'T'C'T'T'C'T'T'G'T'C'G'T'C' | 72 | 86 |
| h79-93 | C'G'T'C'C'T'T'C'T'T'C'T'T'C'T' | 79 | 93 |
| h98-112 | T'T'C'T'T'G'G'C'C'G'A'C'T'T'T' | 98 | 112 |
| h99-113 | T'T'T'C'T'T'G'G'C'C'G'A'C'T'T' | 99 | 113 |
| h100-114 | C'T'T'T'C'T'T'G'G'C'C'G'A'C'T' | 100 | 114 |
| h101-115 | T'C'T'T'T'C'T'T'G'G'C'C'G'A'C' | 101 | 115 |
| h102-116 | G'T'C'T'T'T'C'T'T'G'G'C'C'G'A' | 102 | 116 |
| h103-117 | T'G'T'C'T'T'T'C'T'T'G'G'C'C'G' | 103 | 117 |
| h104-118 | T'T'G'T'C'T'T'T'C'T'T'G'G'C'C' | 104 | 118 |

[Table 1-2]

| Sequence name | Antisense oligonucleotide sequence (5'-3') | hRPS25 target region (SEQ ID NO: 1) | |
|---|---|---|---|
| | | 5' end position | 3' end position |
| h105-119 | T'T'T'G'T'C'T'T'T'C'T'T'G'G'C' | 105 | 119 |
| h106-120 | C'T'T'T'G'T'C'T'T'T'C'T'T'G'G' | 106 | 120 |
| h107-121 | T'C'T'T'T'G'T'C'T'T'T'C'T'T'G' | 107 | 121 |
| h122-136 | G'A'T'T'T'G'T'T'C'A'C'T'G'G'G' | 122 | 136 |
| h123-137 | G'G'A'T'T'T'G'T'T'C'A'C'T'G'G' | 123 | 137 |
| h124-138 | C'G'G'A'T'T'T'G'T'T'C'A'C'T'G' | 124 | 138 |
| h125-139 | C'C'G'G'A'T'T'T'G'T'T'C'A'C'T' | 125 | 139 |
| h126-140 | C'C'C'G'G'A'T'T'T'G'T'T'C'A'C' | 126 | 140 |
| h127-141 | C'C'C'C'G'G'A'T'T'T'G'T'T'C'A' | 127 | 141 |
| h128-142 | C'C'C'C'C'G'G'A'T'T'T'G'T'T'C' | 128 | 142 |
| h129-143 | G'C'C'C'C'C'G'G'A'T'T'T'G'T'T' | 129 | 143 |

(continued)

| Sequence name | Antisense oligonucleotide sequence (5'-3') | hRPS25 target region (SEQ ID NO: 1) | |
|---|---|---|---|
| | | 5' end position | 3' end position |
| h130-144 | T'G'C'C'C'C'G'G'A'T'T'G'T' | 130 | 144 |
| h140-154 | T'T'T'T'G'G'C'C'T'T'G'C'C'C' | 140 | 154 |
| h160-174 | T'G'C'C'T'T'T'G'G'A'C'C'A'C'T' | 160 | 174 |
| h161-175 | T'T'G'C'C'T'T'T'G'G'A'C'C'A'C' | 161 | 175 |
| h180-194 | A'T'T'G'A'G'C'T'T'G'T'C'C'C'G' | 180 | 194 |
| h181-195 | T'A'T'T'G'A'G'C'T'T'G'T'C'C'C' | 181 | 195 |
| h182-196 | T'T'A'T'T'G'A'G'C'T'T'G'T'C'C' | 182 | 196 |
| h183-197 | G'T'T'A'T'T'G'A'G'C'T'T'G'T'C' | 183 | 197 |
| h184-198 | A'G'T'T'A'T'T'G'A'G'C'T'T'G'T' | 184 | 198 |
| h185-199 | A'A'G'T'T'A'T'T'G'A'G'C'T'T'G' | 185 | 199 |
| h186-200 | T'A'A'G'T'T'A'T'T'G'A'G'C'T'T' | 186 | 200 |
| h187-201 | C'T'A'A'G'T'T'A'T'T'G'A'G'C'T' | 187 | 201 |
| h188-202 | A'C'T'A'A'G'T'T'A'T'T'G'A'G'C' | 188 | 202 |
| h189-203 | G'A'C'T'A'A'G'T'T'A'T'T'G'A'G' | 189 | 203 |

[Table 1-3]

| Sequence name | Antisense oligonucleotide sequence (5'-3') | hRPS25 target region (SEQ ID NO: 1) | |
|---|---|---|---|
| | | 5' end position | 3' end position |
| h190-204 | A'G'A'C'T'A'A'G'T'T'A'T'T'G'A' | 190 | 204 |
| h191-205 | A'A'G'A'C'T'A'A'G'T'T'A'T'T'G' | 191 | 205 |
| h193-207 | A'C'A'A'G'A'C'T'A'A'G'T'T'A'T' | 193 | 207 |
| h196-210 | C'A'A'A'C'A'A'G'A'C'T'A'A'G'T' | 196 | 210 |
| h197-211 | T'C'A'A'A'C'A'A'G'A'C'T'A'A'G' | 197 | 211 |
| h208-222 | A'G'G'T'A'G'C'T'T'T'G'T'C'A'A' | 208 | 222 |
| h209-223 | T'A'G'G'T'A'G'C'T'T'T'G'T'C'A' | 209 | 223 |
| h210-224 | A'T'A'G'G'T'A'G'C'T'T'T'G'T'C' | 210 | 224 |
| h211-225 | C'A'T'A'G'G'T'A'G'C'T'T'T'G'T' | 211 | 225 |
| h212-226 | T'C'A'T'A'G'G'T'A'G'C'T'T'T'G' | 212 | 226 |
| h213-227 | A'T'C'A'T'A'G'G'T'A'G'C'T'T'T' | 213 | 227 |
| h214-228 | T'A'T'C'A'T'A'G'G'T'A'G'C'T'T' | 214 | 228 |
| h215-229 | T'T'A'T'C'A'T'A'G'G'T'A'G'C'T' | 215 | 229 |
| h216-230 | T'T'T'A'T'C'A'T'A'G'G'T'A'G'C' | 216 | 230 |
| h217-231 | G'T'T'T'A'T'C'A'T'A'G'G'T'A'G' | 217 | 231 |
| h218-232 | A'G'T'T'T'A'T'C'A'T'A'G'G'T'A' | 218 | 232 |
| h219-233 | G'A'G'T'T'T'A'T'C'A'T'A'G'G'T' | 219 | 233 |
| h220-234 | A'G'A'G'T'T'T'A'T'C'A'T'A'G'G' | 220 | 234 |

(continued)

| Sequence name | Antisense oligonucleotide sequence (5'-3') | hRPS25 target region (SEQ ID NO: 1) | |
|---|---|---|---|
| | | 5' end position | 3' end position |
| h221-235 | C'A'G'A'G'T'T'T'A'T'C'A'T'A'G' | 221 | 235 |
| h222-236 | A'C'A'G'A'G'T'T'T'A'T'C'A'T'A' | 222 | 236 |
| h223-237 | T'A'C'A'G'A'G'T'T'T'A'T'C'A'T' | 223 | 237 |
| h224-238 | T'T'A'C'A'G'A'G'T'T'T'A'T'C'A' | 224 | 238 |
| h242-256 | T'T'A'T'A'G'T'T'G'G'G'A'A'C'T' | 242 | 256 |
| h243-257 | T'T'T'A'T'A'G'T'T'G'G'G'A'A'C' | 243 | 257 |
| h253-267 | G'G'G'T'T'A'T'A'A'G'T'T'T'A'T' | 253 | 267 |

[Table 1-4]

| Sequence name | Antisense oligonucleotide sequence (5'-3') | hRPS25 target region (SEQ ID NO: 1) | |
|---|---|---|---|
| | | 5' end position | 3' end position |
| h254-268 | G'G'G'G'T'T'A'T'A'A'G'T'T'T'A' | 254 | 268 |
| h255-269 | T'G'G'G'G'T'T'A'T'A'A'G'T'T'T' | 255 | 269 |
| h256-270 | C'T'G'G'G'G'T'T'A'T'A'A'G'T'T' | 256 | 270 |
| h257-271 | G'C'T'G'G'G'G'T'T'A'T'A'A'G'T' | 257 | 271 |
| h258-272 | A'G'C'T'G'G'G'G'T'T'A'T'A'A'G' | 258 | 272 |
| h259-273 | C'A'G'C'T'G'G'G'G'T'T'A'T'A'A' | 259 | 273 |
| h260-274 | A'C'A'G'C'T'G'G'G'G'T'T'A'T'A' | 260 | 274 |
| h261-275 | C'A'C'A'G'C'T'G'G'G'G'T'T'A'T' | 261 | 275 |
| h262-276 | C'C'A'C'A'G'C'T'G'G'G'G'T'T'A' | 262 | 276 |
| h263-277 | A'C'C'A'C'A'G'C'T'G'G'G'G'T'T' | 263 | 277 |
| h264-278 | G'A'C'C'A'C'A'G'C'T'G'G'G'G'T' | 264 | 278 |
| h285-299 | T'C'G'A'A'T'C'T'T'C'A'G'T'C'T' | 285 | 299 |
| h286-300 | C'T'C'G'A'A'T'C'T'T'C'A'G'T'C' | 286 | 300 |
| h291-305 | G'G'A'G'C'C'T'C'G'A'A'T'C'T'T' | 291 | 305 |
| h292-306 | G'G'G'A'G'C'C'T'C'G'A'A'T'C'T' | 292 | 306 |
| h293-307 | A'G'G'G'A'G'C'C'T'C'G'A'A'T'C' | 293 | 307 |
| h294-308 | C'A'G'G'G'A'G'C'C'T'C'G'A'A'T' | 294 | 308 |
| h295-309 | C'C'A'G'G'G'A'G'C'C'T'C'G'A'A' | 295 | 309 |
| h296-310 | G'C'C'A'G'G'G'A'G'C'C'T'C'G'A' | 296 | 310 |
| h297-311 | G'G'C'C'A'G'G'G'A'G'C'C'T'C'G' | 297 | 311 |
| h298-312 | T'G'G'C'C'A'G'G'G'A'G'C'C'T'C' | 298 | 312 |
| h299-313 | C'T'G'G'C'C'A'G'G'G'A'G'C'C'T' | 299 | 313 |
| h300-314 | C'C'T'G'G'C'C'A'G'G'G'A'G'C'C' | 300 | 314 |
| h321-335 | A'A'G'G'A'G'C'T'C'C'T'G'A'A'G' | 321 | 335 |
| h322-336 | T'A'A'G'G'A'G'C'T'C'C'T'G'A'A' | 322 | 336 |

[Table 1-5]

| Sequence name | Antisense oligonucleotide sequence (5'-3') | hRPS25 target region (SEQ ID NO: 1) | |
|---|---|---|---|
| | | 5' end position | 3' end position |
| h323-337 | C'T'A'A'G'G'A'G'C'T'C'C'T'G'A' | 323 | 337 |
| h324-338 | A'C'T'A'A'G'G'A'G'C'T'C'C'T'G' | 324 | 338 |
| h325-339 | T'A'C'T'A'A'G'G'A'G'C'T'C'C'T' | 325 | 339 |
| h326-340 | T'T'A'C'T'A'A'G'G'A'G'C'T'C'C' | 326 | 340 |
| h327-341 | T'T'T'A'C'T'A'A'G'G'A'G'C'T'C' | 327 | 341 |
| h328-342 | C'T'T'T'A'C'T'A'A'G'G'A'G'C'T' | 328 | 342 |
| h329-343 | C'C'T'T'T'A'C'T'A'A'G'G'A'G'C' | 329 | 343 |
| h330-344 | T'C'C'T'T'T'A'C'T'A'A'G'G'A'G' | 330 | 344 |
| h331-345 | G'T'C'C'T'T'T'A'C'T'A'A'G'G'A' | 331 | 345 |
| h340-354 | G'T'T'T'G'A'T'A'A'G'T'C'C'T'T' | 340 | 354 |
| h341-355 | A'G'T'T'T'G'A'T'A'A'G'T'C'C'T' | 341 | 355 |
| h342-356 | C'A'G'T'T'T'G'A'T'A'A'G'T'C'C' | 342 | 356 |
| h343-357 | C'C'A'G'T'T'T'G'A'T'A'A'G'T'C' | 343 | 357 |
| h344-358 | A'C'C'A'G'T'T'T'G'A'T'A'A'G'T' | 344 | 358 |
| h365-379 | A'C'T'T'G'A'G'C'T'C'T'G'T'G'C' | 365 | 379 |
| h375-389 | G'G'T'G'T'A'A'A'T'T'A'C'T'T'G' | 375 | 389 |
| h390-404 | A'C'C'C'T'T'G'G'T'A'T'T'T'C'T' | 390 | 404 |
| h393-407 | T'C'C'A'C'C'C'T'T'G'G'T'A'T'T' | 393 | 407 |
| h394-408 | C'T'C'C'A'C'C'C'T'T'G'G'T'A'T' | 394 | 408 |
| h426-440 | T'A'T'T'C'A'T'G'C'A'T'C'T'T'C' | 426 | 440 |
| h427-441 | C'T'A'T'T'C'A'T'G'C'A'T'C'T'T' | 427 | 441 |
| h428-442 | C'C'T'A'T'T'C'A'T'G'C'A'T'C'T' | 428 | 442 |
| h429-443 | A'C'C'T'A'T'T'C'A'T'G'C'A'T'C' | 429 | 443 |
| h430-444 | G'A'C'C'T'A'T'T'C'A'T'G'C'A'T' | 430 | 444 |
| h431-445 | G'G'A'C'C'T'A'T'T'C'A'T'G'C'A' | 431 | 445 |

[Table 1-6]

| Sequence name | Antisense oligonucleotide sequence (5'-3') | hRPS25 target region (SEQ ID NO: 1) | |
|---|---|---|---|
| | | 5' end position | 3' end position |
| h432-446 | T'G'G'A'C'C'T'A'T'T'C'A'T'G'C' | 432 | 446 |
| h433-447 | T'T'G'G'A'C'C'T'A'T'T'C'A'T'G' | 433 | 447 |
| h434-448 | G'T'T'G'G'A'C'C'T'A'T'T'C'A'T' | 434 | 448 |
| h435-449 | G'G'T'T'G'G'A'C'C'T'A'T'T'C'A' | 435 | 449 |
| h436-450 | T'G'G'T'T'G'G'A'C'C'T'A'T'T'C' | 436 | 450 |
| h437-451 | C'T'G'G'T'T'G'G'A'C'C'T'A'T'T' | 437 | 451 |
| h438-452 | G'C'T'G'G'T'T'G'G'A'C'C'T'A'T' | 438 | 452 |

(continued)

| Sequence name | Antisense oligonucleotide sequence (5'-3') | hRPS25 target region (SEQ ID NO: 1) | |
|---|---|---|---|
| | | 5' end position | 3' end position |
| h439-453 | A'G'C'T'G'G'T'T'G'G'A'C'C'T'A' | 439 | 453 |
| h440-454 | C'A'G'C'T'G'G'T'T'G'G'A'C'C'T' | 440 | 454 |
| h441-455 | A'C'A'G'C'T'G'G'T'T'G'G'A'C'C' | 441 | 455 |
| h442-456 | T'A'C'A'G'C'T'G'G'T'T'G'G'A'C' | 442 | 456 |
| h443-457 | G'T'A'C'A'G'C'T'G'G'T'T'G'G'A' | 443 | 457 |
| h444-458 | T'G'T'A'C'A'G'C'T'G'G'T'T'G'G' | 444 | 458 |
| h445-459 | A'T'G'T'A'C'A'G'C'T'G'G'T'T'G' | 445 | 459 |
| h446-460 | A'A'T'G'T'A'C'A'G'C'T'G'G'T'T' | 446 | 460 |
| h447-461 | A'A'A'T'G'T'A'C'A'G'C'T'G'G'T' | 447 | 461 |
| h448-462 | C'A'A'A'T'G'T'A'C'A'G'C'T'G'G' | 448 | 462 |
| h449-463 | C'C'A'A'A'T'G'T'A'C'A'G'C'T'G' | 449 | 463 |
| h450-464 | T'C'C'A'A'A'T'G'T'A'C'A'G'C'T' | 450 | 464 |
| h451-465 | T'T'C'C'A'A'A'T'G'T'A'C'A'G'C' | 451 | 465 |
| h452-466 | T'T'T'C'C'A'A'A'T'G'T'A'C'A'G' | 452 | 466 |
| h453-467 | T'T'T'T'C'C'A'A'A'T'G'T'A'C'A' | 453 | 467 |
| h454-468 | T'T'T'T'T'C'C'A'A'A'T'G'T'A'C' | 454 | 468 |
| h455-469 | A'T'T'T'T'T'C'C'A'A'A'T'G'T'A' | 455 | 469 |

[Table 1-7]

| Sequence name | Antisense oligonucleotide sequence (5'-3') | hRPS25 target region (SEQ ID NO: 1) | |
|---|---|---|---|
| | | 5' end position | 3' end position |
| h123-141 | C'C'C'C'G'G'A'T'T'T'G'T'T'C'A'C'T'G'G' | 123 | 141 |
| h124-140 | C'C'C'G'G'A'T'T'T'G'T'T'C'A'C'T'G' | 124 | 140 |
| h125-140 | C'C'C'G'G'A'T'T'T'G'T'T'C'A'C'T' | 125 | 140 |
| h185-203 | G'A'C'T'A'A'G'T'T'A'T'T'G'A'G'C'T'T'G' | 185 | 203 |
| h186-201 | C'T'A'A'G'T'T'A'T'T'G'A'G'C'T'T' | 186 | 201 |
| h186-202 | A'C'T'A'A'G'T'T'A'T'T'G'A'G'C'T'T' | 186 | 202 |
| h186-203 | G'A'C'T'A'A'G'T'T'A'T'T'G'A'G'C'T'T' | 186 | 203 |
| h211-229 | T'T'A'T'C'A'T'A'G'G'T'A'G'C'T'T'T'G'T' | 211 | 229 |
| h212-228 | T'A'T'C'A'T'A'G'G'T'A'G'C'T'T'T'G' | 212 | 228 |
| h213-228 | T'A'T'C'A'T'A'G'G'T'A'G'C'T'T'T' | 213 | 228 |
| h214-229 | T'T'A'T'C'A'T'A'G'G'T'A'G'C'T'T' | 214 | 229 |
| h259-274 | A'C'A'G'C'T'G'G'G'G'T'T'A'T'A'A' | 259 | 274 |
| h263-279 | A'G'A'C'C'A'C'A'G'C'T'G'G'G'G'T'T' | 263 | 279 |
| h264-280 | G'A'G'A'C'C'A'C'A'G'C'T'G'G'G'G'T' | 264 | 280 |
| h265-279 | A'G'A'C'C'A'C'A'G'C'T'G'G'G'G' | 265 | 279 |

(continued)

| Sequence name | Antisense oligonucleotide sequence (5'-3') | hRPS25 target region (SEQ ID NO: 1) | |
|---|---|---|---|
| | | 5' end position | 3' end position |
| h266-280 | G'A'G'A'C'C'A'C'A'G'C'T'G'G'G' | 266 | 280 |
| h267-281 | A'G'A'G'A'C'C'A'C'A'G'C'T'G'G' | 267 | 281 |
| h268-282 | C'A'G'A'G'A'C'C'A'C'A'G'C'T'G' | 268 | 282 |
| h295-311 | G'G'C'C'A'G'G'G'A'G'C'C'T'C'G'A'A' | 295 | 311 |
| h296-311 | G'G'C'C'A'G'G'G'A'G'C'C'T'C'G'A' | 296 | 311 |
| h300-316 | G'C'C'C'T'G'G'C'C'A'G'G'G'A'G'C'C' | 300 | 316 |
| h301-315 | C'C'C'T'G'G'C'C'A'G'G'G'A'G'C' | 301 | 315 |
| h302-316 | G'C'C'C'T'G'G'C'C'A'G'G'G'A'G' | 302 | 316 |
| h303-317 | T'G'C'C'C'T'G'G'C'C'A'G'G'G'A' | 303 | 317 |
| h304-318 | C'T'G'C'C'C'T'G'G'C'C'A'G'G'G' | 304 | 318 |

[Table 1-8]

| Sequence name | Antisense oligonucleotide sequence (5'-3') | hRPS25 target region (SEQ ID NO: 1) | |
|---|---|---|---|
| | | 5' end position | 3' end position |
| h322-338 | A'C'T'A'A'G'G'A'G'C'T'C'C'T'G'A'A' | 322 | 338 |
| h324-342 | C'T'T'T'A'C'T'A'A'G'G'A'G'C'T'C'C'T'G' | 324 | 342 |
| h325-340 | T'T'A'C'T'A'A'G'G'A'G'C'T'C'C'T' | 325 | 340 |
| h325-341 | T'T'T'A'C'T'A'A'G'G'A'G'C'T'C'C'T' | 325 | 341 |
| h326-341 | T'T'T'A'C'T'A'A'G'G'A'G'C'T'C'C' | 326 | 341 |
| h429-445 | G'G'A'C'C'T'A'T'T'C'A'T'G'C'A'T'C' | 429 | 445 |
| h430-446 | T'G'G'A'C'C'T'A'T'T'C'A'T'G'C'A'T' | 430 | 446 |
| h434-450 | T'G'G'T'T'G'G'A'C'C'T'A'T'T'C'A'T' | 434 | 450 |
| h439-454 | C'A'G'C'T'G'G'T'T'G'G'A'C'C'T'A' | 439 | 454 |
| h439-455 | A'C'A'G'C'T'G'G'T'T'G'G'A'C'C'T'A' | 439 | 455 |
| h441-457 | G'T'A'C'A'G'C'T'G'G'T'T'G'G'A'C'C' | 441 | 457 |
| h442-457 | G'T'A'C'A'G'C'T'G'G'T'T'G'G'A'C' | 442 | 457 |
| h442-458 | T'G'T'A'C'A'G'C'T'G'G'T'T'G'G'A'C' | 442 | 458 |
| h442-459 | A'T'G'T'A'C'A'G'C'T'G'G'T'T'G'G'A'C' | 442 | 459 |
| h442-460 | A'A'T'G'T'A'C'A'G'C'T'G'G'T'T'G'G'A'C' | 442 | 460 |
| h443-458 | T'G'T'A'C'A'G'C'T'G'G'T'T'G'G'A' | 443 | 458 |
| h443-459 | A'T'G'T'A'C'A'G'C'T'G'G'T'T'G'G'A' | 443 | 459 |
| h443-460 | A'A'T'G'T'A'C'A'G'C'T'G'G'T'T'G'G'A' | 443 | 460 |
| h447-465 | T'T'C'C'A'A'A'T'G'T'A'C'A'G'C'T'G'G'T' | 447 | 465 |
| h448-465 | T'T'C'C'A'A'A'T'G'T'A'C'A'G'C'T'G'G' | 448 | 465 |
| h448-466 | T'T'T'C'C'A'A'A'T'G'T'A'C'A'G'C'T'G'G' | 448 | 466 |
| h449-465 | T'T'C'C'A'A'A'T'G'T'A'C'A'G'C'T'G' | 449 | 465 |

(continued)

| Sequence name | Antisense oligonucleotide sequence (5'-3') | hRPS25 target region (SEQ ID NO: 1) | |
|---|---|---|---|
| | | 5' end position | 3' end position |
| h449-466 | T'T'T'C'C'A'A'A'T'G'T'A'C'A'G'C'T'G' | 449 | 466 |
| h449-467 | T'T'T'T'C'C'A'A'A'T'G'T'A'C'A'G'C'T'G' | 449 | 467 |
| h450-465 | T'T'C'C'A'A'A'T'G'T'A'C'A'G'C'T' | 450 | 465 |

[Table 1-9]

| Sequence name | Antisense oligonucleotide sequence (5'-3') | hRPS25 target region (SEQ ID NO: 1) | |
|---|---|---|---|
| | | 5' end position | 3' end position |
| h450-466 | T'T'T'C'C'A'A'A'T'G'T'A'C'A'G'C'T' | 450 | 466 |
| h450-467 | T'T'T'T'C'C'A'A'A'T'G'T'A'C'A'G'C'T' | 450 | 467 |
| h450-468 | T'T'T'T'T'C'C'A'A'A'T'G'T'A'C'A'G'C'T' | 450 | 468 |
| h451-466 | T'T'T'C'C'A'A'A'T'G'T'A'C'A'G'C' | 451 | 466 |
| h451-467 | T'T'T'T'C'C'A'A'A'T'G'T'A'C'A'G'C' | 451 | 467 |
| h451-468 | T'T'T'T'T'C'C'A'A'A'T'G'T'A'C'A'G'C' | 451 | 468 |
| h451-469 | A'T'T'T'T'T'C'C'A'A'A'T'G'T'A'C'A'G'C' | 451 | 469 |

In Table 1-1 to Table 1-9 provided above and Table 2-1 to Table 2-5 provided below, each of symbol "A'", symbol "C'", symbol "G'", and symbol "T'" is selected from natural nucleosides (a, A, c, C, g, G, t, and U described below) or modified nucleosides (including sugar-modified nucleosides). As for the sugar-modified nucleosides, symbol "A'" is selected from A (M), A (m), A (L), A (Y), A (Gx), A (5'-CP), A (Mx), and A (S) described below; symbol "C'" is selected from 5 (x), C (M), 5 (m), 5 (L), 5 (Y), 5 (Gx), 5 (5'-CP), C (Mx), and 5 (S) described below; symbol "G'" is selected from G (M), G (m), G (L), G (Y), G (Gx), G (5'-CP), G (Mx), and G (S) described below; and symbol "T'" is selected from U (M), T (m), T (L), T (Y), T (Gx), T (5'-CP), U (Mx), and T (S) described below.

An aspect of the single-stranded antisense oligonucleotide according to the present invention is a single-stranded oligonucleotide that is capable of modulating expression of RPS25 gene and that has one of the base sequences shown in Table 2-1 to Table 2-5, where the single-stranded oligonucleotide is complementary to a target region in human RPS25 mRNA precursor shown in Table 2-1 to Table 2-5. As long as it includes a base sequence shown in Table 2-1 to Table 2-5, the single-stranded oligonucleotide may be longer by one to five bases toward the 3' end and/or the 5' end. It can be understood that the target region is a region of human RPS25 mRNA precursor that is associated with modulation of expression of human RPS25 gene in particular (such as, for example, a region that has an mRNA precursor secondary structure to which the antisense nucleotide can bind easily). For example, in Table 2-1, the 5' end position being "1" and the 3' end position being "15" mean that a base sequence from position 1 to position 15 of the base sequence as set forth in SEQ ID NO: 2 counted from the 5' end thereof is the target region in human RPS25 mRNA precursor targeted by the corresponding single-stranded antisense oligonucleotide (sequence name: "hp1-15").

[Table 2-1]

| Sequence name | Antisense oligonucleotide sequence (5'-3') | hpRPS25 target region (SEQ ID NO: 2) | |
|---|---|---|---|
| | | 5' end position | 3' end position |
| hp1-15 | C'G'G'A'C'A'A'A'A'A'G'G'A'A'G' | 1 | 15 |
| hp72-86 | T'T'C'A'C'A'C'C'C'C'T'G'A'A'G' | 72 | 86 |
| hp73-87 | C'T'T'C'A'C'A'C'C'C'C'T'G'A'A' | 73 | 87 |
| hp74-88 | A'C'T'T'C'A'C'A'C'C'C'C'T'G'A' | 74 | 88 |
| hp75-89 | G'A'C'T'T'C'A'C'A'C'C'C'C'T'G' | 75 | 89 |

(continued)

| Sequence name | Antisense oligonucleotide sequence (5’-3’) | hpRPS25 target region (SEQ ID NO: 2) | |
|---|---|---|---|
| | | 5’ end position | 3’ end position |
| hp76-90 | C’G’A’C’T’T’C’A’C’A’C’C’C’C’T’ | 76 | 90 |
| hp231-245 | T’C’C’C’C’T’A’A’T’A’C’T’G’C’G’ | 231 | 245 |
| hp232-246 | G’T’C’C’C’C’T’A’A’T’A’C’T’G’C’ | 232 | 246 |
| hp233-247 | A’G’T’C’C’C’C’T’A’A’T’A’C’T’G’ | 233 | 247 |
| hp261-275 | A’T’G’C’A’A’C’C’A’G’G’T’C’C’T’ | 261 | 275 |
| hp262-276 | A’A’T’G’C’A’A’C’C’A’G’G’T’C’C’ | 262 | 276 |
| hp278-292 | G’T’A’G’G’A’G’G’G’C’A’G’C’G’G’ | 278 | 292 |
| hp279-293 | T’G’T’A’G’G’A’G’G’G’C’A’G’C’G’ | 279 | 293 |
| hp280-294 | C’T’G’T’A’G’G’A’G’G’G’C’A’G’C’ | 280 | 294 |
| hp390-404 | G’G’T’C’T’T’A’T’T’T’C’T’A’C’C’ | 390 | 404 |
| hp392-406 | G’A’G’G’T’C’T’T’A’T’T’T’C’T’A’ | 392 | 406 |
| hp417-431 | A’G’A’G’T’T’A’C’C’C’C’T’A’G’T’ | 417 | 431 |
| hp418-432 | G’A’G’A’G’T’T’A’C’C’C’C’T’A’G’ | 418 | 432 |
| hp419-433 | A’G’A’G’A’G’T’T’A’C’C’C’C’T’A’ | 419 | 433 |
| hp420-434 | G’A’G’A’G’A’G’T’T’A’C’C’C’C’T’ | 420 | 434 |
| hp421-435 | C’G’A’G’A’G’A’G’T’T’A’C’C’C’C’ | 421 | 435 |
| hp422-436 | A’C’G’A’G’A’G’A’G’T’T’A’C’C’C’ | 422 | 436 |
| hp423-437 | T’A’C’G’A’G’A’G’A’G’T’T’A’C’C’ | 423 | 437 |
| hp445-459 | A’A’G’T’T’A’C’C’T’A’T’T’A’C’T’ | 445 | 459 |
| hp446-460 | C’A’A’G’T’T’A’C’C’T’A’T’T’A’C’ | 446 | 460 |

[Table 2-2]

| Sequence name | Antisense oligonucleotide sequence (5’-3’) | hpRPS25 target region (SEQ ID NO: 2) | |
|---|---|---|---|
| | | 5’ end position | 3’ end position |
| hp447-461 | A’C’A’A’G’T’T’A’C’C’T’A’T’T’A’ | 447 | 461 |
| hp448-462 | T’A’C’A’A’G’T’T’A’C’C’T’A’T’T’ | 448 | 462 |
| hp458-472 | C’C’A’C’T’T’A’C’T’A’T’A’C’A’A’ | 458 | 472 |
| hp460-474 | A’A’C’C’A’C’T’T’A’C’T’A’T’A’C’ | 460 | 474 |
| hp461-475 | A’A’A’C’C’A’C’T’T’A’C’T’A’T’A’ | 461 | 475 |
| hp510-524 | G’A’C’G’G’G’A’A’G’A’T’A’A’A’C’ | 510 | 524 |
| hp561-575 | T’C’G’C’A’C’A’A’C’A’G’A’C’C’C’ | 561 | 575 |
| hp562-576 | T’T’C’G’C’A’C’A’A’C’A’G’A’C’C’ | 562 | 576 |
| hp589-603 | T’A’A’C’A’C’A’G’C’A’G’G’C’A’C’ | 589 | 603 |
| hp605-619 | C’T’A’G’A’T’C’A’G’T’T’A’A’A’A’ | 605 | 619 |
| hp606-620 | A’C’T’A’G’A’T’C’A’G’T’T’A’A’A’ | 606 | 620 |
| hp626-640 | T’C’A’A’A’C’A’G’G’G’G’C’C’G’A’ | 626 | 640 |

(continued)

| Sequence name | Antisense oligonucleotide sequence (5'-3') | hpRPS25 target region (SEQ ID NO: 2) | |
|---|---|---|---|
| | | 5' end position | 3' end position |
| hp627-641 | T'T'C'A'A'A'C'A'G'G'G'G'C'C'G' | 627 | 641 |
| hp628-642 | C'T'T'C'A'A'A'C'A'G'G'G'G'C'C' | 628 | 642 |
| hp629-643 | C'C'T'T'C'A'A'A'C'A'G'G'G'G'C' | 629 | 643 |
| hp632-646 | T'G'G'C'C'T'T'C'A'A'A'C'A'G'G' | 632 | 646 |
| hp633-647 | T'T'G'G'C'C'T'T'C'A'A'A'C'A'G' | 633 | 647 |
| hp634-648 | T'T'T'G'G'C'C'T'T'C'A'A'A'C'A' | 634 | 648 |
| hp654-668 | A'A'A'A'A'A'A'A'C'A'C'C'G'A'C' | 654 | 668 |
| hp681-695 | A'A'T'T'A'C'A'C'A'T'T'A'C'T'A' | 681 | 695 |
| hp696-710 | C'C'G'T'T'A'T'C'A'A'G'G'A'T'A' | 696 | 710 |
| hp697-711 | A'C'C'G'T'T'A'T'C'A'A'G'G'A'T' | 697 | 711 |
| hp761-775 | G'T'T'A'G'T'A'T'T'T'C'T'G'G'C' | 761 | 775 |
| hp762-776 | A'G'T'T'A'G'T'A'T'T'T'C'T'G'G' | 762 | 776 |
| hp764-778 | C'A'A'G'T'T'A'G'T'A'T'T'T'C'T' | 764 | 778 |

[Table 2-3]

| Sequence name | Antisense oligonucleotide sequence (5'-3') | hpRPS25 target region (SEQ ID NO: 2) | |
|---|---|---|---|
| | | 5' end position | 3' end position |
| hp1034-1048 | A'T'G'C'T'T'A'A'C'A'T'G'G'T'C' | 1034 | 1048 |
| hp1035-1049 | G'A'T'G'C'T'T'A'A'C'A'T'G'G'T' | 1035 | 1049 |
| hp1103-1117 | T'T'A'C'T'A'A'C'A'G'C'C'A'A'T' | 1103 | 1117 |
| hp1104-1118 | A'T'T'A'C'T'A'A'C'A'G'C'C'A'A' | 1104 | 1118 |
| hp1105-1119 | C'A'T'T'A'C'T'A'A'C'A'G'C'C'A' | 1105 | 1119 |
| hp1106-1120 | A'C'A'T'T'A'C'T'A'A'C'A'G'C'C' | 1106 | 1120 |
| hp1107-1121 | T'A'C'A'T'T'A'C'T'A'A'C'A'G'C' | 1107 | 1121 |
| hp1108-1122 | T'T'A'C'A'T'T'A'C'T'A'A'C'A'G' | 1108 | 1122 |
| hpl110-1124 | A'A'T'T'A'C'A'T'T'A'C'T'A'A'C' | 1110 | 1124 |
| hp1128-1142 | T'C'A'G'G'A'G'T'A'A'G'A'C'G'T' | 1128 | 1142 |
| hp1129-1143 | A'T'C'A'G'G'A'G'T'A'A'G'A'C'G' | 1129 | 1143 |
| hp1196-1210 | G'C'T'T'C'A'C'T'A'A'A'C'T'G'C' | 1196 | 1210 |
| hp1197-1211 | G'G'C'T'T'C'A'C'T'A'A'A'C'T'G' | 1197 | 1211 |
| hp1217-1231 | C'G'A'A'A'C'A'T'A'A'A'A'G'A'T' | 1217 | 1231 |
| hp1218-1232 | T'C'G'A'A'A'C'A'T'A'A'A'A'G'A' | 1218 | 1232 |
| hp1219-1233 | C'T'C'G'A'A'A'C'A'T'A'A'A'A'G' | 1219 | 1233 |
| hp1398-1412 | G'T'G'G'A'A'T'T'A'T'G'G'T'A'A' | 1398 | 1412 |
| hp1399-1413 | T'G'T'G'G'A'A'T'T'A'T'G'G'T'A' | 1399 | 1413 |
| hp1402-1416 | T'A'T'T'G'T'G'G'A'A'T'T'A'T'G' | 1402 | 1416 |

(continued)

| Sequence name | Antisense oligonucleotide sequence (5'-3') | hpRPS25 target region (SEQ ID NO: 2) | |
|---|---|---|---|
| | | 5' end position | 3' end position |
| hp1408-1422 | C'C'T'T'A'T'T'A'T'T'G'T'G'G'A' | 1408 | 1422 |
| hp1409-1423 | G'C'C'T'T'A'T'T'A'T'T'G'T'G'G' | 1409 | 1423 |
| hp1410-1424 | A'G'C'C'T'T'A'T'T'A'T'T'G'T'G' | 1410 | 1424 |
| hp1411-1425 | G'A'G'C'C'T'T'A'T'T'A'T'T'G'T' | 1411 | 1425 |
| hp1412-1426 | T'G'A'G'C'C'T'T'A'T'T'A'T'T'G' | 1412 | 1426 |
| hp1478-1492 | C'G'T'T'G'A'T'T'C'A'C'C'C'G'C' | 1478 | 1492 |

[Table 2-4]

| Sequence name | Antisense oligonucleotide sequence (5'-3') | hpRPS25 target region (SEQ ID NO: 2) | |
|---|---|---|---|
| | | 5' end position | 3' end position |
| hp1480-1494 | T'C'C'G'T'T'G'A'T'T'C'A'C'C'C' | 1480 | 1494 |
| hp1715-1729 | G'C'T'C'C'A'T'T'A'T'C'T'T'C'C' | 1715 | 1729 |
| hp1749-1763 | C'G'A'T'C'A'T'C'T'A'T'C'C'T'T' | 1749 | 1763 |
| hp1750-1764 | A'C'G'A'T'C'A'T'C'T'A'T'C'C'T' | 1750 | 1764 |
| hp1751-1765 | A'A'C'G'A'T'C'A'T'C'T'A'T'C'C' | 1751 | 1765 |
| hp1763-1777 | A'T'C'C'T'A'G'T'T'T'T'T'A'A'C' | 1763 | 1777 |
| hp1793-1807 | A'T'T'G'C'T'T'A'A'T'C'T'G'A'C' | 1793 | 1807 |
| hp1885-1899 | A'T'G'G'T'C'T'T'A'A'A'A'C'T'C' | 1885 | 1899 |
| hp1887-1901 | G'A'A'T'G'G'T'C'T'T'A'A'A'A'C' | 1887 | 1901 |
| hp2047-2061 | G'T'T'T'G'T'T'T'T'G'G'C'C'G'G' | 2047 | 2061 |
| hp2048-2062 | G'G'T'T'T'G'T'T'T'T'G'G'C'C'G' | 2048 | 2062 |
| hp2049-2063 | A'G'G'T'T'T'G'T'T'T'T'G'G'C'C' | 2049 | 2063 |
| hp2121-2135 | G'A'A'T'T'G'G'T'G'G'T'T'G'C'A' | 2121 | 2135 |
| hp2122-2136 | G'G'A'A'T'T'G'G'T'G'G'T'T'G'C' | 2122 | 2136 |
| hp2123-2137 | A'G'G'A'A'T'T'G'G'T'G'G'T'T'G' | 2123 | 2137 |
| hp2124-2138 | C'A'G'G'A'A'T'T'G'G'T'G'G'T'T' | 2124 | 2138 |
| hp2260-2274 | G'G'T'A'A'G'G'A'G'T'T'G'C'A'C' | 2260 | 2274 |
| hp2261-2275 | A'G'G'T'A'A'G'G'A'G'T'T'G'C'A' | 2261 | 2275 |
| hp2262-2276 | G'A'G'G'T'A'A'G'G'A'G'T'T'G'C' | 2262 | 2276 |
| hp2268-2282 | T'A'G'C'T'T'G'A'G'G'T'A'A'G'G' | 2268 | 2282 |
| hp2269-2283 | A'T'A'G'C'T'T'G'A'G'G'T'A'A'G' | 2269 | 2283 |
| hp2271-2285 | A'G'A'T'A'G'C'T'T'G'A'G'G'T'A' | 2271 | 2285 |
| hp2277-2291 | A'C'G'G'G'C'A'G'A'T'A'G'C'T'T' | 2277 | 2291 |
| hp2339-2353 | G'T'A'A'G'G'T'T'T'T'T'G'G'C'T' | 2339 | 2353 |
| hp2341-2355 | T'A'G'T'A'A'G'G'T'T'T'T'T'G'G' | 2341 | 2355 |

[Table 2-5]

| Sequence name | Antisense oligonucleotide sequence (5'-3') | hpRPS25 target region (SEQ ID NO: 2) | |
|---|---|---|---|
| | | 5' end position | 3' end position |
| hp2342-2356 | C'T'A'G'T'A'A'G'G'T'T'T'T'T'G' | 2342 | 2356 |
| hp2386-2400 | A'G'A'G'A'A'T'A'G'C'A'C'G'A'T' | 2386 | 2400 |
| hp2406-2420 | G'T'T'T'G'A'T'A'A'G'T'C'C'T'A' | 2406 | 2420 |
| hp2538-2552 | T'T'T'G'T'A'T'C'T'A'C'C'T'C'C' | 2538 | 2552 |
| hp2540-2554 | G'C'T'T'T'G'T'A'T'C'T'A'C'C'T' | 2540 | 2554 |
| hp2541-2555 | A'G'C'T'T'T'G'T'A'T'C'T'A'C'C' | 2541 | 2555 |
| hp2585-2599 | C'T'G'G'T'T'G'G'A'C'C'T'G'T'A' | 2585 | 2599 |
| hp2586-2600 | G'C'T'G'G'T'T'G'G'A'C'C'T'G'T' | 2586 | 2600 |
| hp25 87-2601 | A'G'C'T'G'G'T'T'G'G'A'C'C'T'G' | 2587 | 2601 |
| hp2583-2597 | G'G'T'T'G'G'A'C'C'T'G'T'A'A'A' | 2583 | 2597 |
| hp2584-2598 | T'G'G'T'T'G'G'A'C'C'T'G'T'A'A' | 2584 | 2598 |

In an aspect of the present embodiment, preferably, the base sequence of the single-stranded antisense oligonucleotide is a base sequence selected from the group consisting of base sequences as set forth in SEQ ID NOs: 7, 9, 11 to 12, 17 to 19, 23 to 25, 27 to 29, 31, 33, 36 to 38, 46 to 50, 52 to 53, 58 to 61, 63 to 68, 70, 73 to 74, 79 to 81, 84 to 107, 111, 113 to 130, 136, 140, 143, 148, 150, 159, 161 to 162, 169 to 173, 183 to 186, 188 to 190, 203, 208 to 212, 229, 232 to 234, 238, 298 to 300, 303 to 313, 317, 319, 321 to 323, 326 to 338, 340 to 349, 351 to 367, 370 to 382, 385 to 398, 400 to 411, 413, 415, 416, 418 to 427, and 430 to 432.

In an aspect of the present embodiment, more preferably, the base sequence of the single-stranded antisense oligonucleotide is a base sequence selected from the group consisting of base sequences as set forth in SEQ ID NOs: 18, 24 to 25, 28 to 29, 38, 48 to 49, 53, 58 to 59, 63 to 64, 66 to 68, 79 to 80, 84, 86 to 88, 91, 93 to 95, 97, 99, 101 to 105, 113 to 119, 121 to 123, 125, 127 to 130, 140, 162, 169, 171 to 173, 183, 188, 190, 304 to 306, 309, 310, 312, 313, 317, 321 to 323, 326, 327, 331, 332, 334, 337, 340 to 342, 344, 346, 348, 349, 351, 353, 355 to 364, 366 to 367, 371 to 382, 385, 386, 388, 389, 391, 394, 396, 397, 407, 408, 410, 418 to 424, 426, 427, 431, and 432.

In an aspect of the present embodiment, further preferably, the base sequence of the single-stranded antisense oligonucleotide is a base sequence selected from the group consisting of base sequences as set forth in SEQ ID NOs: 24 to 25, 28, 64, 80, 91, 94, 97, 113 to 114, 116, 119, 127, 129 to 130, 162, 172, 183, 305, 306, 309, 312, 322, 323, 326, 331, 340, 341, 346, 348, 349, 351, 355, 358 to 361, 363, 366, 367, 372 to 379, 381, 382, 386, 394, 397, 419, 421 to 424, 426, 431, and 432.

<Pharmacologically Acceptable Salt>

The single-stranded antisense oligonucleotide according to the present embodiment may be in the form of a pharmacologically acceptable salt. Herein, the "pharmacologically acceptable salt" means a salt of the single-stranded antisense oligonucleotide according to the present invention that is a physiologically acceptable salt of the single-stranded antisense oligonucleotide according to the present invention, more specifically, a salt of the single-stranded antisense oligonucleotide that has a desirable biological activity and that does not have any undesirable toxicological effect. The same is true for a double-stranded antisense oligonucleotide and an antisense oligonucleotide complex, which are described below.

<Pharmaceutically Acceptable Salt>

In an aspect of the present embodiment, the single-stranded antisense oligonucleotide may be in the form of a pharmaceutically acceptable salt. Herein, the "pharmaceutically acceptable salt" means the pharmacologically acceptable salt as described above in the form of an acid addition salt or a base addition salt. Examples of the acid addition salt include inorganic acid salts such as hydrochloride, hydrobromide, sulfate, hydriodide, nitrate, and phosphate, as well as organic acid salts such as citrate, oxalate, phthalate, fumarate, maleate, succinate, malate, acetate, formate,

propionate, benzoate, trifluoroacetate, methanesulfonate, benzenesulfonate, para-toluenesulfonate, and camphorsulfonate. Examples of the base addition salt include inorganic base salts such as sodium salt, potassium salt, calcium salt, magnesium salt, barium salt, and aluminum salt, as well as organic base salts such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N,N-dibenzylethylamine, and the like. Further examples thereof include salts with basic amino acids or acidic amino acids such as arginine, lysine, ornithine, aspartic acid, and glutamic acid (amino acid salts). The same is true for a double-stranded antisense oligonucleotide and an antisense oligonucleotide complex, which are described below.

<Structure of Single-Stranded Antisense Oligonucleotide>

The single-stranded antisense oligonucleotide according to the present embodiment includes a gap region, a 3’ wing region bonded to a 3’ end of the gap region, and a 5’ wing region bonded to a 5’ end of the gap region (see Fig. 1, for example). The single-stranded antisense oligonucleotide is preferably in the form of a single strand. In an aspect of the present embodiment, the single-stranded antisense oligonucleotide may be hybridized with a second oligonucleotide (which is described below) to form a double strand (a double-stranded antisense oligonucleotide). Preferably, the base sequence of the second oligonucleotide is a base sequence with a sequence identity of 90% to 100% to a base sequence complementary to the base sequence of the single-stranded antisense oligonucleotide.

The single-stranded antisense oligonucleotide is a so-called gapmer-type single-stranded antisense oligonucleotide. A gapmer-type single-stranded antisense oligonucleotide inhibits the function of the target RNA by the mechanism described below. Firstly, the single-stranded antisense oligonucleotide binds to the target region of the target RNA (in Fig. 2, top and center). Then, RNaseH, which is an RNase, recognizes the complex of the single-stranded antisense oligonucleotide and the target RNA, and binds to it (in Fig. 2, center). Subsequently, due to the RNaseH-driven enzymatic degradation reaction, the target RNA is cleaved and degraded. At this point, the single-stranded antisense oligonucleotide is not affected by the RNaseH-driven enzymatic degradation (in Fig. 2, bottom). As a result, the single-stranded antisense oligonucleotide can bind to another target RNA and cleave and degrade this RNA. Because a gapmer-type single-stranded antisense oligonucleotide functions as a catalyst in the above RNaseH-driven enzymatic degradation reaction, administration of only a small amount is expected to exhibit a certain level of sustained effect.

Moreover, the single-stranded antisense oligonucleotide, in the present embodiment, can be suitably used for modulating expression of RPS25 gene by the above-described mechanism (including the case where it acts via modulation of maturing of RPS25 mRNA precursor. Further, according to the present embodiment, even via intrathecal injection, which is a route of administration usually adopted in clinical settings, the effect of modulating RPS25 gene expression of the single-stranded antisense oligonucleotide can be exhibited. Herein, "modulating RPS25 gene expression" means, at least, inhibition of expression of RPS25 gene, and, as a result, it means, at least, inhibition of function of RPS25 protein (such as RAN translation).

(Gap Region)

The gap region is preferably a 5- to 20-mer deoxyribose-based nucleic acid optionally including a nucleic acid having a modified sugar moiety. In other words, the gap region is a 5- to 20-mer nucleic acid including deoxyribose whose sugar moiety may be modified. Another way of understanding it is that the gap region is composed of one of or both a natural nucleotide and a non-natural nucleotide of 5- to 20-mer whose sugar moiety is deoxyribose. Because its sugar moiety is deoxyribose or modified deoxyribose, the gap region is capable of forming, together with the target RNA (RPS25 mRNA and/or the like), a complex that can be recognized by RNaseH. Here, examples of the nucleic acid including modified deoxyribose include 5’-CP nucleic acid.

The gap region preferably has a base count of 5- to 20-mer, more preferably 6-to 17-mer, further preferably 7- to 13-mer, further more preferably 7- to 11-mer.

Examples of the natural nucleotide whose sugar moiety is deoxyribose include deoxyadenosine monophosphate, deoxyguanosine monophosphate, thymidine monophosphate, deoxycytidine monophosphate, deoxy-5-methylcytidine monophosphate (also called 5-methyldeoxycytidine), and the like. In other words, examples of the natural nucleotide constituting the gap region include those including structural formulae corresponding to each of the symbols a, g, t, and c described below.

Examples of the non-natural nucleotide whose sugar moiety is deoxyribose or modified deoxyribose include 5’-CP nucleic acid, 2-thio-thymidine monophosphate, 2-aminoadenosine monophosphate, 7-deazaguanosine monophosphate, and the like.

As long as the effect of the present invention is exhibited, the gap region may be a natural nucleotide whose sugar moiety is deoxyribose where part of the sugar moiety is a modified sugar. In other words, in an aspect of the present embodiment, the gap region may be a nucleic acid in which part of the sugar moiety is deoxyribose and the

other part of the sugar moiety is a modified sugar (such as a modified deoxyribose, for example).

(3' Wing Region)

The 3' wing region is a modified nucleic acid. Another way of understanding it is that the 3' wing region is composed of a modified nucleotide. Preferably, the modified nucleic acid of the 3' wing region includes at least one selected from the group consisting of 2'-modified nucleic acids (2'-O-methyl nucleic acid, 2'-MOE nucleic acid, and MCE nucleic acid) and bridged nucleic acids (LNA, AmNA, GuNA, and scpBNA). When the 3' wing region described above and the 5' wing region described below are composed of these particular modified nucleotides, high binding affinity for the target RNA is expected to be obtained and thereby the function of the target RNA can be effectively reduced. In an aspect of the present embodiment, the 3' wing region may be a modified nucleic acid whose sugar moiety is a modified sugar. Examples of the modified nucleic acid whose sugar moiety is a modified sugar include those mentioned above in the section (Sugar Modification, Modified Sugar). In an aspect of the present embodiment, the modified nucleic acid of the 3' wing region may be composed of 2'-MOE nucleic acid alone. The 3' wing region in one single-stranded antisense oligonucleotide may include a plurality of types of modified nucleic acids.

The 3' wing region preferably has a base count of 1- to 5-mer, more preferably 2- to 5-mer, further preferably 2- to 4-mer, further more preferably 3- to 4-mer.

(5' Wing Region)

The 5' wing region is a modified nucleic acid. Another way of understanding it is that the 5' wing region is composed of a modified nucleotide. Preferably, the modified nucleic acid of the 5' wing region includes at least one selected from the group consisting of 2'-modified nucleic acids (2'-O-methyl nucleic acid, 2'-MOE nucleic acid, and MCE nucleic acid) and bridged nucleic acids (LNA, AmNA, GuNA, and scpBNA). In an aspect of the present embodiment, the 5' wing region may be a modified nucleic acid whose sugar moiety is a modified sugar. Examples of the modified nucleic acid whose sugar moiety is a modified sugar include those mentioned above in the section (Sugar Modification, Modified Sugar). In an aspect of the present embodiment, the modified nucleic acid of the 5' wing region may be composed of 2'-MOE nucleic acid alone. The 5' wing region in one single-stranded antisense oligonucleotide may include a plurality of types of modified nucleic acids. In another aspect of the present embodiment, the modified nucleic acid of the 3' wing region and the 5' wing region may be composed of 2'-MOE nucleic acid alone.

The 5' wing region preferably has a base count of 1- to 5-mer, more preferably 2- to 5-mer, further preferably 2- to 4-mer.

In an aspect of the present embodiment, preferably, the gap region has a base count of 6- to 17-mer, the 3' wing region has a base count of 2- to 4-mer, and the 5' wing region has a base count of 2- to 4-mer.

In an aspect of the present embodiment, more preferably, the gap region has a base count of 7- to 13-mer, the 3' wing region has a base count of 2- to 4-mer, and the 5' wing region has a base count of 2- to 4-mer.

In an aspect of the present embodiment, further preferably, the gap region has a base count of 7- to 11-mer, the 3' wing region has a base count of 2- to 4-mer, and the 5' wing region has a base count of 2- to 4-mer.

(Other Configurations)

In an aspect of the present embodiment, the single-stranded antisense oligonucleotide may further include a natural nucleotide bonded to the 3' end of the 3' wing region. The base count of the natural nucleotide bonded to the 3' end of the 3' wing region may be one or several, and may be one.

(Description Style for Gapmer-Type Structure)

The single-stranded antisense oligonucleotide according to the present invention is of gapmer-type. For describing the structure of the gapmer-type, "X-Y-Z" or "X-Y-Z-W" may also be used. In these description styles, "X" represents the base count of the 5' wing region, "Y" represents the base count of the gap region, "Z" represents the base count of the 3' wing region, and "W" represents the base count of the natural nucleoside bonded to the 3' end of the 3' wing region.

Examples of "X-Y-Z" include 2-8-4, 2-8-3, 2-8-5, 2-9-2, 2-9-3, 2-9-4, 2-9-5, 2-10-3, 2-10-4, 2-10-5, 2-11-3, 2-11-4, 2-11-5, 2-12-3, 2-12-4, 2-12-5, 3-8-2, 3-8-3, 3-8-4, 3-8-5, 3-9-3, 3-9-4, 3-9-5, 3-10-3, 3-10-4, 3-10-5, 3-11-3, 3-11-4, 3-11-5, 3-12-3, 3-12-4, 3-12-5, 3-13-3, 4-8-2, 4-8-3, 4-8-4, 4-8-5, 4-9-3, 4-9-4, 4-9-5, 4-10-3, 4-10-4, 4-10-5, 4-11-2, 4-11-3, 4-11-4, 4-11-5, 5-8-2, 5-8-3, 5-8-4, 5-8-5, 5-9-2, 5-9-3, 5-9-4, 5-9-5, 5-10-2, 5-10-3, 5-10-4, 5-10-5, 5-11-2, 5-11-3, 5-11-4, 5-11-5, and the like. For example, "2-8-4" means that the 5' wing region is a 2-mer oligonucleotide, the gap region is an 8-mer oligonucleotide, and the 3' wing region is a 4-mer oligonucleotide.

Examples of "X-Y-Z-W" include 2-8-4-1, 2-8-3-1, 2-8-5-1, 2-9-2-1, 2-9-3-1, 2-9-4-1, 2-9-5-1, 2-10-3-1, 2-10-4-1, 2-10-5-1, 2-11-3-1, 2-11-4-1, 2-11-5-1, 2-12-3-1, 2-12-4-1, 2-12-5-1, 3-8-2-1, 3-8-3-1, 3-8-4-1, 3-8-5-1, 3-9-2-1, 3-9-3-1, 3-9-4-1, 3-9-5-1, 3-10-3-1, 3-10-4-1, 3-10-5-1, 3-11-3-1, 3-11-4-1, 3-11-5-1, 3-12-3-1, 3-12-4-1, 3-12-5-1, 4-8-2-1, 4-8-3-1, 4-8-4-1, 4-8-5-1, 4-9-3-1, 4-9-4-1, 4-9-5-1, 4-10-3-1, 4-10-4-1, 4-10-5-1, 4-11-2-1, 4-11-3-1, 4-11-4-1, 4-11-5-1, 5-8-2-1, 5-8-3-1, 5-8-4-1, 5-8-5-1, 5-9-2-1, 5-9-3-1, 5-9-4-1, 5-9-5-1, 5-10-2-1, 5-10-3-1, 5-10-4-1, 5-10-5-1, 5-11-2-1, 5-11-3-1, 5-11-4-1, 5-11-5-1, and the like. For example, 2-8-4-1 means that the 5' wing region is a 2-mer oligonucleotide, the gap region is an 8-mer oligonucleotide, the 3' wing region is a 4-mer oligonucleotide, and the natural nucleoside bonded to the 3' end of the 3' wing region is of one nucleotide.

The single-stranded antisense oligonucleotide according to the present invention has a base length of 12- to 30-mer, preferably 12- to 22-mer, more preferably 14- to 20-mer, further preferably 14- to 18-mer, particularly preferably 15- to 17-mer. When the single-stranded antisense oligonucleotide according to the present invention has a base length of 12- to 22-mer, 14- to 20-mer, 14- to 18-mer, or 15- to 17-mer, the binding to RPS25 mRNA or the binding to RPS25 mRNA precursor is particularly strong, allowing for effective modulation of RPS25 gene expression. It should be noted that when a natural nucleotide is further bonded to the 3' end of the 3' wing region, the base length count of the antisense oligonucleotide includes the base count of the natural nucleoside.

In the present embodiment, nucleosides of the single-stranded antisense oligonucleotide are bonded to each other via a phosphate group and/or a modified phosphate group, preferably via a phosphodiester bond or a phosphorothioate bond.

An aspect of the single-stranded antisense oligonucleotide according to the present invention is a gapmer-type single-stranded antisense oligonucleotide that has a 5- to 20-mer gap region, a 2- to 5-mer 5' wing region, and a 2- to 5-mer 3' wing region. The gap region is positioned between the 5' wing region and the 3' wing region. Preferably, each of the 5' wing region and the 3' wing region includes 2'-MOE nucleic acid, LNA, AmNA, GuNA, or scpBNA, in a number of at least one. Each of the 5' wing region and the 3' wing region may include 2'-O-alkylated nucleotide or 2'-F nucleotide. As the 2'-O-alkylated nucleotide, a nucleotide having 2'-O-alkylated (such as 2'-O-methylated, for example) D-ribofuranose may be used. The gapmer-type single-stranded antisense oligonucleotide may be hybridized with a second oligonucleotide to form a double strand.

<Double-Stranded Antisense Oligonucleotide>

The double-stranded antisense oligonucleotide according to the present embodiment is a double-stranded antisense oligonucleotide or a pharmaceutically acceptable salt thereof comprising:

the single-stranded antisense oligonucleotide; and
a second oligonucleotide hybridized to the single-stranded antisense oligonucleotide. Preferably, the base sequence of the second oligonucleotide is a base sequence with a sequence identity of 90% to 100% to a base sequence complementary to the base sequence of the single-stranded antisense oligonucleotide.

The double-stranded antisense oligonucleotide may dissociate in a solution to separate into the single-stranded antisense oligonucleotide and the second oligonucleotide. After separation, the single-stranded antisense oligonucleotide is capable of binding to the above-described target RNA. The single-stranded antisense oligonucleotide may also be understood as "a first oligonucleotide" from its relationship with the second oligonucleotide. An antisense strand for the above-described target RNA is present only in the first oligonucleotide among the constituent oligonucleotides of the double-stranded antisense oligonucleotide, but, for the sake of convenience, the double-stranded oligonucleotide consisting of the first oligonucleotide and the second oligonucleotide is called "a double-stranded antisense oligonucleotide".

<<Method of Producing Single-Stranded Antisense Oligonucleotide>>

The single-stranded antisense oligonucleotide according to the present invention can be produced by solid phase synthesis by means of a phosphoramidite method. Firstly, with the use of a commercially-available automatic nucleic-acid synthesizer, for example, a single-stranded oligonucleotide having a certain base sequence is synthesized on a solid support. Then, with the use of a basic substance and/or the like, the single-stranded oligonucleotide thus synthesized is removed from the solid support, followed by deprotection, and thereby a crude single-stranded oligonucleotide is obtained. Subsequently, the resulting crude single-stranded oligonucleotide is purified by HPLC and/or the like. This production method is not the only method to produce the single-stranded antisense oligonucleotide according to the present invention; the single-stranded antisense oligonucleotide according to the present invention can also be produced by other methods known to a person skilled in the art where the base sequence, the modified moiety, and/or the like of the nucleic acid are changed as appropriate. AmNA, GuNA, and scpBNA can be produced by methods described by International Patent Laying-Open No. WO 2011/052436 (PTL 2), International Patent Laying-Open No.

WO 2014/046212 (PTL 3), and International Patent Laying-Open No. WO 2015/125783 (PTL 4), respectively. 2'-MOE nucleic acid can be produced by using an amidite that is available as a reagent. 5'-CP nucleic acid can be produced by a method described by International Patent Laying-Open No. WO 2020/158910 (PTL 5). LNA can be produced by a method described by International Patent Laying-Open No. WO 99/14226 (PTL 6).

<<Method of Producing Double-Stranded Antisense Oligonucleotide>>

The double-stranded antisense oligonucleotide according to the present invention can be produced in the following manner: firstly, by the same method as for producing the single-stranded antisense oligonucleotide, an oligonucleotide (a second oligonucleotide) having a certain sequence identity to a base sequence complementary to the single-stranded antisense oligonucleotide is produced, and, subsequently, the single-stranded antisense oligonucleotide and the second oligonucleotide are hybridized to each other.

<<Antisense Oligonucleotide Complex>>

The antisense oligonucleotide complex according to the present embodiment has:

the above-described single-stranded antisense oligonucleotide or a pharmaceutically acceptable salt thereof, or the above-described double-stranded antisense oligonucleotide or a pharmaceutically acceptable salt thereof; and
an additional substance bonded to the single-stranded antisense oligonucleotide or to the second oligonucleotide. The additional substance is selected from the group consisting of polyethylene glycol, peptide, alkyl chain (such as saturated aliphatic hydrocarbons, for example), ligand compound, antibody, protein, and sugar chain (such as hydrocarbons and polysaccharides, for example).

In an aspect of the present embodiment, the antisense oligonucleotide complex has:

the single-stranded antisense oligonucleotide or a pharmaceutically acceptable salt thereof; and
an additional substance bonded to the single-stranded antisense oligonucleotide, wherein the additional substance is selected from the group consisting of polyethylene glycol, peptide, alkyl chain (such as saturated aliphatic hydrocarbons, for example), ligand compound, antibody, protein, and sugar chain (such as hydrocarbons and polysaccharides, for example).

In another aspect of the present embodiment, the antisense oligonucleotide complex has:

the double-stranded antisense oligonucleotide or a pharmaceutically acceptable salt thereof, and
an additional substance bonded to the single-stranded antisense oligonucleotide or to the second oligonucleotide, wherein the additional substance is selected from the group consisting of polyethylene glycol, peptide, alkyl chain (such as saturated aliphatic hydrocarbons, for example), ligand compound, antibody, protein, and sugar chain (such as hydrocarbons and polysaccharides, for example).

Herein, "additional substance" means a substance bonded to the single-stranded antisense oligonucleotide or to the second oligonucleotide, used for providing certain action. The additional substance may be bonded to the 5' end of the single-stranded antisense oligonucleotide, or may be bonded to the 3' end thereof, or may be bonded to both the 5' end and the 3' end thereof. Also, the additional substance may be bonded to the 5' end of the second oligonucleotide, or may be bonded to the 3' end thereof, or may be bonded to both the 5' end and the 3' end thereof. In an aspect of the present embodiment, preferably, the additional substance is bonded to any one of the 5' end of the single-stranded antisense oligonucleotide, that of the second oligonucleotide, the 3' end of the single-stranded antisense oligonucleotide, and that of the second oligonucleotide. Also, the additional substance may be bonded to the single-stranded antisense oligonucleotide or to the second oligonucleotide directly via a covalent bond. The additional substance may be bonded to the single-stranded antisense oligonucleotide or to the second oligonucleotide via a linker substance. Examples of the linker substance include linkers that are composed of alkyl, polyethylene glycol, peptide, disulfide, or nucleic acid, or of a combination of these. The method for binding the additional substance to the single-stranded antisense oligonucleotide or to the second oligonucleotide may be, for example, a method described in Examples below.
Non-limiting examples of peptides used as the additional substance include the following: CPPs (Cell Penetrating Peptides), nuclear translocation peptides, TAT (Trans-Activator of Transcription protein), polyarginine, glucagon-like peptide 1 analogs, synthetic cyclic RGD peptides, and brain translocation peptides.
Non-limiting examples of ligand compounds used as the additional substance include the following: N-acetylgalactosamine (GalNAc), sugars (such as glucose and mannose), lipids (such as cholesterol, palmitic acid, and docosahexaenoic acid), vitamins (such as folic acid, vitamin A, and vitamin E (tocopherol)), amino acids, and monoamine receptor ligands (such as indatraline).

Non-limiting examples of antibodies used as the additional substance include the following: anti-insulin-receptor antibody, anti-transferrin-receptor antibody, anti-LDL-receptor-associated-protein antibody, anti-CD22 antibody, anti-CD30 antibody, and anti-HER2 antibody.

Non-limiting examples of proteins used as the additional substance include the following: albumin.

<<Agcnt for Modulating Expression of RPS25 Gene>>

An agent for modulating expression of RPS25 gene according to the present embodiment comprises the single-stranded antisense oligonucleotide, the double-stranded antisense oligonucleotide, or the antisense oligonucleotide complex according to the present invention, as an active ingredient. In an aspect of the present embodiment, the agent for modulating expression may be an agent for reducing expression of RPS25 gene. In another aspect of the present embodiment, the agent for modulating expression may be an agent for reducing RAN translation. In another aspect of the present embodiment, the agent for modulating expression may be an agent for reducing expression of a dipeptide repeat through reduction of RAN translation. The single-stranded antisense oligonucleotide according to the present invention binds to RPS25 mRNA or mRNA precursor to reduce expression of RPS25 gene and, in turn, reduce RAN translation which is caused by the translation product of the gene. The administration method and the preparation for the agent for modulating expression of RPS25 gene according to the present invention may be any administration method and preparation that are known in the field.

«Pharmaceutical Composition Containing Single-Stranded Antisense Oligonucleotide and/or the Like as Active Ingredient»

A pharmaceutical composition according to the present embodiment comprises the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof according to the present invention, as an active ingredient. The administration method and the preparation for the pharmaceutical composition according to the present embodiment may be any administration method and preparation that are known in the field. Hereinafter, the pharmaceutical composition may also be expressed as "a pharmaceutical composition of the antisense oligonucleotide and/or the like".

The pharmaceutical composition is used for treating or preventing a disease associated with RPS25 gene, more specifically, a disease that can be induced by a dipeptide repeat produced by RAN translation. Another way of understanding it is that the pharmaceutical composition can be used for treating or preventing a disease whose symptom(s) is expected to be alleviated by reduction of expression of RPS25 gene. A disease of this kind may also be expressed as "a repeat disease". Specific examples of repeat diseases include various neuropsychiatric diseases and muscular diseases including C9orf72 ALS, C9orf72 FTLD, Huntington's disease, spinocerebellar ataxia (type 1, 2, 3, 6, 7, 8, 12, 17), dentatorubral-pallidoluysian atrophy, spinal and bulbar muscular atrophy, Friedreich ataxia, fragile X-associated tremor/ataxia syndrome, myotonic dystrophy, and the like.

<<Agent for Treating Repeat Disease and Agent for Preventing Repeat Disease>>

An agent for treating a repeat disease according to the present embodiment comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient. An agent for preventing a repeat disease according to the present embodiment comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient. Preferably, the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

<C9orf72 ALS>

C9orf72 ALS as mentioned above means a type of ALS that has a mutation that involves abnormal repeat expansion of GGGGCC sequence present in the intron region of C9orf72 gene between the exon 1a region and the

exon 1b region. C9orf72 gene is the most frequently found ALS-causing gene, and responsible for about 6% of sporadic ALS and about 40% of familial ALS. ALS is a neurodegenerative disease that causes selective death of motor neurons leading to muscle atrophy. Diagnosis of ALS is made based on clinical characteristics or electrophysiological characteristics of a combination of upper and lower motor neuron degeneration. More specifically, when one of four regions (namely, brainstem, cervical cord, thoracic cord, and lumbosacral cord) shows signs of upper and lower motor neuron degeneration and the other regions have electromyographic finding, the diagnosis is "laboratory-supported probable"; when two of the four regions show the signs, the diagnosis is "probable"; and three of the four regions show the signs, the diagnosis is "definite".

<C9orf72 FTLD>

C9orf72 FTLD as mentioned above means an FTLD that has a mutation that involves abnormal repeat expansion of GGGGCC sequence present in the intron region of C9orf72 gene between the exon 1a region and the exon 1b region. FTLD of this type manifests in the form of progressive abnormal behavior and cognitive dysfunction causing daily living to be more challenging, and it also shows at least three symptoms from behavioral disinhibition, indifference and/or inertia, adherence and/or stereotyped behavior, hyperorality, changes in dietary habits, and the like. FTLD of this type is diagnosed as behavioral variant FTLD when imaging examination shows atrophy and/or impaired metabolism and/or impaired blood flow in the frontal lobe and/or in the anterior temporal lobe and when the disease can be differentiated from certain diseases. FTLD of this type is diagnosed as semantic dementia FTLD when memory loss of names of objects and meaning of words, and symptoms of loss of knowledge of objects or superficial dyslexia and/or agraphia are observed, atrophy is found in the anterior dominant temporal lobe, and the disease can be differentiated from certain diseases.

<Huntington's disease>

Huntington's disease as mentioned above means a hereditary neurodegenerative disease that exhibits autosomal dominant inheritance developed by abnormal repeat expansion of CAG sequence present in the exon 1 region of huntingtin gene. Huntington's disease manifests in the form of movement disorder characterized in involuntary movement, psychiatric symptoms, and cognitive symptoms. Diagnosis of Huntington's disease is made when particular neurologic findings are observed and genetic diagnosis identifies abnormal expansion mutation of CAG sequence, or when the course of the disease is progressive, family history of autosomal dominant inheritance and certain neurologic findings and clinical laboratory findings are observed, and differential diagnosis denies the possibility of similar diseases.

<Spinocerebellar Ataxia (Type 1, 2, 3, 6, 7, 8, 12, 17), and Dentatorubral-Pallidoluysian Atrophy>

Each of spinocerebellar ataxia (type 1, 2, 3, 6, 7, 8, 12, 17) and dentatorubral-pallidoluysian atrophy as mentioned above means a hereditary neurodegenerative disease that exhibits autosomal dominant inheritance developed by abnormal repeat expansion of a particular three-base sequence (CAG or CTG) present on the disease gene in the disease. In spinocerebellar ataxia type 1, 2, 3, 6, 7, 12, and 17 and in dentatorubral-pallidoluysian atrophy, CAG repeats are observed. In spinocerebellar ataxia type 8, CTG repeats are observed. Spinocerebellar ataxia and dentatorubral-pallidoluysian atrophy manifest themselves in the form of cerebellar or posterior column ataxia or spastic paraplegia as their major symptom and are basically indolent, and diagnosis of them is made by a combination of genetic diagnosis, neuropathological diagnosis, and the like.

<Spinal and Bulbar Muscular Atrophy>

Spinal and bulbar muscular atrophy as mentioned above means a hereditary disease that is developed by abnormal repeat expansion of CAG sequence present in the exon region of androgen receptor gene. Diagnosis of spinal and bulbar muscular atrophy is made by a combination of neurologic finding (bulbar syndrome, lower motor neuron signs, finger tremor, tendon hyporeflexia of extremities), clinical finding, laboratory finding, genetic diagnosis, and the like.

<Friedreich Ataxia>

Friedreich ataxia as mentioned above means a hereditary neurodegenerative disease that exhibits autosomal recessive inheritance developed by mutation of frataxin gene. Most cases of Friedreich ataxia are caused by abnormal repeat expansion of GAA sequence present in the first intron.

<Fragile X-Associated Tremor/Ataxia Syndrome>

Fragile X-associated tremor/ataxia syndrome as mentioned above means a hereditary neurodegenerative disease developed by abnormal repeat expansion of CGG sequence present in 5'UTR of FMP1 gene. Diagnosis of fragile X-associated tremor/ataxia syndrome is made by a combination of clinical symptoms (cerebellar ataxia, action tremor, parkinsonism, dementia, mental retardation), signs of middle cerebellar peduncle by MRI examination, genetic diagnosis, and the like.

<Myotonic Dystrophy>

Myotonic dystrophy as mentioned above means a hereditary muscular disease that exhibits autosomal dominant inheritance developed by abnormal repeat expansion of CUG sequence present in 3'UTR of DMPK gene.

<Individuals>

The individual mentioned above means a mammal. Preferably, the individual refers to a human, a monkey and ape, a marmoset, a dog, a pig, a rabbit, a guinea pig, a rat, and a mouse. More preferably, the individual is a human.

For the administration of the single-stranded antisense oligonucleotide according to the present invention or a pharmaceutical composition thereof (including an agent for treating C9orf72 ALS and an agent for preventing C9orf72 ALS), the method and form of administration are not particularly limited. In other words, any administration method and any preparation known in the field can be used as the administration method and preparation for the antisense oligonucleotide according to the present invention and the like. Examples of the administration method include oral administration, parenteral administration, and the like. Examples of the parenteral administration include ophthalmic administration, vaginal administration, intrarectal administration, intranasal administration, transdermal administration, intravenous injection, infusion, subcutaneous, intraperitoneal, or intramuscular injection, pulmonary administration via suction or inhalation, intrathecal injection, intraventricular administration, and the like.

To the preparation of the antisense oligonucleotide according to the present invention and the like, various pharmaceutical additives such as excipient, binder, wetting agent, disintegrating agent, lubricant, diluent, taste masking agent, fragrant agent, dissolution promoter, suspending agent, emulsifier, stabilizer, preservative, isotonic agent, and the like can be mixed, as needed.

In the case of topical administration of a pharmaceutical composition of the antisense oligonucleotide according to the present invention and the like, preparations such as transdermal patch, ointment, lotion, cream, gel, drops, suppository, spray, liquid preparation, powder, and the like can be used, for example.

In the case of oral administration of a pharmaceutical composition of the antisense oligonucleotide according to the present invention and the like, preparations such as powder, granule, suspension in or solution dissolved in water or non-aqueous medium, capsule, powder agent, pill, and the like can be used, for example.

In the case of parenteral, intrathecal, or intraventricular administration of a pharmaceutical composition of the antisense oligonucleotide according to the present invention and the like, preparations such as sterile aqueous solution can be used, for example.

The effective dose of the single-stranded antisense oligonucleotide according to the present invention can be determined as appropriate depending on the sex, age, body weight, symptoms, and the like of the individual who is the administration target. Further, it can also be determined as appropriate depending on the method, route, frequency, and the like of administration. For example, the dose may be from 0.01 to 100 mg/kg and the like. It is preferably from 0.1 to 50 mg/kg, further preferably from 0.1 to 10 mg/kg.

<<Method of Modulating Expression of RPS25 Gene>>

A method of modulating expression of RPS25 gene according to the present embodiment comprises administering the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient, to a cell, a tissue, or an individual expressing the RPS25 gene.

In the present embodiment, the method for administering the single-stranded antisense oligonucleotide and the like to a cell, a tissue, or an individual may be performed in vitro or in vivo. In the case of in vivo administration, the route of administration is the above-described route of administration.

In the present embodiment, examples of "a cell expressing RPS25 gene" include neurons constituting the central nervous system, neurons constituting the peripheral nervous system, cells constituting other dermal tissue, and the like.

A method of treating or preventing a repeat disease according to the present embodiment comprises administering the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient, to an individual suffering from the repeat disease.

Examples of the repeat disease include neuropsychiatric diseases, muscular diseases, and the like described above. The form, administration route, and dose for administration to an individual can be selected as appropriate from those described above.

In the above, the antisense oligonucleotide according to the present embodiment is described. The single-stranded antisense oligonucleotide having the above-described configuration can modulate RPS25 gene expression. Here, the activity to reduce RPS25 gene expression (knockdown activity) can be measured by a known method. Examples of the method for measuring the knockdown activity include a method described in Nature (2015) 518(7539):409-12 (NPL 10), and the like. It can also be measured by transfection of the antisense oligonucleotide to HEK293T cells, as described below.

<<Method for Evaluating RPS25 Gene Expression-Reducing Activity>>

<Introduction of Antisense Oligonucleotide into Cells>

"Cells expressing RPS25 gene" are treated with the antisense oligonucleotide for 6 hours to 3 days by a method such as lipofection, electroporation, or direct addition introduction. The cells to be used may be any cells as long as they express RPS25 gene, and examples thereof include HEK293T cells, more preferably neurons, further preferably human-derived neurons. The cells thus treated with the antisense oligonucleotide may be collected immediately after the treatment, or may be continued to be cultured after removal of the antisense oligonucleotide.

<Evaluation of Amount of RPS25 mRNA>

Total RNA is extracted from the collected cells and subjected to reverse transcription reaction, and the resulting complementary DNA is subjected to real-time PCR and/or the like with the use of a probe specific to RPS25 gene, to measure the amount of RPS25 mRNA. Examples of the probe for use in real-time PCR include Taqman probe. Examples of the method for the reaction include a method that involves repeating, a number of times that is not particularly limited, a three-step procedure of "(cDNA denaturing)-(annealing)-(elongation reaction)" or a two-step procedure of "(cDNA denaturing)-(annealing and elongation reaction)". For example, the two- or three-step procedure is repeated 25 to 45 times, preferably 35 to 40 times. For example, the temperature for (cDNA denaturing) is from 90°C to 98°C, preferably from 92°C to 95°C. For example, the temperature for (annealing) is from 40°C to 70°C, preferably from 50°C to 60°C. For example, the temperature for (elongation reaction) is from 65°C to 75°C, preferably it is the optimum temperature for the polymerase used in the reaction. For example, the temperature for (annealing and elongation reaction) is from 55°C to 70°C.

<Evaluation of Amount of RPS25 Protein>

The cells thus collected are lysed to obtain an extract. By an immunochemical technique such as Western blotting and ELISA (Enzyme-Linked Immuno Sorbent Assay), the amount of RPS25 protein contained in the extract is assessed. In the case of Western blotting, the apparatus to be used in each of the steps of electrophoresis, transfer, and detection is not particularly limited. The reaction time and reaction temperature for reaction of the membrane with the primary antibody or the secondary antibody can be selected as needed, and examples thereof include overnight at 4°C, and 1 to 3 hours at room temperature.

It should be noted that the present invention is not limited to the above-described embodiment. For example, the single-stranded antisense oligonucleotide encompasses the aspects described below.

An aspect of the single-stranded antisense oligonucleotide according to the present invention is a single-stranded antisense oligonucleotide, or a pharmaceutically acceptable salt thereof, capable of modulating expression of RPS25 gene, wherein

nucleotides of the single-stranded antisense oligonucleotide are bonded to each other via a phosphate group and/or a modified phosphate group,

the single-stranded antisense oligonucleotide includes a gap region, a 3' wing region bonded to a 3' end of the gap region, and a 5' wing region bonded to a 5' end of the gap region,

the gap region is a deoxyribose-based nucleic acid optionally including a nucleic acid having a modified sugar moiety,

each of the 3' wing region and the 5' wing region is a modified nucleic acid,
the single-stranded antisense oligonucleotide has a base length of 12- to 30-mer, and
a base sequence of the single-stranded antisense oligonucleotide is:
a base sequence with a sequence identity of 90% to 100% to a base sequence complementary to at least one target region of the same base length as the single-stranded antisense oligonucleotide present in the base sequence as set forth in SEQ ID NO: 1 or SEQ ID NO: 2.

In another aspect of the single-stranded antisense oligonucleotide according to the present invention, the base sequence of the single-stranded antisense oligonucleotide is:
a base sequence with a sequence identity of 95% to 100% to a base sequence complementary to at least one target region of the same base length as the single-stranded antisense oligonucleotide present in the base sequence as set forth in SEQ ID NO: 1 or SEQ ID NO: 2.

In another aspect of the single-stranded antisense oligonucleotide according to the present invention, the base sequence of the single-stranded antisense oligonucleotide is a base sequence complementary to at least one target region of the same base length as the single-stranded antisense oligonucleotide present in the base sequence as set forth in SEQ ID NO: 1 or SEQ ID NO: 2.

In another aspect of the single-stranded antisense oligonucleotide according to the present invention,

the gap region is a 5- to 20-mer deoxyribose-based nucleic acid optionally including a nucleic acid having a modified sugar moiety,
the 3' wing region is a 1- to 5-mer modified nucleic acid,
the modified nucleic acid of the 3' wing region is a 2'-modified nucleic acid and/or a bridged nucleic acid,
the 5' wing region is a 1- to 5-mer modified nucleic acid, and
the modified nucleic acid of the 5' wing region is a 2'-modified nucleic acid and/or a bridged nucleic acid.

In another aspect of the single-stranded antisense oligonucleotide according to the present invention, the single-stranded antisense oligonucleotide has a base length of 14- to 22-mer,

the gap region is a 6- to 17-mer deoxyribose-based nucleic acid optionally including a nucleic acid having a modified sugar moiety,
the 3' wing region is a 2- to 5-mer modified nucleic acid,
the modified nucleic acid of the 3' wing region includes at least one selected from the group consisting of LNA, AmNA, GuNA, and scpBNA,
the 5' wing region is a 2- to 5-mer modified nucleic acid,
the modified nucleic acid of the 5' wing region includes at least one selected from the group consisting of LNA, AmNA, GuNA, and scpBNA, and
at least one bond between nucleotides of the single-stranded antisense oligonucleotide is a phosphorothioate bond.

In another aspect of the single-stranded antisense oligonucleotide according to the present invention, the single-stranded antisense oligonucleotide has a chain length of 14- to 20-mer,

the gap region is a 7- to 13-mer deoxyribose-based nucleic acid optionally including a nucleic acid having a modified sugar moiety,
the nucleic acid of the gap region includes at least one selected from the group consisting of 5-methyldeoxycytidine and 5'-CP nucleic acid,
the 3' wing region is a 2- to 4-mer modified nucleic acid,
the modified nucleic acid of the 3' wing region includes at least one selected from the group consisting of 2'-MOE nucleic acid, AmNA, GuNA, and scpBNA,
the 5' wing region is a 2- to 4-mer modified nucleic acid, and
the modified nucleic acid of the 5' wing region includes at least one selected from the group consisting of 2'-MOE nucleic acid, AmNA, GuNA, and scpBNA.

In another aspect of the single-stranded antisense oligonucleotide according to the present invention, the single-stranded antisense oligonucleotide has a base length of 14- to 18-mer,

the gap region is a 7- to 11-mer,
the 3' wing region is a 2- to 4-mer modified nucleic acid,
the modified nucleic acid of the 3' wing region includes at least one selected from the group consisting of AmNA,

GuNA, and scpBNA,
the 5' wing region is a 2- to 4-mer modified nucleic acid, and
the modified nucleic acid of the 5' wing region includes at least one selected from the group consisting of AmNA, GuNA, and scpBNA.

[Examples]

In the following, Examples of the present invention will be described, but the scope of the present invention is not limited to these Examples.

<<Preparation of Single-Stranded Antisense Oligonucleotide for RPS25 Gene>>

Firstly, the single-stranded antisense oligonucleotides specified in Table 3-1 to Table 3-17 and Table 4-1 to Table 4-5 were designed. For some of the designed ones, a single-stranded antisense oligonucleotide for RPS25 gene was prepared in the manner described below.

A single-stranded antisense oligonucleotide that included, as a modified nucleic acid, 2'-O-methyl nucleic acid, 2'-MOE nucleic acid, AmNA, scpBNA, 5'-CP nucleic acid, and/or GuNA, and/or a nucleic acid whose nucleobase moiety was 5-methylcytosine was synthesized, with the use of an automatic nucleic-acid synthesizer (model nS-8, manufactured by GeneDesign), in a scale of 0.2 μmol. The chain length expansion was carried out by following a standard phosphoramidite protocol. As the solid support, CPG resin was used. For sulfidation for forming a phosphorothioated (PS) backbone, DDTT (((Dimethylamino-methylidene)amino)-3H-1,2,4-dithiazaoline-3-thione) and/or the like was used. The single-stranded antisense oligonucleotide including 2'-MOE nucleic acid, AmNA, and/or scpBNA was obtained in the form where the terminal 5' hydroxy group was not protected by DMTr (4,4'-dimethoxytrityl) group and the 3' position was supported on the solid phase. Then, the single-stranded antisense oligonucleotide was removed from the solid support by alkali treatment, and then collected in the form of solution. Subsequently, solvent was distilled off from the solution thus collected, and thereby a crude product was obtained. The resulting crude product was purified by reversed-phase HPLC, and thereby a purified single-stranded antisense oligonucleotide was obtained. The purity and the structure of the resulting single-stranded antisense oligonucleotide were checked by LC-MS (manufactured by Waters).

Examples 412 to 416 shown in Table 3-14 were synthesized according to the above protocol, with the use of phosphoramidite having a corresponding additional substance (for example, synthesis of compounds in Figs. 3 to 7). As the phosphoramidite having an additional substance, α-Tocopherol-TEG phosphoramidite (Glen research, product code 10-1977-02) was used in Example 412, and 5'-Palmitate-C6 CE-Phosphoramidite (Link technologies Ltd, item number 2199) was used in Examples 413 and 414. In Examples 415, 416, firstly, an oligonucleotide having an amino group at 5' position (amino ASO) was synthesized according to the above protocol with the use of a corresponding phosphoramidite (5'-amino-modifier C6 (Glen research, catalog no. 10-1906-02). To the resulting amino ASO, in Example 415, 2,5-dioxopyrrolidin-1-yl 8-(((1R,3S)-3-(3,4-dichlorophenyl)-2,3-dihydro-1H-inden-1-yl)(methyl)amino)-8-oxooctanoate synthesized by the method described below was used to synthesize a compound. In Example 416, the above-described amino ASO was condensed with Docosahexaenoic acid (DHA, Fujifilm, catalog no. 90310) to synthesize a compound.

Synthesis of 2,5-dioxopyrrolidin-1-yl 8-(((1R,3S)-3-(3,4-dichlorophenyl)-2,3-dihydro-1H-inden-1-yl)(methyl)amino)-8-oxooctanoate [2,5-dioxopyrrolidin-1-yl 8-(((1R,3S)-3-(3,4-dichlorophenyl)-2,3-dihydro-1H-inden-1-yl)(methyl)amino)-8-oxooctanoate]

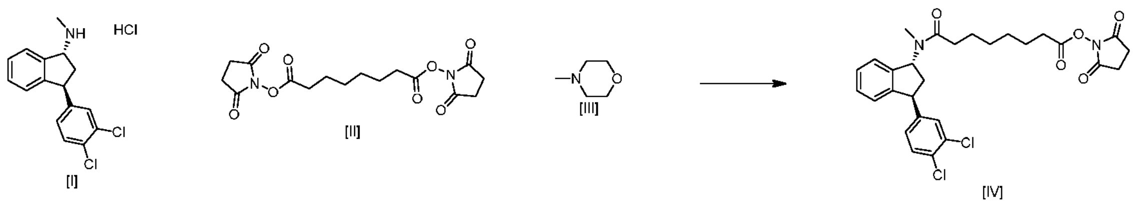

To a suspension of N,N'-disuccinimidyl suberate (101 mg, 0.274 mmol) and N-methylmorpholine (0.040 mL, 0.365 mmol) in N,N-dimethylacetamide (1 mL), a solution of indatraline hydrochloride (10 mg, 0.030 mmol) in N,N-dimethylacetamide was added at 0°C. Subsequently, the temperature of the reaction liquid was raised to reach 40°C, where stirring was carried out for 2 hours. The resulting reaction liquid was dried under reduced pressure, and the

resulting crude product was purified by silica gel column chromatography to obtain 7.0 mg of a mixture of the title compound and N,N'-disuccinimidyl suberate.

LC/MS:545[M+H]

In Table 3-1 to Table 3-17 and Table 4-1 to Table 4-5 below, single-stranded antisense oligonucleotides produced by the above-described method are listed. The single-stranded antisense oligonucleotides in Table 3-1 to Table 3-17 are single-stranded antisense oligonucleotides for human RPS25 mRNA (SEQ ID NO: 1). Examples 463 and 464 correspond to negative controls.

[Table 3-1]

| Ex. | Sequence name | Antisense oligonucleotide sequence (5'-3') | X-Y-Z X-Y-Z-W | Mw (actual) | SEQ ID NO: |
|---|---|---|---|---|---|
| 1 | h8-22-A | 5(Y)^G(Y)^T(Y)^c^a^a^g^a^t^g^t^c^G(Y)^G(Y)^a | 3-9-2-1 | 5129.4 | 7 |
| 2 | h9-23-A | T(Y)^5(Y)^G(Y)^t^c^a^a^g^a^t^g^t^5(Y)^G(Y)^g | 3-9-2-1 | 5133.4 | 8 |
| 3 | h 10-24-A | 5(Y)^T(Y)^5(Y)^g^t^c^a^a^g^a^t^g^A^T(Y)^5(Y)^g | 3-9-2-1 | 5108.2 | 9 |
| 4 | h27-41-A | G(Y)^5(Y)^A(Y)^g^c^a^e^a^c^a^c^c^G(Y)^5(Y)^a | 3-9-2-1 | 5082.3 | 10 |
| 5 | h28-42-A | A(Y)^G(Y)^5(Y)^a^g^c^a^g^a^c^a^c^5(Y)^G(Y)^c | 3-9-2-1 | 5081.8 | 11 |
| 6 | h29-43-A | T(Y)^A(Y)^G(Y)^c^a^g^c^a^g^a^c^a^5(Y)^5(Y)^g | 3-9-2-1 | 5098.0 | 12 |
| 7 | h30-44-A | A(Y)^T(Y)^A(Y)^g^c^a^g^c^a^g^a^c^A(Y)^5(Y)^c | 3-9-2-1 | 5065.4 | 13 |
| 8 | h32-46-A | G(Y)^A(Y)^A(Y)^t^a^g^c^a^g^c^a^g^A(Y)^5(Y)^a | 3-9-2-1 | 5131.3 | 14 |
| 9 | h33-47-A | A(Y)^G(Y)^A(Y)^a^t^a^g^c^a^g^c^a^G(Y)^A(Y)^c | 3-9-2-1 | 5118.5 | 15 |
| 10 | h34-48-A | G(Y)^A(Y)^G(Y)^a^a^t^a^g^c^a^g^c^A(Y)^G(Y)^a | 3-9-2-1 | 5157.5 | 16 |
| 11 | h35-49-A | G(Y)^G(Y)^A(Y)^g^a^a^t^a^g^c^a^g^5(Y)^A(Y)^g | 3-9-2-1 | 5187.8 | 17 |
| 12 | h36-50-A | 5(Y)^G(Y)^G(Y)^a^g^a^a^t^a^g^c^a^G(Y)^5(Y)^a | 3-9-2-1 | 5163.8 | 18 |
| 13 | h37-51-A | T(Y)^5(Y)^G(Y)^g^a^g^a^a^t^a^g^c^A(Y)^G(Y)^c | 3-9-2-1 | 5138.4 | 19 |
| 14 | h38-52-A | 5(Y)^T(Y)^5(Y)^g^g^a^g^a^a^t^a^g^5(Y)^A(Y)^g | 3-9-2-1 | 5166.8 | 20 |
| 15 | h72-86-A | 5(Y)^T(Y)^T(Y)^c^t^t^c^t^t^g^t^c^G(Y)^T(Y)^c | 3-9-2-1 | 4988.0 | 21 |
| 16 | h79-93-A | 5(Y)^G(Y)^T(Y)^c^c^t^t^c^t^t^c^t^T(Y)^5(Y)^t | 3-9-2-1 | 4963.9 | 22 |
| 17 | h101-115-A | T(Y)^5(Y)^T(Y)^t^t^c^t^t^g^g^c^c^G(Y)^A(Y)^c | 3-9-2-1 | 5020.6 | 23 |
| 18 | h102-116-A | G(Y)^T(Y)^5(Y)^t^t^t^c^t^t^g^g^c^5(Y)^G(Y)^a | 3-9-2-1 | 5078.7 | 24 |
| 19 | h103-117-A | T(Y)^G(Y)^T(Y)^c^t^t^t^c^t^t^g^g^5(Y)^5(Y)^g | 3-9-2-1 | 5069.2 | 25 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 20 | h122-136-A | G(Y)^A(Y)^T(Y)^t^t^g^t^t^c^a^c^t^G(Y)^G(Y)^g | 3-9-2-1 | 5113.3 | 26 |
| 21 | h124-138-A | 5(Y)^G(Y)^G(Y)^a^t^t^t^g^t^t^c^a^5(Y)^T(Y)^g | 3-9-2-1 | 5101.9 | 27 |
| 22 | h125-139-A | 5(Y)^5(Y)^G(Y)^g^a^t^t^t^g^t^t^c^A(Y)^5(Y)^t | 3-9-2-1 | 5074.9 | 28 |
| 23 | h126-140-A | 5(Y)^5(Y)^5(Y)^g^g^a^t^t^t^g^t^t^5(Y)^A(Y)^c | 3-9-2-1 | 5073.0 | 29 |
| 24 | h140-154-A | T(Y)^T(Y)^T(Y)^t^t^g^g^c^c^t^t^a^5(Y)^5(Y)^C | 3-9-2-1 | 5027.6 | 30 |
| 25 | h160-174-A | T(Y)^G(Y)^5(Y)^c^t^t^t^g^g^a^c^c^A(Y)^5(Y)^t | 3-9-2-1 | 5045.3 | 31 |

[Table 3-2]

| Ex. | Sequence name | Antisense oligonucleotide sequence (5'-3') | X-Y-Z X-Y-Z-W | Mw (actual) | SEQ ID NO: |
|---|---|---|---|---|---|
| 26 | h161-175-A | T(Y)^T(Y)^G(Y)^c^c^t^t^t^g^g^a^c^5(Y)^A(Y)^c | 3-9-2-1 | 5033.3 | 32 |
| 27 | h180-194-A | A(Y)^T(Y)^T(Y)^g^a^g^c^t^t^g^t^c^5(Y)^5(Y)^g | 3-9-2-1 | 5087.3 | 33 |
| 28 | h181-195-A | T(Y)^A(Y)^T(Y)^t^g^a^g^c^t^t^g^t^5(Y)^5(Y)^c | 3-9-2-1 | 5061.0 | 34 |
| 29 | h182-196-A | T(Y)^T(Y)^A(Y)^t^t^g^a^g^c^t^t^g^T(Y)^5(Y)^c | 3-9-2-1 | 5061.7 | 35 |
| 30 | h183-197-A | G(Y)^T(Y)^T(Y)^a^t^t^g^a^a^c^t^t^G(Y)^T(Y)^c | 3-9-2-1 | 5088.3 | 36 |
| 31 | h184-198-A | A(Y)^G(Y)^T(Y)^t^a^t^t^g^a^g^c^t^T(Y)^G(Y)^t | 3-9-2-1 | 5111.2 | 37 |
| 32 | h187-201-A | 5(Y)^T(Y)6A(Y)^a^g^t^t^a^t^t^g^a^G(Y)^5(Y)^t | 3-9-2-1 | 5109.4 | 38 |
| 33 | h188-202-A | A(Y)^5(Y)^T(Y)^a^a^g^t^t^a^t^t^g^A(Y)^G(Y)^c | 3-9-2-1 | 5106.0 | 39 |
| 34 | h189-203-A | G(Y)^A(Y)^5(Y)^t^a^a^g^t^t^a^t^t^G(Y)^A(Y)^g | 3-9-2-1 | 5146.5 | 40 |
| 35 | h191-205-A | A(Y)^A(Y)^G(Y)^a^c^t^a^a^g^t^t^a^T(Y)^T(Y)^g | 3-9-2-1 | 5114.9 | 41 |
| 36 | h193-207-A | A(Y)^5(Y)^A(Y)^a^g^a^c^t^a^a^g^t^T(Y)^A(Y)^t | 3-9-2-1 | 5098.4 | 42 |
| 37 | h 196-210-A | 5(Y)^A(Y)^A(Y)^a^c^a^a^g^a^c^t^a^A(Y)^G(Y)^t | 3-9-2-1 | 5093.6 | 43 |
| 38 | h197-211-A | T(Y)^5(Y)^A(Y)^a^a^c^a^a^g^a^c^t^A(Y)^A(Y)^g | 3-9-2-1 | 5093.2 | 44 |
| 39 | h208-222-A | A(Y)^G(Y)^G(Y)^t^a^g^c^t^t^t^g^t^5(Y)^A(Y)^a | 3-9-2-1 | 5123.0 | 45 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 40 | h209-223-A | T(Y)^A(Y)^G(Y)^g^t^a^g^c^t^t^t^g^T(Y)^5(Y)^a | 3-9-2-1 | 5113.8 | 46 |
| 41 | h210-224-A | A(Y)^T(Y)^(Y)^g^g^t^a^g^c^t^t^t^G(Y)^T(Y)^c | 3-9-2-1 | 5096.7 | 47 |
| 42 | h213-227-A | A(Y)^T(Y)^5(Y)^a^t^a^g^g^t^a^g^c^T(Y)^T(Y)^t | 3-9-2-1 | 5093.2 | 48 |
| 43 | h214-228-A | T(Y)^A(Y)^T(Y)^c^a^t^a^g^g^t^a^g^5(Y)^T(Y)^t | 3-9-2-1 | 5093.7 | 49 |
| 44 | h216-230-A | T(Y)^T(Y)^T(Y)^a^t^c^a^t^a^g^g^t^A(Y)^G(Y)^c | 3-9-2-1 | 5079.6 | 50 |
| 45 | h217-231-A | G(Y)^T(Y)^T(Y)^t^a^t^c^a^t^a^g^g^T(Y)^A(Y)^F | 3-9-2-1 | 5120.7 | 51 |
| 46 | h219-233-A | G(Y)^A(Y)^G(Y)^t^t^t^a^t^c^a^t^a^G(Y)^G(Y)^t | 3-9-2-1 | 5119.9 | 52 |
| 47 | h220-234-A | A(Y)^G(Y)^A(Y)^g^t^t^t^a^t^c^a^t^A(Y)^G(Y)^g | 3-9-2-1 | 5128.9 | 53 |
| 48 | h242-256-A | T(Y)^T(Y)^A(Y)^t^a^g^t^t^g^g^g^a^A(Y)^5(Y)^t | 3-9-2-1 | 5133.9 | 54 |
| 49 | h243-257-A | T(Y)^T(Y)^T(Y)^a^t^a^g^t^t^g^g^g^A(Y)^A(Y)^c | 3-9-2-1 | 5120.8 | 55 |
| 50 | h253-267-A | G(Y)^G(Y)^G(Y)^t^t^a^t^a^a^g^t^t^T(Y)^A(Y)^t | 3-9-2-1 | 5136.0 | 56 |

[Table 3-3]

| Ex. | Sequence name | Antisense oligonucleotide sequence (5'-3') | X-Y-Z X-Y-Z-W | Mw (actual) | SEQ ID NO: |
|---|---|---|---|---|---|
| 51 | h254-268-A | G(Y)^G(Y)^G(Y)^g^t^t^a^t^a^a^g^t^T(Y)^T(Y)^a | 3-9-2-1 | 5161.2 | 57 |
| 52 | h259-273-A | 5(Y)^A(Y)^G(Y)^c^t^g^g^g^g^t^t^a^T(Y)^A(Y)^a | 3-9-2-1 | 5145.6 | 58 |
| 53 | h260-274-A | A(Y)^5(Y)^A(Y)^g^c^t^g^g^g^g^t^t^A(Y)^T(Y)^a | 3-9-2-1 | 5145.0 | 59 |
| 54 | h261-275-A | 5(Y)^A(Y)^5(Y)^a^g^c^t^g^g^g^g^t^T(Y)^A(Y)^t | 3-9-2-1 | 5135.6 | 60 |
| 55 | h285-299-A | T(Y)^5(Y)^G(Y)^alla^tllclltltllcllall$_g$llT(Y),15(Y)^t | 3-9-2-1 | 5045.0 | 61 |
| 56 | h286-300-A | 5(Y)^T(Y)^5(Y)^g^a^a^t^c^t^t^c^a^G(Y)^T(Y)^c | 3-9-2-1 | 5030.5 | 62 |
| 57 | h295-309-A | 5(Y)^5(Y)^A(Y)^g^g^g^a^g^c^c^t^c^G(Y)^A(Y)^a | 3-9-2-1 | 5114.1 | 63 |
| 58 | h296-310-A | G(Y)^5(Y)^5(Y)^a^g^g^g^a^g^c^c^t^5(Y)^G(Y)^a | 3-9-2-1 | 5144.9 | 64 |
| 59 | h297-311-A | G(Y)^G(Y)^5(Y)^c^a^g^g^g^a^g^c^c^T(Y)^5(Y)^g | 3-9-2-1 | 5146.4 | 65 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 60 | h325-339-A | T(Y)^A(Y)^5(Y)^t^a^a^g^g^a^g6c^t^5(Y)^5(Y)^t | 3-9-2-1 | 5094.0 | 66 |
| 61 | h326-340-A | T(Y)^T(Y)^A(Y)^c^t^a^a^g^g^a^g^c^T(Y)^5(Y)^c | 3-9-2-1 | 5065.8 | 67 |
| 62 | h327-341-A | T(Y)^T(Y)^T(Y)^a^c^t^a^a^g^g^a^g^5(Y)^T(Y)^c | 3-9-2-1 | 5080.7 | 68 |
| 63 | h340-354-A | G(Y)^T(Y)^T(Y)^t^g^a^t^a^a^g^t^c^5(Y)^T(Y)^t | 3-9-2-1 | 5086.3 | 69 |
| 64 | h341-355-A | A(Y)^G(Y)^T(Y)^t^t^g^a^t^a^a^g^t^5(Y)^5(Y)^t | 3-9-2-1 | 5109.6 | 70 |
| 65 | h342-356-A | 5(Y)^A(Y)^G(Y)^t^t^t^g^a^t^a^a^g^T(Y)^5(Y)^c | 3-9-2-1 | 5094.0 | 71 |
| 66 | h343-357-A | 5(Y)^5(Y)^A(Y)^g^t^t^t^g^a^t^a^a^G(Y)^T(Y)^c | 3-9-2-1 | 5093.8 | 72 |
| 67 | h344-358-A | A(Y)^5(Y)^5(Y)^a^g^t^t^t^g^t^a^A(Y)^G(Y)^t | 3-9-2-1 | 5118.6 | 73 |
| 68 | h365-379-A | A(Y)^5(Y)^T(Y)^t^g^a^g^c^t^c^t^g^T(Y)^G(Y)^c | 3-9-2-1 | 5073.2 | 74 |
| 69 | h375-389-A | G(Y)^G(Y)^T(Y)^g^t^a^a^a^t^t^a^c^T(Y)^T(Y)^g | 3-9-2-1 | 5121.1 | 75 |
| 70 | h390-404-A | A(Y)^5(Y)^5(Y)^c^t^t^g^g^t^a^t^t^T(Y)^5(Y)^t | 3-9-2-1 | 5050.3 | 76 |
| 71 | h393-407-A | T(Y)^5(Y)^5(Y)^a^c^c^c^t^t^g^g^t^A(Y)^T(Y)^t | 3-9-2-1 | 5021.7 | 77 |
| 72 | h394-408-A | 5(Y)^T(Y)^5(Y)^c^a^c^c^c^t^t^g^g^T(Y)^A(Y)^t | 3-9-2-1 | 5006.4 | 78 |
| 73 | h430-444-A | G(Y)^A(Y)^5(Y)^c^t^a^t^t^c^a^t^g^5(Y)^A(Y)^t | 3-9-2-1 | 5054.4 | 79 |
| 74 | h431-445-A | G(Y)^G(Y)^A(Y)^c^c^t^a^t^t^c^a^t^G(Y)^5(Y)^a | 3-9-2-1 | 5066.4 | 80 |
| 75 | h432-446-A | T(Y)^G(Y)^G(Y)^a^c^c^t^a^t^t^c^a^T(Y)^G(Y)^c | 3-9-2-1 | 5042.2 | 81 |

[Table 3-4]

| Ex. | Sequence name | Antisense oligonucleotide sequence (5'-3') | X-Y-Z X-Y-Z-W | Mw (actual) | SEQ ID NO: |
|---|---|---|---|---|---|
| 76 | h433-447-A | T(Y)^T(Y)^G(Y)^g^a^c^c^t^a^t^t^c^A(Y)^T(Y)^g | 3-9-2-1 | 5056.9 | 82 |
| 77 | h434-448-A | G(Y)^T(Y)^T(Y)^g^g^a^c^c^t^a^t^t^5(Y)^A(Y)^t | 3-9-2-1 | 5071.3 | 83 |
| 78 | h435-449-A | G(Y)^G(Y)^T(Y)^t^g^g^a^c^c^t^a^t^T(Y)^5(Y)^a | 3-9-2-1 | 5096.6 | 84 |
| 79 | h436-450-A | T(Y)^G(Y)^G(Y)^t^t^g^g^a^c^c^t^a^T(Y)^T(Y)^c | 3-9-2-1 | 5073.3 | 85 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 80 | h438-452-A | G(Y)^5(Y)^T(Y)^g^g^t^t^g^g^a^c^c^T(Y)^A(Y)^t | 3-9-2-1 | 5112.4 | 86 |
| 81 | h439-453-A | A(Y)^G(Y)^5(Y)^t^g^g^t^t^g^g^a^c^5(Y)^T(Y)^a | 3-9-2-1 | 5136.2 | 87 |
| 82 | h440-454-A | 5(Y)^A(Y)^G(Y)^c^t^g^g^t^t^g^g^a^5(Y)^5(Y)^t | 3-9-2-1 | 5125.2 | 88 |
| 83 | h441-455-A | A(Y)^5(Y)^A(Y)^g^c^t^g^g^t^t^g^g^A(Y)^5(Y)^c | 3-9-2-1 | 5120.6 | 89 |
| 84 | h442-456-A | T(Y)^A(Y)^5(Y)^a^g^c^t^g^g^t^t^g^G(Y)^A(Y)^c | 3-9-2-1 | 5121.5 | 90 |
| 85 | h444-458-A | T(Y)^G(Y)^T(Y)^a^c^a^g^c^t^g^g^t^T(Y)^G(Y)^g | 3-9-2-1 | 5138.9 | 91 |
| 86 | h446-460-A | A(Y)^A(Y)^T(Y)^g^t^a^c^a^g^c^t^g^G(Y)^T(Y)^t | 3-9-2-1 | 5105.9 | 92 |
| 87 | h447-461-A | A(Y)^A(Y)^A(Y)^t^g^t^a^c^a^g^c^t^G(Y)^G(Y)^t | 3-9-2-1 | 5115.7 | 93 |
| 88 | h451-465-A | T(Y)^T(Y)^5(Y)^c^a^a^a^t^g^t^a^c^A(Y)^G(Y)^c | 3-9-2-1 | 5049.5 | 94 |
| 89 | h125-139-B | 5(Y)^5(Y)^G(Y)^e^a^t^t^t^e^t^t^c^A(Y)^5(Y)^T(S) | 3-9-3 | 5130.4 | 95 |
| 90 | h125-139-C | 5(Y)-5(Y)-G(Y)^g^a^t^t^t^g^t^t^c^A(Y)-5(Y)-T(S) | 3-9-3 | 5066.3 | 96 |
| 91 | h124-140-A | 5(Y)^5(Y)^5(Y)^g^g^a^t^t^t^g^t^t^c^a^5(Y)^T(Y)^G (S) | 3-11-3 | 5794.7 | 97 |
| 92 | h124-140-B | 5(Y)-5(Y)-5(Y)^g^g^a^t^t^t^g^t^t^c^a^5(Y)-T(Y)-G(S) | 3-11-3 | 5730.7 | 98 |
| 93 | h123-141-A | 5(Y)^5(Y)^5(Y)^c^e^g^a^t^t^t^g^t^t^c^a^c^T(Y)^G(Y) ^G(S) | 3-13-3 | 6430.9 | 99 |
| 94 | h123-141-B | 5(Y)-5(Y)-5(Y)^c^g^g^a^t^t^t^g^t^t^c^a^c^T(Y)-G (Y)-G(S) | 3-13-3 | 6366.8 | 100 |
| 95 | h187-201-B | 5(Y)^T(Y)^A(Y)^a^g^t^t^a^t^t^g^a^G(Y)^5(Y)^T(S) | 3-9-3 | 5164.6 | 101 |
| 96 | h187-201-C | 5(Y)-T(Y)-A(Y)^a^g^t^t^a^t^t^g^a^G(Y)-5(Y)-T(S) | 3-9-3 | 5099.6 | 102 |
| 97 | h 186-202-A | A(Y)^5(Y)^T(Y)^a^a^g^t^t^a^t^t^g^a^g^5(Y)^T(Y)^T (S) | 3-11-3 | 5813.5 | 103 |
| 98 | h186-202-B | A(Y)-5(Y)-T(Y)^a^a^g^t^t^a^t^t^g^a^g^5(Y)-T(Y)-T (S) | 3-11-3 | 5748.8 | 104 |
| 99 | h185-203-A | G(Y)^A(Y)^5(Y)^t^a^a^g^t^t^a^t^t^g^a^g^c^T(Y)^T (Y)^G(S) | 3-13-3 | 6488.7 | 105 |
| 100 | h185-203-B | G(Y)-A(Y)-5(Y)^t^a^a^g^t^t^a^t^t^g^a^g^c^T(Y)-T (Y)-G(S) | 3-13-3 | 6424.9 | 106 |

[Table 3-5]

| Ex. | Sequence name | Antisense oligonucleotide sequence (5'-3') | X-Y-Z X-Y-Z-W | Mw (actual) | SEQ ID NO: |
|---|---|---|---|---|---|
| 101 | h213-227-B | A(Y)^T(Y)^5(Y)^a^t^a^g^g^t^a^g^c^T(Y)^T(Y)^T(S) | 3-9-3 | 5149.1 | 107 |
| 102 | h213-227-C | A(Y)-T(Y)-5(Y)^a^t^a^g^g^t^a^g^c^T(Y)-T(Y)-T(S) | 3-9-3 | 5085.9 | 108 |
| 103 | h212-228-A | T(Y)^A(Y)^T(Y)^c^a^t^a^g^g^t^a^g^c^t^T(Y)^T(Y)^G (S) | 3-11-3 | 5801.0 | 109 |
| 104 | h212-228-B | T(Y)-A(Y)-T(Y)^c^a^t^a^g^t^t^a^g^c^t^T(Y)-T(Y)-G (S) | 3-11-3 | 5737.2 | 110 |
| 105 | h211-229-A | T(Y)^T(Y)^A(Y)^t^c^a^t^a^g^g^t^a^g^c^t^t^T(Y)^G (Y)^T(S) | 3-13-3 | 6442.2 | 111 |
| 106 | h211-229-B | T(Y)-T(Y)-A(Y)^t^c^a^t^a^g^g^t^a^g^c^t^t^T(Y)-G (Y)-T(S) | 3-13-3 | 6377.7 | 112 |
| 107 | h326-340-B | T(Y)^T(Y)^A(Y)^c^t^a^a^g^g^a^g^c^T(Y)^5(Y)^5(S) | 3-9-3 | 5133.6 | 113 |
| 108 | h326-340-C | T(Y)-T(Y)-A(Y)^c^t^a^a^g^g^a^g^c^T(Y)-5(Y)-5(S) | 3-9-3 | 5069.8 | 114 |
| 109 | h325-341-A | T(Y)^T(Y)^T(Y)^a^c^t^a^a^g^g^a^g^c^t^5(Y)^5(Y)^T (S) | 3-11-3 | 5774.1 | 115 |
| 110 | h325-341-B | T(Y)-T(Y)-T(Y)^a^c^t^a^a^g^g^a^p^c^t^5(Y)-5(Y)-T (S) | 3-11-3 | 5710.2 | 116 |
| 111 | h324-342-A | 5(Y)^T(Y)^T(Y)^t^a^c^t^a^a^g^g^a^g^c^t^c^5(Y)^T (Y)^G(S) | 3-13-3 | 6424.7 | 117 |
| 112 | h324-342-B | 5(Y)-T(Y)-T(Y)^t^a^c^t^a^a^g^g^a^g^c^t^c^5(Y)-T (Y)-G(S) | 3-13-3 | 6360.9 | 118 |
| 113 | h444-458-B | T(v)^G(Y)^T(v)^a^c^a^g^c^t^g^g^t^T(Y)^G(v)^G(s) | 3-9-3 | 5192.8 | 119 |
| 114 | h444-458-C | T(Y)-G(Y)-T(Y)^a^c^a^g^c^t^g^g^t^T(Y)-G(Y)-G(S) | 3-9-3 | 5128.2 | 120 |
| 115 | h442-458-A | T(Y)^G(Y)^T(Y)^a^c^a^g^c^t^g^g^t^t^g^G(Y)^A(Y) ^5(S) | 3-11-3 | 5840.9 | 121 |
| 116 | h442-458-B | T(Y)-G(Y)-T(Y)^a^c^a^g^c^t^g^g^t^t^g^G(Y)-A(Y)-5 (S) | 3-11-3 | 5777.2 | 122 |
| 117 | h442-460-A | A(Y)^A(Y)^T(Y)^g^t^a^c^a^g^c^t^g^g^t^t^g^G(Y)^A (Y)^5(S) | 3-13-3 | 6500.1 | 123 |
| 118 | h442-460-B | A(Y)-A(Y)-T(Y)^g^t^a^c^a^g^c^t^g^g^t^t^g^G(Y)-A (Y)-5(S) | 3-13-3 | 6436.7 | 124 |
| 119 | h451-465-B | T(Y)^T(Y)^5(Y)^c^a^a^a^t^g^t^a^c^A(Y)^G(Y)^5(S) | 3-9-3 | 5117.7 | 125 |
| 120 | h451-465-C | T(Y)-T(Y)-5(Y)^c^a^a^a^t^g^t^a^c^A(Y)-G(Y)-5(S) | 3-9-3 | 5052.6 | 126 |
| 121 | h450-466-A | T(Y)^T(Y)^T(Y)^c^c^a^a^a^t^g^t^a^c^a^G(Y)^5(Y) ^T(S) | 3-11-3 | 5744.1 | 127 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 122 | h450-466-B | T(Y)-T(Y)-T(Y)^c^c^a^a^a^t^g^t^a^c^a^G(Y)-5(Y)-T (S) | 3-11-3 | 5680.2 | 128 |
| 123 | h449-467-A | T(Y)^T(Y)^T(Y)^t^c^c^a^a^a^t^g^t^a^c^a^g^5(Y)^T (Y)^G(S) | 3-13-3 | 6409.6 | 129 |
| 124 | h449-467-B | T(Y)-T(Y)-T(Y)^t^c^c^a^a^a^t^g^t^a^c^a^g^5(Y)-T (Y)-G(S) | 3-13-3 | 6345.4 | 130 |
| 125 | h39-53-A | G(Y)^5(Y)^T(Y)^c^g^g^a^g^a^a^t^a^G(Y)^5(Y)^a | 3-9-2-1 | 5153.9 | 131 |

[Table 3-6]

| Ex. | Sequence name | Antisense oligonucleotide sequence (5'-3') | X-Y-Z X-Y-Z-W | Mw (actual) | SEQ ID NO: |
|---|---|---|---|---|---|
| 126 | h40-54-A | A(Y)^G(Y)^5(Y)^t^c^g^g^a^g^a^a^t^A(Y)^G (Y)^c | 3-9-2-1 | 5139.9 | 132 |
| 127 | h98-112-A | T(Y)^T(Y)^5(Y)^t^t^g^g^c^c^g^a^c^T(Y)^T(Y) ^t | 3-9-2-1 | 5037.9 | 133 |
| 128 | h99-113-A | T(Y)^T(Y)^T(Y)^c^t^t^g^g^c^c^g^a^5(Y)^T(Y) ^t | 3-9-2-1 | 5038.7 | 134 |
| 129 | h100-114-A | 5(Y)^T(Y)^T(Y)^t^c^t^t^g^g^c^c^g^A(Y)^5(Y) ^t | 3-9-2-1 | 5037.9 | 135 |
| 130 | h104-118-A | T(Y)^T(Y)^G(Y)^t^c^t^t^t^c^t^t^g^G(Y)^5(Y) ^C | 3-9-2-1 | 5029.8 | 136 |
| 131 | h105-119-A | T(Y)^T(Y)^T(Y)^g^t^c^t^t^t^c^t^t^G(Y)^G(Y) ^c | 3-9-2-1 | 5030.6 | 137 |
| 132 | h106-120-A | 5(Y)^T(Y)^T(Y)^t^g^t^c^t^t^t^c^t^T(Y)^G(Y) ^g | 3-9-2-1 | 5044.5 | 138 |
| 133 | h107-121-A | T(Y)^5(Y)^T(Y)^t^t^g^t^c^t^t^t^c^T(Y)^T(Y)^g | 3-9-2-1 | 5019.3 | 139 |
| 134 | h123-137-C | G(Y)^G(Y)^A(Y)^t^t^t^g^t^t^c^a^c^T(Y)^G(Y) ^g | 3-9-2-1 | 5113.7 | 140 |
| 135 | h127-141-A | 5(Y)^5(Y)^5(Y)^c^g^g^a^t^t^t^g^t^T(Y)^5(Y) ^a | 3-9-2-1 | 5075.2 | 141 |
| 136 | h128-142-A | 5(Y)^5(Y)^5(Y)^c^c^g^g^a^t^t^t^g^T(Y)^T(Y) ^c | 3-9-2-1 | 5036.3 | 142 |
| 137 | h129-143-A | G(Y)^5(Y)^5(Y)^c^c^c^g^g^a^t^t^t^G(Y)^T(Y) ^t | 3-9-2-1 | 5062.3 | 143 |
| 138 | h130-144-A | T(Y)^G(Y)^5(Y)^c^c^c^c^g^g^a^t^t^T(Y)^G (Y)^t | 3-9-2-1 | 5048.9 | 144 |
| 139 | h185-199-C | A(Y)^A(Y)^G(Y)^t^t^a^t^t^g^a^g^c^T(Y)^T(Y) ^g | 3-9-2-1 | 5120.7 | 145 |
| 140 | h186-200-C | T(Y)^A(Y)^A(Y)^g^t^t^a^t^t^g^a^g^5(Y)^T(Y) ^t | 3-9-2-1 | 5110.2 | 146 |
| 141 | h190-204-A | A(Y)^G(Y)^A(Y)^c^t^a^a^g^t^t^a^t^T(Y)^G(Y) ^a | 3-9-2-1 | 5114.2 | 147 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 142 | h211-225-A | 5(Y)^A(Y)^T(Y)^a^g^g^ta^g^c^t^t^T(Y)^G(Y)^t | 3-9-2-1 | 5111.7 | 148 |
| 143 | h212-226-A | T(Y)^5(Y)^A(Y)^t^a^g^g^t^a^g^c^t^T(Y)^T(Y)^g | 3-9-2-1 | 5111.6 | 149 |
| 144 | h215-229-A | T(Y)^T(Y)^A(Y)^t^c^a^t^a^g^g^t^a^G(Y)^5(Y)^t | 3-9-2-1 | 5095.0 | 150 |
| 145 | h218-232-A | A(Y)^G(Y)^T(Y)^t^t^a^t^c^a^t^a^g^G(Y)^T(Y)^a | 3-9-2-1 | 5106.1 | 151 |
| 146 | h221-235-A | 5(Y)^A(Y)^G(Y)^a^g^t^t^t^a^t^c^a^T(Y)^A(Y)^g | 3-9-2-1 | 5104.9 | 152 |
| 147 | h222-236-A | A(Y)^5(Y)^A(Y)^g^a^g^t^t^t^a^t^c^A(Y)^T(Y)^a | 3-9-2-1 | 5088.0 | 153 |
| 148 | h223-237-A | T(Y)^A(Y)^5(Y)^a^g^a^g^t^t^t^a^t^5(Y)^A(Y)^t | 3-9-2-1 | 5093.1 | 154 |
| 149 | h224-238-A | T(Y)^T(Y)^A(Y)^c^a^g^a^g^t^t^t^a^T(Y)^5(Y)^a | 3-9-2-1 | 5089.8 | 155 |
| 150 | h255-269-A | T(Y)^G(Y)^G(Y)^g^g^t^t^a^t^a^a^g^T(Y)^T(Y)^t | 3-9-2-1 | 5132.1 | 156 |

[Table 3-7]

| Ex. | Sequence name | Antisense oligonucleotide sequence (5'-3') | X-Y-Z X-Y-Z-W | Mw (actual) | SEQ ID NO: |
|---|---|---|---|---|---|
| 151 | h256-270-A | 5(Y)^T(Y)^G(Y)^g^g^g^t^t^a^t^a^a^G(Y)^T(Y)^t | 3-9-2-1 | 5151.4 | 157 |
| 152 | h257-271-A | G(Y)^5(Y)^T(Y)^g^g^g^g^t^t^a^t^a^A(Y)^G(Y)^t | 3-9-2-1 | 5176.4 | 158 |
| 153 | h258-272-A | A(Y)^G(Y)^5(Y)^t^g^g^g^g^t^t^a^t^A(Y)^A(Y)^g | 3-9-2-1 | 5185.1 | 159 |
| 154 | h262-276-A | 5(Y)^5(Y)^A(Y)^c^a^g^c^t^g^g^g^g^T(Y)^T(Y)^a | 3-9-2-1 | 5121.0 | 160 |
| 155 | h263-277-A | A(Y)^5(Y)^5(Y)^a^c^a^g^c^t^g^g^g^G(Y)^T(Y)^t | 3-9-2-1 | 5120.6 | 161 |
| 156 | h264-278-A | G(Y)^A(Y)^5(Y)^c^a^c^a^g^c^t^g^g^G(Y)^G(Y)^t | 3-9-2-1 | 5132.2 | 162 |
| 157 | h291-305-A | G(Y)^G(Y)^A(Y)^g^c^c^t^c^g^a^a^t^5(Y)^T(Y)^t | 3-9-2-1 | 5081.7 | 163 |
| 158 | h292-306-A | G(Y)^G(Y)^G(Y)^a^g^c^c^t^c^g^a^a^T(Y)^5(Y)^t | 3-9-2-1 | 5106.6 | 164 |
| 159 | h293-307-A | A(Y)^G(Y)^G(Y)^g^a^g^c^c^t^c^g^a^A(Y)^T(Y)^c | 3-9-2-1 | 5101.4 | 165 |
| 160 | h294-308-A | 5(Y)^A(Y)^G(Y)^g^g^a^g^c^c^t^c^g^A(Y)^A(Y)^t | 3-9-2-1 | 5116.2 | 166 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 161 | h298-312-A | T(Y)^G(Y)^G(Y)^c^c^a^g^g^g^a^g^c^5(Y)^T (Y)^c | 3-9-2-1 | 5107.9 | 167 |
| 162 | h299-313-A | 5(Y)^T(Y)^G(Y)^g^c^c^a^g^g^g^a^g^5(Y)^5 (Y)^t | 3-9-2-1 | 5135.0 | 168 |
| 163 | h300-314-A | 5(Y)^5(Y)^T(Y)^g^g^c^c^a^g^g^g^a^G(Y)^5 (Y)^c | 3-9-2-1 | 5120.7 | 169 |
| 164 | h321-335-A | A(Y)^A(Y)^G(Y)^g^a^g^c^t^c^c^t^g^A(Y)^A(Y) ^g | 3-9-2-1 | 5126.1 | 170 |
| 165 | h322-336-A | T(Y)^A(Y)^A(Y)^g^g^a^g^c^t^c^c^t^G(Y)^A(Y) ^a | 3-9-2-1 | 5100.9 | 171 |
| 166 | h323-337-A | 5(Y)^T(Y)^A(Y)^a^g^g^a^c^t^c^c^T(Y)^G(Y)^a | 3-9-2-1 | 5091.1 | 172 |
| 167 | h324-338-A | A(Y)^5(Y)^T(Y)^a^a^g^g^a^g^c^t^c^5(Y)^T(Y) ^g | 3-9-2-1 | 5104.5 | 173 |
| 168 | h328-342-A | 5(Y)^T(Y)^T(Y)^t^a^c^t^a^a^g^g^a^G(Y)^5(Y) ^t | 3-9-2-1 | 5094.6 | 174 |
| 169 | h329-343-A | 5(Y)^5(Y)^T(Y)^t^t^a^c^t^a^a^g^g^A(Y)^G(Y) ^c | 3-9-2-1 | 5078.6 | 175 |
| 170 | h330-344-A | T(Y)^5(Y)^5(Y)^t^t^t^a^c^t^a^a^g^G(Y)^A(Y) ^g | 3-9-2-1 | 5094.7 | 176 |
| 171 | h331-345-A | G(Y)^T(Y)^5(Y)^c^t^t^t^a^c^t^a^a^G(Y)^G(Y) ^a | 3-9-2-1 | 5080.2 | 177 |
| 172 | h426-440-A | T(Y)^A(Y)^T(Y)^t^c^a^t^g^c^a^t^c^T(Y)^T(Y) ^c | 3-9-2-1 | 4992.6 | 178 |
| 173 | h427-441-A | 5(Y)^T(Y)^A(Y)^t^t^c^a^t^g^c^a^t^5(Y)^T(Y)^t | 3-9-2-1 | 5020.5 | 179 |
| 174 | h428-442-A | 5(Y)^5(Y)^T(Y)^a^t^t^c^a^t^g^c^a^T(Y)^5(Y)^t | 3-9-2-1 | 5119.7 | 180 |
| 175 | h429-443-A | A(Y)^5(Y)^5(Y)^t^a^t^t^c^g^t^g^c^A(Y)^T(Y) ^c | 3-9-2-1 | 5014.8 | 181 |

[Table 3-8]

| Ex. | Sequence name | Antisense oligonucleotide sequence (5'-3') | X-Y-Z X-Y-Z-W | Mw (actual) | SEQ ID NO: |
|---|---|---|---|---|---|
| 176 | h437-451-A | 5(Y)^T(Y)^G(Y)^g^t^t^g^g^a^c^c^t^A(Y)^T(Y) ^t | 3-9-2-1 | 5087.9 | 182 |
| 177 | h443-457-A | G(Y)^T(Y)^A(Y)^c^a^e^c^t^g^g^t^t^G(Y)^G (Y)^a | 3-9-2-1 | 5147.2 | 183 |
| 178 | h445-459-A | A(Y)^T(Y)^G(Y)^t^a^c^a^g^c^t^g^g^T(Y)^T (Y)^g | 3-9-2-1 | 5122.2 | 184 |
| 179 | h448-462-A | 5(Y)^A(Y)^A(Y)^a^t^g^t^a^c^a^g^c^T(Y)^G (Y)^g | 3-9-2-1 | 5114.3 | 185 |
| 180 | h449-463-A | 5(Y)^5(Y)^A(Y)^a^g^t^g^t^a^c^a^g^5(Y)^T(Y) ^g | 3-9-2-1 | 5102.7 | 186 |
| 181 | h450-464-A | T(Y)^5(Y)^5(Y)^a^a^a^t^g^t^a^c^a^G(Y)^5(Y) ^t | 3-9-2-1 | 5077.5 | 187 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 182 | h452-466-A | T(Y)^T(Y)^T(Y)^c^c^a^a^a^t^g^t^a^5(Y)^A(Y)^g | 3-9-2-1 | 5065.0 | 188 |
| 183 | h453-467-A | T(Y)^T(Y)^T(Y)^t^c^c^a^a^a^t^g^t^A(Y)^5(Y)^a | 3-9-2-1 | 5039.7 | 189 |
| 184 | h454-468-A | T(Y)^T(Y)^T(Y)^t^t^c^c^a^a^a^t^g^T(Y)^A(Y)^c | 3-9-2-1 | 5016.0 | 190 |
| 185 | h455-469-A | A(Y)^T(Y)^T(Y)^t^t^t^c^c^a^a^a^t^G(Y)^T(Y)^a | 3-9-2-1 | 5041.2 | 191 |

[Table 3-9]

| Ex. | Sequence name | Antisense oligonucleotide sequence (5'-3') | X-Y-Z X-Y-Z-W | Mw (actual) | SEQ ID NO: |
|---|---|---|---|---|---|
| 297 | h125-140-A | 5(Y)-5(Y)-C(M)-G(Y)^g^a^t^t^t^g^t^t^c^A(Y)-5(Y)-T(S) | 4-9-3 | 5385.0 | 303 |
| 298 | h125-140-B | 5(Y)-5(Y)-5(Y)^g^g^a^t^t^t^p^t^t^c^A(Y)-5(Y)-T(S) | 4-9-3 | 5384.9 | 304 |
| 299 | h124-140-C | 5(Y)-5(Y)-C(M)-G(Y)^g^a^t^t^t^g^t^t^c^a-5(S)-T(Y)-G(GtB) | 3-11-3 | 5784.1 | 305 |
| 300 | h187-201-D | 5(Y)-T(Y)-A(Y)-a^g^t^t^a^t^t^g^a^G(Y)-5(Y)-T(S) | 3-9-3 | 5084.0 | 306 |
| 301 | h187-201-E | 5(S)-T(S)-A(Y)-a^g^t^t^a^t^t^g^a^G(Y)-5(Y)-T(S) | 3-9-3 | 5081.5 | 307 |
| 302 | h186-201-A | 5(S)-T(Y)-A(Y)^a^g^t^t^a^t^t^g^a^G(Y)-C(M)-T(Y)-T(S) | 3-9-4 | 5418.5 | 308 |
| 303 | h186-202-A1 | A(Y)-5(Y)-T(S)-a^a^g^t^t^a^t^t^g^a^G(Y)-C(M)-T(Y)-T(S) | 3-10-4 | 5732.1 | 309 |
| 304 | h186-202-B1 | A(Y)-5(Y)-T(Y)-a^a^g^t^t^a^t^t^g^a^p^5(Y)-T(Y)-T(S) | 3-10-4 | 5733.5 | 310 |
| 305 | h186-202-C | A(Y)-5(S)-T(S)-a^a^g^t^t^a^t^t^g^a^g^5(Y)-T(Y)-T(S) | 3-10-4 | 5730.5 | 311 |
| 306 | h186-203-A | G(Y)-A(Y)-C(M)-T(Y)^a^a^g^t^t^a^t^t^g^a^g-5(S)-T(Y)-T(S) | 4-11-3 | 6077.2 | 312 |
| 307 | h214-228-B | T(Y)-A(Y)-T(S)-c^a^t^a^g^g^t^a^g^5(Y)-T(Y)-T(S) | 3-9-3 | 5068.3 | 313 |
| 308 | h213-228-A | T(S)-A(Y)-U(M)-5(Y)^a^t^a^g^g^t^t^a^g^c-T(Y)-T(Y)-T(S) | 4-9-3 | 5389.1 | 314 |
| 309 | h214-229-A | T(S)-T(Y)-A(M)-T(Y)^c^a^t^a^g^g^t^a^g-5(S)-T(Y)-T(S) | 4-9-3 | 5401.4 | 315 |
| 310 | h259-273-B | 5(S)-A(Y)-G(Y)^c^t^g^g^g^t^t^a^T(Y)-A(Y)-A(GtB) | 3-9-3 | 5206.1 | 316 |
| 311 | h265-279-A | A(Y)-G(Y)-A(Y)^c^c^a^c^a^g^c^t^g^G(Y)-G(Y)-G(GtB) | 3-9-3 | 5188.0 | 317 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 312 | h266-280-A | G(Y)-A(Y)-G(Y)^a^c^c^a^c^a^g^c^t^G(Y)-G(Y)-G (GtB) | 3-9-3 | 5187.9 | 318 |
| 313 | h267-281-A | A(Y)-G(Y)-A(Y)^g^a^c^c^a^c^a^g^c^T(Y)-G(Y)-G (GtB) | 3-9-3 | 5171.4 | 319 |
| 314 | h268-282-A | 5(Y)-A(Y)-G(Y)^a^g^a^c^c^a^c^a^g^5(Y)-T(Y)-G(GtB) | 3-9-3 | 5160.1 | 320 |
| 315 | h259-274-A | A(Y)-5(Y)-A(M)-g^c^t^g^g^g^g^t^t^a^T(Y)-A(Y)-A (GtB) | 3-10-3 | 5494.7 | 321 |
| 316 | h263-279-A | A(Y)-G(Y)-A(Y)-c^c^a^c^a^g^c^t^g^g^g^G(Y)-T(S)-T (S) | 3-11-3 | 5740.0 | 322 |
| 317 | h264-280-A | G(Y)-A(Y)-G(Y)-a^c^c^a^c^a^g^c^t^g^g^G(Y)-G(Y)-T (S) | 3-11-3 | 5766.1 | 323 |
| 318 | h295-309-B | 5(Y)^5(Y)-A(Y)-g^g^g^a^g^c^c^t^c^G(Y)-A(Y)-A(GtB) | 3-9-3 | 5176.0 | 324 |
| 319 | h295-309-C | 5(S)-5(Y)-A(Y)-g^g^g^a^g^c^c^t^c^G(Y)-A(Y)-A(GtB) | 3-9-3 | 5159.1 | 325 |
| 320 | h296-310-B | G(Y)-5(Y)-5(Y)^a^g^g^g^a^g^c^c^t^5(S)-G(Y)-A(GtB) | 3-9-3 | 5204.6 | 326 |
| 321 | h296-310-C | G(Y)-5(Y)-5(Y)^a^g^g^g^a^g^c^c^t-5(S)-G(Y)-A(GtB) | 3-9-3 | 5188.6 | 327 |

[Table 3-10]

| Ex. | Sequence name | Antisense oligonucleotide sequence (5'-3') | X-Y-Z X-Y-Z-W | Mw (actual) | SEQ ID NO: |
|---|---|---|---|---|---|
| 322 | h300-314-B | 5(S)-5(Y)-T(S)^g^g^c^c^a^g^g^g^a^G(Y)-5(Y)-5(S) | 3-9-3 | 5122.3 | 328 |
| 323 | h300-314-C | 5(S)-5(Y)-T(S)-g^g^c^c^a^g^g^g^a^G(Y)-5(Y)-5(S) | 3-9-3 | 5107.0 | 329 |
| 324 | h301-315-A | 5(Y)-5(Y)-5(Y)^t^g^g^c^c^a^g^g^g^A(Y)-G(Y)-5(S) | 3-9-3 | 5124.5 | 330 |
| 325 | h302-316-A | G(Y)-5(Y)-5(Y)^c^t^g^g^c^c^a^g^g^G(Y)-A(Y)-G (GtB) | 3-9-3 | 5207.5 | 331 |
| 326 | h303-317-A | T(Y)-G(Y)-5(Y)^c^c^t^g^g^c^c^a^g^G(Y)-G(Y)-A (GtB) | 3-9-3 | 5169.0 | 332 |
| 327 | h304-318-A | 5(Y)-T(Y)-G(Y)^c^c^c^t^g^g^c^c^a^G(Y)-G(Y)-G (GtB) | 3-9-3 | 5144.3 | 333 |
| 328 | h296-311-A | G(Y)-G(Y)-5(Y)^c^a^g^g^g^a^g^c^c^t^5(S)-G(Y)-A (GtB) | 3-10-3 | 5535.9 | 334 |
| 329 | h296-311-B | G(Y)-G(Y)-5(S)-c^a^g^g^g^a^g^c^c^t-5(S)-G(Y)-A (GtB) | 3-10-3 | 5502.3 | 335 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 330 | h295-311-A | G(Y)-G(Y)-5(S)-c^a^g^g^g^a^g^c^c^t^c^G(Y)-A(Y)-A (GtB) | 3-11-3 | 5835.0 | 336 |
| 331 | h300-316-A | G(Y)-5(S)-5(S)-c^t^g^g^c^c^a^g^g^g^a^G(Y)-5(Y)-5 (S) | 3-11-3 | 5757.1 | 337 |
| 332 | h323-337-B | 5(S)-T(Y)-A(Y)^a^g^g^a^g^c^t^c^c^T(Y)-G(Y)-A (GtB) | 3-9-3 | 5150.5 | 338 |
| 333 | h323-337-C | 5(S)-T(Y)-A(Y)-a^g^g^a^g^c^t^c^c^T(Y)-G(Y)-A(GtB) | 3-9-3 | 5134.2 | 339 |
| 334 | h326-340-D | T(S)-T(Y)-A(Y)^c^t^a^a^g^g^a^g^c^T(Y)-5(Y)-5(S) | 3-9-3 | 5068.8 | 340 |
| 335 | h326-340-E | T(S)-T(Y)-A(Y)^c^t^a^a^g^g^a^e^c^T(S)-5(Y)-5(S) | 3-9-3 | 5068.0 | 341 |
| 336 | h325-340-A | T(S)-T(Y)-A(Y)^c^t^a^a^g^g^a^g^c^T(Y)-C(M)-5 (Y)-T(S) | 3-9-4 | 5389.0 | 342 |
| 337 | h325-340-B | T(S)-T(Y)-A(Y)^c^t^a^a^g^g^a^g^c-T(S)-5(Y)-5(Y)-T (S) | 3-9-4 | 5410.5 | 343 |
| 338 | h326-341-A | T(S)-T(Y)-U(M)-A(Y)^c^t^a^a^g^g^a^g^c^T(Y)-5(Y)-5 (S) | 4-9-3 | 5389.1 | 344 |
| 339 | h326-341-B | T(S)-T(Y)-U(M)-A(Y)^c^t^a^a^g^g^a^g^c-T(S)-5(Y)-5 (S) | 4-9-3 | 5372.1 | 345 |
| 340 | h326-341-C | T(S)-T(Y)-T(Y)-a^c^t^a^a^g^g^a^g^c-T(S)-5(Y)-5(S) | 3-10-3 | 5356.0 | 346 |
| 341 | h326-341-D | T(Y)-T(Y)-T(Y)-a^c^t^a^a^g^g^a^g-5(S)-U(M)-5(Y)^5 (S) | 3-9-4 | 5386.9 | 347 |
| 342 | h326-341-E | T(S)-T(Y)-T(S)-a^c^t^a^a^g^g^a^g-5(S)-U(M)-5(Y)^5 (S) | 3-9-4 | 5384.6 | 348 |
| 343 | h322-338-A | A(Y)-5(Y)-T(S)^a^a^g^g^a^g^c^t^c^c^t^G(Y)-A(Y)-A (GtB) | 3-11-3 | 5809.1 | 349 |
| 344 | h322-338-B | A(Y)-5(Y)-T(S)-a^a^g^g^a^g^c^t^c^c^t^G(Y)-A(Y)-A (GtB) | 3-11-3 | 5793.6 | 350 |
| 345 | h325-341-C | T(Y)^T(Y)-T(S)-a^c^t^a^a^g^g^a^g^c^t-5(S)-5(Y)^T (S) | 3-11-3 | 5708.2 | 351 |
| 346 | h325-341-D | T(Y)-T(Y)-T(S)^a^c^t^a^a^g^g^a^g^c^t^5(Y)-5(Y)-T (S) | 3-11-3 | 5708.7 | 352 |

[Table 3-11]

| Ex. | Sequence name | Antisense oligonucleotide sequence (5'-3') | X-Y-Z X-Y-Z-W | Mw (actual) | SEQ ID NO: |
|---|---|---|---|---|---|
| 347 | h325-341-E | T(S)-T(Y)-T(S)^a^c^t^a^a^g^g^a^g^c^t^5(Y)-5(Y)-T (S) | 3-11-3 | 5707.8 | 353 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 348 | h325-341-F | T(Y)-T(Y)-T(Y)-a^c^t^a^a^g^g^a^g^c^t^5(Y)-5(Y)-T (S) | 3-11-3 | 5694.0 | 354 |
| 349 | h325-341-G | T(S)-T(Y)-T(S)-a^c^t^a^a^g^g^a^g^c^t^5(Y)-5(Y)-T (S) | 3-11-3 | 5692.3 | 355 |
| 350 | h439-453-B | A(Y)-G(Y)-5(S)-t^g^g^t^t^g^a^c^5(Y)-T(Y)-A(GtB) | 3-9-3 | 5179.2 | 356 |
| 351 | h440-454-B | 5(Y)-A(Y)-G(Y)^c^t^g^g^t^t^g^g^a^5(Y)-5(Y)-T(S) | 3-9-3 | 5115.6 | 357 |
| 352 | h440-454-C | 5(Y)-A(Y)-G(Y)^c^t^g^g^t^t^g^g^a-5(S)-5(Y)-T(S) | 3-9-3 | 5099.7 | 358 |
| 353 | h440-454-D | 5(Y)-A(Y)-G(Y)-c^t^g^g^t^t^g^g^a^5(Y)-5(Y)-T(S) | 3-9-3 | 5099.7 | 359 |
| 354 | h440-454-E | 5(Y)-A(Y)-G(Y)-c^t^g^g^t^t^g^g^a-5(S)-5(Y)-T(S) | 3-9-3 | 5082.6 | 360 |
| 355 | h451-465-D | T(Y)-T(Y)-5(S)^c^a^a^a^t^g^t^a^c^A(Y)-G(Y)-5(S) | 3-9-3 | 5055.0 | 361 |
| 356 | h451-465-E | T(S)-T(Y)-5(S)^c^a^a^a^t^g^t^a^c^A(Y)-G(Y)-5(S) | 3-9-3 | 5051.5 | 362 |
| 357 | h451-465-F | T(Y)-T(Y)-5(S)-c^a^a^a^t^g^t^a^c^A(Y)-G(Y)-5(S) | 3-9-3 | 5036.4 | 363 |
| 358 | h451-465-G | T(S)-T(Y)-5(S)-c^a^a^a^t^g^t^a^c^A(Y)-G(Y)-5(S) | 3-9-3 | 5035.5 | 364 |
| 359 | h451-465-H | T(Y)-T(Y)-5(Y)^c^a^a^a^t^g^t^a^5(Y)-A(M)-G(Y)-5(S) | 3-8-4 | 5082.0 | 365 |
| 360 | h439-454-A | 5(Y)-A(Y)-G(Y)-5(Y)^t^g^g^t^t^g^g^a^c^5(Y)-T(Y)-A (GtB) | 4-9-3 | 5555.1 | 366 |
| 361 | h439-454-B | 5(Y)-A(Y)-G(Y)-5(Y)-t^g^g^t^t^g^g^a^c^5(Y)-T(Y)-A (GtB) | 4-9-3 | 5538.6 | 367 |
| 362 | h439-454-C | 5(Y)-A(Y)-G(Y)-5(S)t^g^g^t^t^g^g^a^c^5(Y)-T(Y)-A (GtB) | 4-9-3 | 5538.0 | 368 |
| 363 | h442-457-A | G(Y)-T(Y)-A(Y)^c^a^g^c^t^g^g^t^t^G(Y)-G(Y)-A(Y)-5 (S) | 3-9-4 | 5495.9 | 369 |
| 364 | h442-457-B | G(Y)-T(Y)-A(Y)-c^a^g^c^t^g^g^t^t^g^G(Y)-A(Y)-5(S) | 3-10-3 | 5440.8 | 370 |
| 365 | h443-458-A | T(Y)-G(Y)-T(Y)-a^c^a^g^c^t^g^g^t^T(Y)-G(M)-G(Y)-A (GtB) | 3-9-4 | 5526.9 | 371 |
| 366 | h450-465-A | T(Y)-T(Y)-5(Y)^c^a^a^a^t^g^t^a^c^a^G(Y)-5(Y)-T(S) | 3-10-3 | 5373.7 | 372 |
| 367 | h450-465-B | T(Y)-T(Y)-5(Y)^c^a^a^a^t^g^t^a^c^A(Y)-G(Y)-5(Y)-T (S) | 3-9-4 | 5412.6 | 373 |
| 368 | h450-465-C | T(Y)-T(Y)-5(S)^c^a^a^a^t^g^t^a^c^A(Y)-G(Y)-5(Y)-T (S) | 3-9-4 | 5411.6 | 374 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 369 | h450-465-D | T(Y)-T(Y)-5(Y)^c^a^a^a^t^g^t^a^c^A(Y)-G(M)-5(Y)-T (S) | 3-9-4 | 5388.1 | 375 |
| 370 | h451-466-A | T(Y)-T(Y)-T(Y)^c^c^a^a^a^t^g^t^a^c^A(Y)-G(Y)-5(S) | 3-10-3 | 5359.8 | 376 |
| 371 | h451-466-B | T(Y)-T(Y)-T(Y)^c^c^a^a^a^t^g^t^a^5(Y)-A(Y)-G(Y)-5 (S) | 3-9-4 | 5412.5 | 377 |

[Table 3-12]

| Ex. | Sequence name | Antisense oligonucleotide sequence (5'-3') | X-Y-Z X-Y-Z-W | Mw (actual) | SEQ ID NO: |
|---|---|---|---|---|---|
| 372 | h451-466-C | T(Y)-T(Y)-T(S)^c^c^a^a^a^t^g^t^a^5(Y)-A(Y)-G(Y)-5(S) | 3-9-4 | 5411.7 | 378 |
| 373 | h451-466-D | T(Y)-T(Y)-T(S)^c^c^a^a^a^t^g^t^a^5(S)-A(Y)-G(Y)-5(S) | 3-9-4 | 5410.6 | 379 |
| 374 | h451-466-E | T(Y)-T(Y)-T(Y)-c^c^a^a^a^t^g^t^a^5(Y)-A(Y)-G(Y)-5(S) | 3-9-4 | 5396.8 | 380 |
| 375 | h451-466-F | T(Y)-T(Y)-T(Y)^c^c^a^a^a^t^g^t^a^5(Y)-A(M)-G(Y)-5(S) | 3-9-4 | 5387.9 | 381 |
| 376 | h429-445-A | G(Y)-G(Y)-A(Y)^c^c^t^a^t^t^c^a^t^g^c^A(Y)-T(Y)-5(S) | 3-11-3 | 5681.2 | 382 |
| 377 | h430-446-A | T(Y)-G(Y)-G(Y)-a^c^c^t^a^t^t^c^a^t^g^5(Y)^A(Y)-T(S) | 3-11-3 | 5695.8 | 383 |
| 378 | h434-450-A | T(Y)-G(Y)-G(Y)^t^t^g^g^a^c^c^t^a^t^t^5(Y)-A(Y)-T(S) | 3-11-3 | 5727.0 | 384 |
| 379 | h439-455-A | A(Y)-5(Y)-A(Y)-g^c^t^g^a^t^t^g^g^a^c^5(Y)-T(Y)-A(GtB) | 3-11-3 | 5830.9 | 385 |
| 380 | h441-457-A | G(Y)-T(Y)-A(Y)-c^a^g^c^t^g^g^t^t^g^g^A(Y)-5(Y)-5(S) | 3-11-3 | 5760.0 | 386 |
| 381 | h441-457-B | G(Y)-T(S)-A(Y)-c^a^g^c^t^g^g^t^t^g^g^A(Y)-5(S)-5(S) | 3-11-3 | 5757.7 | 387 |
| 382 | h442-458-C | T(Y)-G(Y)-T(Y)^a^c^a^g^c^t^g^g^t^t^G(Y)-G(Y)-A(Y)-5(S) | 3-10-4 | 5816.2 | 388 |
| 383 | h442-458-D | T(Y)-G(Y)-T(S)-a^c^a^g^c^t^g^g^t^t^G(Y)-G(Y)-A(Y)-5(S) | 3-10-4 | 5798.8 | 389 |
| 384 | h442-458-E | T(Y)-G(Y)-T(Y)-A(Y)^c^a^g^c^t^g^g^t^t^g^G(Y)-A(Y)-5(S) | 4-10-3 | 5816.8 | 390 |
| 385 | h443-459-A | A(Y)-T(Y)-G(Y)^t^a^c^a^g^c^t^g^g^t-T(S)-G(Y)-G(Y)-A(GtB) | 3-10-4 | 5880.2 | 391 |
| 386 | h449-465-A | T(S)-T(Y)-5(S)^c^a^a^a^t^e^t^a^c^a^Q^5(Y)-T(Y)-G(GtB) | 3-11-3 | 5788.6 | 392 |
| 387 | h449-465-B | T(Y)-T(Y)-5(Y)-c^a^a^a^t^g^t^a^c^a^g^5(Y)-T(Y)-G(GtB) | 3-11-3 | 5774.1 | 393 |
| 388 | h449-465-C | T(S)-T(Y)-5(S)-c^a^a^a^t^g^t^a^c^a^g^5(Y)-T(Y)-G(GtB) | 3-11-3 | 5772.5 | 394 |
| 389 | h449-465-D | T(Y)-T(Y)-5(S)-c^a^a^a^t^g^t^a^c^a^g-5(S)-T(Y)-G(GtB) | 3-11-3 | 5756.1 | 395 |
| 390 | h449-465-E | T(Y)-T(Y)-5(Y)^c^a^a^a^t^g^t^a^c^a^G(Y)-C(M)-T(Y)-G(GtB) | 3-10-4 | 5790.1 | 396 |
| 391 | h449-467-C | T(Y)^T(Y)-U(M)-T(Y)^c^c^a^a^a^t^g^t^a^c^a^G(Y)-C(M)-T(Y)^G(GtB) | 4-11-4 | 6449.0 | 397 |
| 392 | h442-458-F | T(Y)^G(m)-T(m)-A(m)^5(x)^a^g^5(x)^t^g^g^t^t^G(m)-G(m)-A(Y)^5(m) | 4-9-4 | 6030.2 | 398 |
| 393 | h442-460-C | A(m)^A(m)-T(m)-G(m)-T(m)^a^5(x)^a^g^5(x)^t^g^g^t^T(m)-G(m)-G(m)-A(m)^5(m) | 5-9-5 | 6840.3 | 399 |

[Table 3-13]

| Ex. | Sequence name | Antisense oligonucleotide sequence (5'-3') | X-Y-Z X-Y-Z-W | Mw (actual) | SEQ ID NO: |
|---|---|---|---|---|---|
| 394 | h442-459-A | A(Y)^T(m)-G(m)-T(m)^a^5(x)^a^g^5(x)^t^g^g^t^T(m)-G(m)-G(m)-A(m)^5(m) | 4-9-5 | 6435.5 | 400 |
| 395 | h442-459-B | A(m)^T(m)-G(m)-T(m)-A(m)^5(x)^a^g^5(x)^t^g^g^t^t^G(m)-G(m)-A(Y)^5(m) | 5-9-4 | 6436.0 | 401 |
| 396 | h443-460-A | A(m)^A(m)-T(m)-G(m)-T(m)^a^5(x)^a^g^5(x)^t^g^g^t^T(m)-G(m)-G(Y)^A(m) | 5-9-4 | 6446.4 | 402 |
| 397 | h443-460-B | A(Y)^A(m)-T(m)-G(m)^t^a^5(x)^a^g^5(x)^t^g^g^T(m)-T(m)-G(m)-G(m)^A(m) | 4-9-5 | 6446.1 | 403 |
| 398 | h447-465-A | T(m)^T(m)-5(m)-5(m)-A(m)^a^a^t^g^t^a^5(x)^a^g^5(m)-T(m)-G(m)-G(m)^T(m) | 5-9-5 | 6790.5 | 404 |
| 399 | h448-465-A | T(m)^T(m)-5(m)-5(m)-A(m)^a^a^t^g^t^a^5(x)^a^g^5(m)-T(m)-G(Y)^G(m) | 5-9-4 | 6394.2 | 405 |
| 400 | h448-465-B | T(Y)^T(m)-5(m)-5(m)^a^a^a^t^g^t^a^5(x)^a^G(m)-5(m)-T(m)-G(m)^G(m) | 4-9-5 | 6394.7 | 406 |
| 401 | h449-466-A | T(Y)^T(m)-T(m)-5(m)^5(x)^a^a^a^t^g^t^a^5(x)^A(m)-G(m)-5(m)-T(m)^G(m) | 4-9-5 | 6368.5 | 407 |
| 402 | h449-466-B | T(m)^T(m)-T(m)-5(m)-5(m)^a^a^a^t^g^t^a^5(x)^a^G(m)-5(m)-T(Y)^G(m) | 5-9-4 | 6371.9 | 408 |
| 403 | h450-467-A | T(m)^T(m)-T(m)-T(m)-5(m)^5(x)^a^a^a^t^g^t^a^5(x)^A(m)-G(m)-5(Y)^T(m) | 5-9-4 | 6345.4 | 409 |
| 404 | h448-466-A | T(m)^T(m)-T(m)-5(m)-5(m)^a^a^a^t^g^t^a^5(x)^a^G(m)-5(m)-T(m)-G(m)^G(m) | 5-9-5 | 6790.0 | 410 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 405 | h450-467-B | T(Y)^T(m)-T(m)-T(m)^5(x)^5(x)^a^a^a^t^g^t^a^5(m)-A(m)-G(m)-5(m)^T(m) | 4-9-5 | 6344.7 | 411 |
| 406 | h450-468-A | T(m)^T(m)-T(m)-T(m)-T(m)^5(x)^5(x)^a^a^a^t^g^t^a^5(m)-A(m)-G(m)-5(m)^T(m) | 5-9-5 | 6738.9 | 412 |

[Table 3-14]

| Ex. | Sequence name | Antisense oligonucleotide sequence (5'-3') | X-Y-Z X-Y-Z-W | Mw (actual) | SEQ ID NO: |
|---|---|---|---|---|---|
| 407 | h451-467-A | T(Y)^T(m)-T(m)-T(m)^5(x)^5(x)^a^a^a^t^g^t^a^5(m)-A(m)-G(Y)^5(m) | 4-9-4 | 5947.8 | 413 |
| 408 | h451-468-A | T(m)^T(m)-T(m)-T(m)-T(m)^5(x)^5(x)^a^a^a^t^g^t^a^5(m)-A(m)-G(Y)^5(m) | 5-9-4 | 6344.8 | 414 |
| 409 | h451-468-B | T(Y)^T(m)-T(m)-T(m)^t^5(x)^5(x)^a^a^a^t^g^t^A(m)-5(m)-A(m)-G(m)^5(m) | 4-9-5 | 6345.0 | 415 |
| 410 | h451-469-A | A(m)^T(m)-T(m)-T(m)-T(m)^t^5(x)^5(x)^a^a^a^t^g^t^A(m)-5(m)-A(m)-G(m)^5(m) | 5-9-5 | 6750.1 | 416 |
| 411 | h449-467-D | T(m)^T(m)-T(m)-T(m)-5(m)^5(x)^a^a^a^t^g^t^a^5(x)^A(m)-G(m)-5(m)-T(m)^G(m) | 5-9-5 | 6764.7 | 417 |
| 412 | h451-465-1 | TocTEG-T(Y)-T(Y)-5(Y)^c^a^a^a^t^g^t^a^c^A(Y)-G(Y)-5(S) | 3-9-3 | 5751.7 | 418 |
| 413 | h451-465-J | PalC6-T(Y)-T(Y)-5(Y)^c^a^a^a^t^g^t^a^c^A(Y)-G(Y)-5(S) | 3-9-3 | 5469.4 | 419 |
| 414 | h451-465-K | PalC6-t-c-a-T(Y)-T(Y)-5(Y)^c^a^a^a^t^g^t^a^c^A(Y)-G(Y)-5(S) | 3-9-3 | 6374.6 | 420 |
| 415 | h451-465-L | IndC6-T(Y)-T(Y)-5(Y)^c^a^a^a^t^g^t^a^c^A(Y)-G(Y)-5(S) | 3-9-3 | 5662.9 | 421 |
| 416 | h451-465-M | DHAC6-T(Y)-T(Y)-5(Y)^c^a^a^a^t^g^t^a^c^A(Y)-G(Y)-5(S) | 3-9-3 | 5543.7 | 422 |
| 417 | h451-465-N | T(Y)^T(Y)-5(Y)-5(5'-CP)-A(5'-CP)^a^a^t^g^t^a^c^A(Y)-G(Y)^5(S) | 3-9-3 | 5119.2 | 423 |
| 418 | h451-465-0 | T(Y)^T(Y)^5(Y)-5(5'-CP)-A(5'-CP)-A(5'-CP)-A(5'-CP)^t^g^t^a^c^A(Y)^G(Y)^5(S) | 3-9-3 | 5171.2 | 424 |
| 419 | h451-465-P | T(Y)^T(G)^5(GtB)^c^a^a^a^t^g^t^a^c^A(Y)-G(Y)-5(S) | 3-9-3 | 5225.9 | 425 |
| 420 | h451-467-B | T(Y)^T(m)^T(m)-T(Y)-5(m)^c^a^a^a^t^a^t^a^c^A(Y)-G(Y)-5(S) | 5-9-3 | 5861.2 | 426 |
| 421 | h451-467-C | T(Y)^T(m)^T(m)-T(m)-5(m)^c^a^a^a^t^g^t^a^5(Y)-A(m)^G(Y)^5(m) | 5-8-4 | 6004.4 | 427 |
| 422 | h451-465-Q | T(Y)-T(Y)^5(5'-CP)^c^a^a^a^t^g^t^a^c^A(5'-CP)^G(Y)-5(S) | 3-9-3 | 5028.4 | 428 |
| 423 | h451-465-R | T(Y)^T(5'-CP)^5(5'-CP)^c^a^a^a^t^e^t^a^c^A(Y)-G(Y)-5(S) | 3-9-3 | 5027.2 | 429 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 424 | h451-467-D | T(Y)^T(m)^T(m)^T(Y)^5(m)^c^a^a^a^t^g^t^a^5(Y)^A(m)^G(Y)^5(m) | 5-8-4 | 6033.1 | 430 |
| 425 | h451-467-E | T(Y)^T(m)^T(m)^T(Y)^5(m)^c^a^a^a^t^g^t^a^c^A(Y)-G(Y)-5(S) | 5-9-3 | 5893.7 | 431 |
| 426 | h451-467-F | T(Y)^T(m)^T(m)-T(Y)-5(m)^c^a^a^a^t^g^t^a^5(Y)-A(m)^G(Y)^5(m) | 5-8-4 | 5985.1 | 432 |
| 427 | h325-341-H | T(S)-T(S)-T(S)^a^c^t^a^a^g^g^a^g^c^t^5(S)-5(S)-T(S) | 3-11-3 | | 433 |

[Table 3-15]

| Ex. | Sequence name | Antisense oligonucleotide sequence (5'-3') | X-Y-Z X-Y-Z-W | Mw (actual) | SEQ ID NO: |
|---|---|---|---|---|---|
| 428 | h325-341-I | T(Y)^T(Y)-T(Y)-A(5'-CP)-5(5'-CP)^t^a^a^g^g^a^g^c^t^5(Y)-5(Y)^T(S) | 3-11-3 | | 434 |
| 429 | h325-341-J | T(Y)^T(Y)^T(Y)-A(5'-CP)-5(5'-CP)^t^a^a^g^g^a^g^5(5'-CP)-T(5'-CP)-5(Y)^5(Y)^T(S) | 3-11-3 | | 435 |
| 430 | h325-341-K | T(Y)^T(Y)-T(Y)^A(5'-CP)-5(5'-CP)^t^a^a^g^g^a^g^5(5'-CP)-T(5'-CP)^5(Y)-5(Y)^T(S) | 3-11-3 | | 436 |
| 431 | h325-341-L | T(Y)-T(Y)-T(Y)-A(5'-CP)^c^t^a^a^g^g^a^g^c^t^5(Y)-5(Y)-T(S) | 3-11-3 | | 437 |
| 432 | h325-341-M | T(Y)-T(Y)-T(Y)^a-5(5'-CP)^t^a^a^g^g^a^g^c^t^5(Y)-5(Y)-T(S) | 3-11-3 | | 438 |
| 433 | h325-341-N | T(Y)-T(Y)-T(Y)^a^c-T(5'-CP)^a^a^g^g^a^g^c^t^5(Y)-5(Y)-T(S) | 3-11-3 | | 439 |
| 434 | h325-341-O | T(Y)-T(Y)-T(Y)^a^c^t-A(5'-CP)^a^g^g^a^g^c^t^5(Y)-5(Y)-T(S) | 3-11-3 | | 440 |
| 435 | h325-341-P | T(Y)-T(Y)-T(Y)^a^c^t^a-A(5'-CP)^g^g^a^g^c^t^5(Y)-5(Y)-T(S) | 3-11-3 | | 441 |
| 436 | h325-341-Q | T(Y)-T(Y)-T(Y)^a^c^t^a^a-G(5'-CP)^g^a^g^c^t^5(Y)-5(Y)-T(S) | 3-11-3 | | 442 |
| 437 | h325-341-R | T(Y)-T(Y)-T(Y)^a^c^t^a^a^g-G(5'-CP)^a^g^c^t^5(Y)-5(Y)-T(S) | 3-11-3 | | 443 |
| 438 | h325-341-S | T(Y)-T(Y)-T(Y)^a^c^t^a^a^g^g-A(5'-CP)^g^c^t^5(Y)-5(Y)-T(S) | 3-11-3 | | 444 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 439 | h325-341-T | T(Y)-T(Y)-T(Y)^a^c^t^a^a^g^g^a-G(5'-CP)^c^t^5(Y)-5(Y)-T(S) | 3-11-3 | | 445 |
| 440 | h325-341-U | T(Y)-T(Y)-T(Y)^a^c^t^a^a^g^g^a^g-5(5'-CP)^t^5(Y)-5(Y)-T(S) | 3-11-3 | | 446 |
| 441 | h325-341-V | T(Y)-T(Y)-T(Y)^a^c^t^a^a^g^g^a^g^c-T(5'-CP)^5(Y)-5(Y)-T(S) | 3-11-3 | | 447 |

[Table 3-16]

| Ex. | Sequence name | Antisense oligonucleotide sequence (5'-3') | X-Y-Z X-Y-Z-W | Mw (actual) | SEQ ID NO: |
|---|---|---|---|---|---|
| 442 | h451-465-S | T(Y)-T(Y)-5(Y)-5(5'-CP)^a^a^a^t^g^t^a^c^A(Y)-G(Y)-5(S) | 3-9-3 | | 448 |
| 443 | h451-465-T | T(Y)-T(Y)-5(Y)^c-A(5'-CP)^a^a^t^e^t^a^c^A(Y)-G(Y)-5(S) | 3-9-3 | | 449 |
| 444 | h451-465-U | T(Y)-T(Y)-5(Y)^c^a-A(5'-CP)^a^t^g^t^a^c^A(Y)-G(Y)-5(S) | 3-9-3 | | 450 |
| 445 | h451-465-V | T(Y)-T(Y)-5(Y)^c^a^a-A(5'-CP)^t^g^t^a^c^A(Y)-G(Y)-5(S) | 3-9-3 | | 451 |
| 446 | h451-465-W | T(Y)-T(Y)-5(Y)^c^a^a^a-T(5'-CP)^g^t^a^c^A(Y)-G(Y)-5(S) | 3-9-3 | | 452 |
| 447 | h451-465-X | T(Y)-T(Y)-5(Y)^c^a^a^a^t-G(5'-CP)^t^a^c^A(Y)-G(Y)-5(S) | 3-9-3 | | 453 |
| 448 | h451-465-Y | T(Y)-T(Y)-5(Y)^c^a^a^a^t^g-T(5'-CP)^a^c^A(Y)-G(Y)-5(S) | 3-9-3 | | 454 |
| 449 | h451-465-Z | T(Y)-T(Y)-5(Y)^c^a^a^a^t^g^t-A(5'-CP)^c^A(Y)-G(Y)-5(S) | 3-9-3 | | 455 |
| 450 | h451-465-AA | T(Y)-T(Y)-5(Y)^c^a^a^a^t^g^t^a-5(5'-CP)^A(Y)-G(Y)-5(S) | 3-9-3 | | 456 |
| 451 | h451-465-AB | T(Y)-T(Y)-5(Y)^5(x)^a^a^a^t^g^t^a^5(x)^A(Y)-G(Y)-5(S) | 3-9-3 | | 457 |
| 452 | h451-465-AC | T(Y)-T(Y)-5(Y)-5(5'-CP)^a^a^a^t^g^t^a^5(x)^A(Y)-G(Y)-5(S) | 3-9-3 | | 458 |
| 453 | h451-465-AD | T(Y)-T(Y)-5(Y)^5(x)-A(5'-CP)^a^a^t^g^t^a^5(x)^A(Y)-G(Y)-5(S) | 3-9-3 | | 459 |
| 454 | h451-465-AE | T(Y)-T(Y)-5(Y)^5(x)^a-A(5'-CP)^a^t^g^t^a^5(x)^A(Y)-G(Y)-5(S) | 3-9-3 | | 460 |
| 455 | h451-465-AF | T(Y)-T(Y)-5(Y)^5(x)^a^a-A(5'-CP)^t^g^t^a^5(x)^A(Y)-G(Y)-5(S) | 3-9-3 | | 461 |
| 456 | h451-465-AG | T(Y)-T(Y)-5(Y)^5(x)^a^a^a-T(5'-CP)^g^t^a^5(x)^A(Y)-G(Y)-5(S) | 3-9-3 | | 462 |
| 457 | h451-465-AH | T(Y)-T(Y)-5(Y)^5(x)^a^a^a^t-G(5'-CP)^t^a^5(x)^A(Y)-G(Y)-5(S) | 3-9-3 | | 463 |
| 458 | h451-465-AI | T(Y)-T(Y)-5(Y)^5(x)^a^a^a^t^g-T(5'-CP)^a^5(x)^A(Y)-G(Y)-5(S) | 3-9-3 | | 464 |

[Table 3-17]

| Ex. | Sequence name | Antisense oligonucleotide sequence (5'-3') | X-Y-Z X-Y-Z-W | Mw (actual) | SEQ ID NO: |
|---|---|---|---|---|---|
| 459 | h451-465-AJ | T(Y)-T(Y)-5(Y)^5(x)^a^a^a^t^g^t-A(5'-CP)^5(x)^A(Y)-G(Y)-5(S) | 3-9-3 | | 465 |
| 460 | h451-465-AK | T(Y)-T(Y)-5(Y)^5(x)^a^a^a^t^g^t^a-5(5'-CP)^A(Y)-G(Y)-5(S) | 3-9-3 | | 466 |
| 461 | h451-465-AL | T(Y)^T(Y)^5(Y)-5(5'-CP)-A(5'-CP)^a^a^t^g^t^A(5'-CP)-5(5'-CP)-A(Y)^G(Y)^5(S) | 3-9-3 | | 467 |
| 462 | h451-465-AM | T(Y)^T(Y)-5(Y)^5(5'-CP)-A(5'-CP)^a^a^t^g^t^A(5'-CP)-5(5'-CP)^A(Y)-G(Y)^5(S) | 3-9-3 | | 468 |
| 463 | h451-465-AN | T(Y)-T(Y)-5(Y)^a^c^t^g^a^c^a^t^a^A(Y)-G(Y)-5(S) | 3-9-3 | 5053.3 | 469 |
| 464 | h451-465-AO | T(Y)-T(Y)-5(Y)^a^t^t^g^a^c^a^c^a^A(Y)-G(Y)-5(S) | 3-9-3 | 5053.1 | 470 |

The single-stranded antisense oligonucleotides in Table 4-1 to Table 4-5 are single-stranded antisense oligonucleotides for human RPS25 mRNA precursor (SEQ ID NO: 2).

[Table 4-1]

| Ex. | Sequence name | Antisense oligonucleotide sequence (5'-3') | X-Y-Z X-Y-Z-W | Mw (actual) | SEQ ID NO: |
|---|---|---|---|---|---|
| 186 | hp1-15-A | 5(Y)^G(Y)^G(Y)^a^c^a^a^a^a^a^g^g^A(Y)^A(Y)^g | 3-9-2-1 | 5181.4 | 192 |
| 187 | hp72-86-A | T(Y)^T(Y)^5(Y)^a^c^a^c^c^c^c^t^g^A(Y)^A(Y)^g | 3-9-2-1 | 5009.8 | 193 |
| 188 | hp73-87-A | 5(Y)^T(Y)^T(Y)^c^a^c^a^c^c^c^c^t^G(Y)^A(Y)^a | 3-9-2-1 | 4969.9 | 194 |
| 189 | hp74-88-A | A(Y)^5(Y)^T(Y)^t^c^a^c^a^c^c^c^c^T(Y)^G(Y)^a | 3-9-2-1 | 4970.7 | 195 |
| 190 | hp75-89-A | G(Y)^A(Y)^5(Y)^t^t^c^a^c^a^c^c^c^5(Y)^T(Y)^Q | 3-9-2-1 | 5000.6 | 196 |
| 191 | hp76-90-A | 5(Y)^G(Y)^A(Y)^c^t^t^c^a^c^a^c^c^5(Y)^5(Y)^t | 3-9-2-1 | 4974.1 | 197 |
| 192 | hp231-245-A | T(Y)^5(Y)^5(Y)^c^c^t^a^a^t^a^c^t^G(Y)^5(Y)^g | 3-9-2-1 | 5030.4 | 198 |
| 193 | hp232-246-A | G(Y)^T(Y)^5(Y)^c^c^c^t^a^a^t^a^c^T(Y)^G(Y)^c | 3-9-2-1 | 5001.7 | 199 |
| 194 | hp233-247-A | A(Y)^G(Y)^T(Y)^c^c^c^c^t^a^a^t^a^5(Y)^T(Y)^g | 3-9-2-1 | 5025.4 | 200 |
| 195 | hp261-275-A | A(Y)^T(Y)^G(Y)^c^a^a^c^c^a^e^e^t^5(Y)^5(Y)^t | 3-9-2-1 | 5065.3 | 201 |
| 196 | hp262-276-A | A(Y)^A(Y)^T(Y)^g^c^a^a^c^c^a^g^g^T(Y)^5(Y)^c | 3-9-2-1 | 5058.8 | 202 |
| 197 | hp278-292-A | G(Y)^T(Y)^A(Y)^g^g^a^g^g^g^c^a^g^5(Y)^G(Y)^g | 3-9-2-1 | 5236.8 | 203 |
| 198 | hp279-293-A | T(Y)^G(Y)^T(Y)^a^g^g^a^g^g^g^c^a^G(Y)^5(Y)^g | 3-9-2-1 | 5210.7 | 204 |
| 199 | hp280-294-A | 5(Y)^T(Y)^G(Y)^t^a^g^g^a^g^g^g^c^A(Y)^G(Y)^c | 3-9-2-1 | 5171.9 | 205 |
| 200 | hp390-404-A | G(Y)^G(Y)^T(Y)^c^t^t^a^t^t^t^c^t^A(Y)^5(Y)^c | 3-9-2-1 | 5022.8 | 206 |
| 201 | hp392-406-A | G(Y)^A(Y)^G(Y)^g^t^c^t^t^a^t^t^t^5(Y)^T(Y)^a | 3-9-2-1 | 5086.9 | 207 |
| 202 | hp417-431-A | A(Y)^G(Y)^A(Y)^g^t^t^a^c^c^c^c^t^A(Y)^G(Y)^t | 3-9-2-1 | 5050.9 | 208 |
| 203 | hp418-432-A | G(Y)^A(Y)^G(Y)^a^g^t^t^a^c^c^c^c^T(Y)^A(Y)^g | 3-9-2-1 | 5077.3 | 209 |
| 204 | hp419-433-A | A(Y)^G(Y)^A(Y)^g^a^g^t^t^a^c^c^c^5(Y)^T(Y)^a | 3-9-2-1 | 5074.5 | 210 |

(continued)

| Ex. | Sequence name | Antisense oligonucleotide sequence (5'-3') | X-Y-Z<br>X-Y-Z-W | Mw (actual) | SEQ ID NO: |
|---|---|---|---|---|---|
| 205 | hp420-434-A | G(Y)^A(Y)^G(Y)^a^g^a^g^t^t^a^c^c^5(Y)^5(Y)^t | 3-9-2-1 | 5104.1 | 211 |
| 206 | hp421-435-A | 5(Y)^G(Y)^A(Y)^g^a^g^a^g^t^t^a^c^5(Y)^5(Y)^c | 3-9-2-1 | 5102.1 | 212 |
| 207 | hp422-436-A | A(Y)^5(Y)^G(Y)^a^g^a^g^a^g^t^t^a^5(Y)^5(Y)^c | 3-9-2-1 | 5127.3 | 213 |
| 208 | hp423-437-A | T(Y)^A(Y)^5(Y)^g^a^g^a^g^a^g^t^t^A(Y)^5(Y)^c | 3-9-2-1 | 5128.2 | 214 |
| 209 | hp445-459-A | A(Y)^A(Y)^G(Y)^t^t^a^c^c^t^a^t^t^A(Y)^5(Y)^t | 3-9-2-1 | 5039.2 | 215 |
| 210 | hp446-460-A | 5(Y)^A(Y)^A(Y)^g^t^t^a^c^c^t^a^t^T(Y)^A(Y)^c | 3-9-2-1 | 5024.8 | 216 |

[Table 4-2]

| Ex. | Sequence name | Antisense oligonucleotide sequence (5'-3') | X-Y-Z<br>X-Y-Z-W | Mw (actual) | SEQ ID NO: |
|---|---|---|---|---|---|
| 211 | hp447-461-A | A(Y)^5(Y)^A(Y)^a^g^t^t^a^c^c^t^a^T(Y)^T(Y)^a | 3-9-2-1 | 5048.0 | 217 |
| 212 | hp448-462-A | T(Y)^A(Y)^5(Y)^a^a^g^t^t^a^c^c^t^A(Y)^T(Y)^t | 3-9-2-1 | 5039.2 | 218 |
| 213 | hp458-472-A | 5(Y)^5(Y)^A(Y)^c^t^t^a^c^t^a^t^a^5(Y)^A(Y)^3 | 3-9-2-1 | 5021.5 | 219 |
| 214 | hp460-474-A | A(Y)^A(Y)^5 (Y)^c^a^c^t^t^a^c^t^a^T(Y)^A(Y)^c | 3-9-2-1 | 4993.3 | 220 |
| 215 | hp461-475-A | A(Y)^A(Y)^A(Y)^c^c^a^c^t^t^a^c^t^A(Y)^T(Y)^S | 3-9-2-1 | 5003.4 | 221 |
| 216 | hp510-524-A | G(Y)^A(Y)^5(Y)^g^g^g^a^a^g^a^t^a^A(Y)^A(Y)^c | 3-9-2-1 | 5173.3 | 222 |
| 217 | hp561-575-A | T(Y)^5(Y)^G(Y)^c^a^c^a^a^c^a^g^a^5(Y)^5(Y)^c | 3-9-2-1 | 5032.2 | 223 |
| 218 | hp562-576-A | T(Y)^T(Y)^5(Y)^g^c^a^c^a^a^c^a^g^A(Y)^5(Y)^c | 3-9-2-1 | 5033.4 | 224 |
| 219 | hp589-603-A | T(Y)^A(Y)^A(Y)^c^a^c^a^g^c^a^g^g^5(Y)^A(Y)^c | 3-9-2-1 | 5068.4 | 225 |
| 220 | hp605-619-A | 5(Y)^T(Y)^A(Y)^g^a^t^c^a^g^t^t^a^A(Y)^A(Y)^a | 3-9-2-1 | 5096.7 | 226 |
| 221 | hp606-620-A | A(Y)^5(Y)^T(Y)^a^g^a^t^c^a^g^t^t^A(Y)^A(Y)^a | 3-9-2-1 | 5096.8 | 227 |
| 222 | hp626-640-A | T(Y)^5(Y)^A(Y)^a^a^c^a^g^g^g^g^c^5(Y)^G(Y)^a | 3-9-2-1 | 5139.3 | 228 |
| 223 | hp627-641-A | T(Y)^T(Y)^5(Y)^a^a^a^c^a"g^g^g^g^5(Y)^5(Y)^g | 3-9-2-1 | 5143.2 | 229 |
| 224 | hp628-642-A | 5(Y)^T(Y)^T(Y)^c^a^a^a^c^a^g^g^g^G(Y)^5(Y)^c | 3-9-2-1 | 5089.9 | 230 |
| 225 | hp629-643-A | 5(Y)^5(Y)^T(Y)^t^c^a^a^a^c^a^g^g^G(Y)^G(Y)^c | 3-9-2-1 | 5089.5 | 231 |
| 226 | hp632-646-A | T(Y)^G(Y)^G(Y)^c^c^t^t^c^a^a^a^c^A(Y)^G(Y)^g | 3-9-2-1 | 5076.9 | 232 |
| 227 | hp633-647-A | T(Y)^T(Y)^G(Y)^g^c^c^t^t^c^a^a^a^5(Y)^A(Y)^g | 3-9-2-1 | 5065.2 | 233 |
| 228 | hp634-648-A | T(Y)^T(Y)^T(Y)^g^g^c^c^t^t^c^a^a^A(Y)^5(Y)^a | 3-9-2-1 | 5040.4 | 234 |
| 229 | hp654-668-A | A(Y)^A(Y)^A(Y)^a^a^a^a^a^c^a^c^c^G(Y)^A(Y)^c | 3-9-2-1 | 5055.8 | 235 |
| 230 | hp681-695-A | A(Y)^A(Y)^T(Y)^t^a^c^a^c^a^t^t^a^5(Y)^T(Y)^a | 3-9-2-1 | 5033.0 | 236 |
| 231 | hp696-710-A | 5(Y)^5(Y)^G(Y)^t^t^a^t^c^a^a^g^g^A(Y)^T(Y)^a | 3-9-2-1 | 5103.2 | 237 |
| 232 | hp697-711-A | A(Y)^5(Y)^5(Y)^g^t^t^a^t^c^a^a^g^G(Y)^A(Y)^t | 3-9-2-1 | 5103.6 | 238 |
| 233 | hp761-775-A | G(Y)^T(Y)^T(Y)^a^g^t^a^t^t^t^c^t^G(Y)^G(Y)^c | 3-9-2-1 | 5087.8 | 239 |
| 234 | hp762-776-A | A(Y)^G(Y)^T(Y)^t^a^g^t^a^t^t^t^c^T(Y)^G(Y)^g | 3-9-2-1 | 5112.2 | 240 |
| 235 | hp764-778-A | 5(Y)^A(Y)^A(Y)^g^t^t^a^g^t^a^t^t^T(Y)^5(Y)^t | 3-9-2-1 | 5084.6 | 241 |

[Table 4-3]

| Ex. | Sequence name | Antisense oligonucleotide sequence (5'-3') | X-Y-Z X-Y-Z-W | Mw (actual) | SEQ ID NO: |
|---|---|---|---|---|---|
| 236 | hp1034-1048-A | A(Y)^T(Y)^G(Y)^c^t^t^a^a^a^c^a^t^g^G(Y)^T(Y)^c | 3-9-2-1 | 5066.8 | 242 |
| 237 | hp1035-1049-A | G(Y)^A(Y)^T(Y)^g^c^t^t^a^a^c^a^t^G(Y)^G(Y)^t | 3-9-2-1 | 5105.8 | 243 |
| 238 | hp1103-1117-A | T(Y)^T(Y)^A(Y)^c^t^a^a^c^a^g^c^c^A(Y)^A(Y)^t | 3-9-2-1 | 5018.7 | 244 |
| 239 | hp1104-1118-A | A(Y)^T(Y)^T(Y)^a^c^t^a^a^c^a^g^c^5(Y)^A(Y)^a | 3-9-2-1 | 5041.9 | 245 |
| 240 | hp1105-1119-A | 5(Y)^A(Y)^T(Y)^t^a^c^t^a^a^c^a^g^5(Y)^5(Y)^a | 3-9-2-1 | 5046.6 | 246 |
| 241 | hp1106-1120-A | A(Y)^5(Y)^A(Y)^t^t^a^c^t^a^a^c^a^G(Y)^5(Y)^c | 3-9-2-1 | 5032.3 | 247 |
| 242 | hp1107-1121-A | T(Y)^A(Y)^5(Y)^a^t^t^a^c^t^a^a^c^A(Y)^G(Y)^c | 3-9-2-1 | 5032.6 | 248 |
| 243 | hp1108-1122-A | T(Y)^T(Y)^A(Y)^c^a^t^t^a^c^t^a^a^5(Y)^A(Y)^g | 3-9-2-1 | 5048.1 | 249 |
| 244 | hp1110-1124-A | A(Y)^A(Y)^T(Y)^t^a^c^a^t^t^a^c^t^A(Y)^A(Y)^c | 3-9-2-1 | 5018.2 | 250 |
| 245 | hp1128-1142-A | T(Y)^5(Y)^A(Y)^g^g^a^g^t^a^a^g^a^5(Y)^G(Y)^t | 3-9-2-1 | 5167.9 | 251 |
| 246 | hp1129-1143-A | A(Y)^T(Y)^5(Y)^a^g^g^a^g^t^a^a^g^A(Y)^5(Y)^g | 3-9-2-1 | 5177.4 | 252 |
| 247 | hp1196-1210-A | G(Y)^5(Y)^T(Y)^t^c^a^c^t^a^a^a^c^T(Y)^G(Y)^c | 3-9-2-1 | 5026 | 253 |
| 248 | hp1197-1211-A | G(Y)^G(Y)^5(Y)^t^t^c^a^c^t^a^a^a^5(Y)^T(Y)^g | 3-9-2-1 | 5079.2 | 254 |
| 249 | hp1217-1231-A | 5(Y)^G(Y)^A(Y)^a^a^c^a^t^a^a^a^a^G(Y)^A(Y)^t | 3-9-2-1 | 5115.4 | 255 |
| 250 | hp1218-1232-A | T(Y)^5(Y)^G(Y)^a^a^a^c^a^t^a^a^a^A(Y)^G(Y)^a | 3-9-2-1 | 5115.5 | 256 |
| 251 | hp1219-1233-A | 5(Y)^T(Y)^5(Y)^g^a^a^a^c^a^t^a^a^A(Y)^A(Y)^g | 3-9-2-1 | 5105.8 | 257 |
| 252 | hp1398-1412-A | G(Y)^T(Y)^G(Y)^g^a^a^t^t^a^t^g^g^T(Y)^A(Y)^a | 3-9-2-1 | 5171.0 | 258 |
| 253 | hp1399-1413-A | T(Y)^G(Y)^T(Y)^g^g^a^a^t^t^a^t^g^G(Y)^T(Y)^a | 3-9-2-1 | 5161.6 | 259 |
| 254 | hp1402-1416-A | T(Y)^A(Y)^T(Y)^t^g^t^g^g^a^a^t^t^A(Y)^T(Y)^g | 3-9-2-1 | 5135.5 | 260 |
| 255 | hp1408-1422-A | 5(Y)^5(Y)^T(Y)^t^a^t^t^a^t^t^Q^t^G(Y)^G(Y)^a | 3-9-2-1 | 5100.0 | 261 |
| 256 | hp1409-1423-A | G(Y)^5(Y)^5(Y)^t^t^a^t^t^a^t^t^p^T(Y)^G(Y)^g | 3-9-2-1 | 5116.8 | 262 |
| 257 | hp1410-1424-A | A(Y)^G(Y)^5(Y)^c^t^t^a^t^t^a^t^t^G(Y)^T(Y)^g | 3-9-2-1 | 5087.0 | 263 |
| 258 | hp1411-1425-A | G(Y)^A(Y)^G(Y)^c^c^t^t^a^t^t^a^t^T(Y)^G(Y)^t | 3-9-2-1 | 5072.7 | 264 |
| 259 | hp1412-1426-A | T(Y)^G(Y)^A(Y)^g^c^c^t^t^a^t^t^a^T(Y)^T(Y)^g | 3-9-2-1 | 5072.5 | 265 |
| 260 | hp1478-1492-A | 5(Y)^G(Y)^T(Y)^t^g^a^t^t^c^a^c^c^5(Y)^G(Y)^c | 3-9-2-1 | 5031.4 | 266 |

[Table 4-4]

| Ex. | Sequence name | Antisense oligonucleotide sequence (5'-3') | X-Y-Z X-Y-Z-W | Mw (actual) | SEQ ID NO: |
|---|---|---|---|---|---|
| 261 | hp1480-1494-A | T(Y)^5(Y)^5(Y)^g^t^t^g^a^t^t^c^a^5(Y)^5(Y)^c | 3-9-2-1 | 5034.8 | 267 |
| 262 | hp1715-1729-A | G(Y)^5(Y)^T(Y)^c^c^a^t^t^a^t^c^t^T(Y)^5(Y)^c | 3-9-2-1 | 4981.7 | 268 |
| 263 | hp1749-1763-A | 5(Y)^G(Y)^A(Y)^t^c^a^t^c^t^a^t^c^5(Y)^T(Y)^t | 3-9-2-1 | 5005.2 | 269 |
| 264 | hp1750-1764-A | A(Y)^5(Y)^G(Y)^a^t^c^a^t^c^t^a^t^5(Y)^5(Y)^t | 3-9-2-1 | 5028.7 | 270 |
| 265 | hp1751-1765-A | A(Y)^A(Y)^5(Y)^g^a^t^c^a^t^c^t^a^T(Y)^5(Y)^c | 3-9-2-1 | 5023.7 | 271 |
| 266 | hp1763-1777-A | A(Y)^T(Y)^5(Y)^c^t^a^g^t^t^t^t^t^A(Y)^A(Y)^c | 3-9-2-1 | 5030.4 | 272 |
| 267 | hp1793-1807-A | A(Y)^T(Y)^T(Y)^g^c^t^t^a^a^t^c^t^G(Y)^A(Y)^c | 3-9-2-1 | 5041.6 | 273 |

(continued)

| Ex. | Sequence name | Antisense oligonucleotide sequence (5'-3') | X-Y-Z X-Y-Z-W | Mw (actual) | SEQ ID NO: |
|---|---|---|---|---|---|
| 268 | hp1885-1899-A | A(Y)^T(Y)^G(Y)^g^t^c^t^t^a^a^a^a^5(Y)^T(Y)^c | 3-9-2-1 | 5064.2 | 274 |
| 269 | hp1887-1901-A | G(Y)^A(Y)^A(Y)^t^g^g^t^c^t^t^a^a^A(Y)^A(Y)^c | 3-9-2-1 | 5099.4 | 275 |
| 270 | hp2047-2061-A | G(Y)^T(Y)^T(Y)^t^g^t^t^t^t^g^g^c^5(Y)^G(Y)^g | 3-9-2-1 | 5133.5 | 276 |
| 271 | hp2048-2062-A | G(Y)-G(Y)-T(Y)-t^t^g^t^t^t^t^g^g^5(Y)^5(Y)^g | 3-9-2-1 | 5149.2 | 277 |
| 272 | hp2049-2063-A | A(Y)^G(Y)^G(Y)^t^t^t^g^t^t^t^t^g^G(Y)^5(Y)^c | 3-9-2-1 | 5118.5 | 278 |
| 273 | hp2121-2135-A | G(Y)^A(Y)^A(Y)^t^t^g^g^t^g^g^t^t^G(Y)^5(Y)^a | 3-9-2-1 | 5176.0 | 279 |
| 274 | hp2122-2136-A | G(Y)^G(Y)^A(Y)^g^t^t^g^g^t^g^g^t^T(Y)^G(Y)^c | 3-9-2-1 | 5179.1 | 280 |
| 275 | hp2123-2137-A | A(Y)^G(Y)^G(Y)^a^a^t^t^g^g^t^g^g^T(Y)^T(Y)^g | 3-9-2-1 | 5202.6 | 281 |
| 276 | hp2124-213 8-A | 5(Y)^A(Y)^G(Y)^g^a^a^t^t^g^g^t^g^G(Y)^T(Y)^t | 3-9-2-1 | 5176.7 | 282 |
| 277 | hp2260-2274-A | G(Y)^G(Y)^T(Y)^a^a^g^g^a^g^t^t^g^5(Y)^A(Y)^c | 3-9-2-1 | 5169.2 | 283 |
| 278 | hp2261-2275-A | A(Y)^G(Y)^G(Y)^t^a^a^g^g^a^g^t^t^G(Y)^5(Y)^a | 3-9-2-1 | 5194.5 | 284 |
| 279 | hp2262-2276-A | G(Y)^A(Y)^G(Y)^g^t^a^a^g^g^a^g^t^T(Y)^G(Y)^c | 3-9-2-1 | 5196.6 | 285 |
| 280 | hp2268-2282-A | T(Y)^A(Y)^G(Y)^c^t^t^g^a^g^g^t^a^A(Y)^G(Y)^g | 3-9-2-1 | 5171.7 | 286 |
| 281 | hp2269-2283-A | A(Y)^T(Y)^A(Y)^g^c^t^t^g^a^g^g^t^A(Y)^A(Y)^g | 3-9-2-1 | 5150.0 | 287 |
| 282 | hp2271-2285-A | A(Y)^G(Y)^A(Y)^t^a^g^c^t^t^g^a^g^G(Y)^T(Y)^a | 3-9-2-1 | 5156.1 | 288 |
| 283 | hp2277-2291-A | A(Y)^5(Y)^G(Y)^g^g^c^a^g^a^t^a^g^5(Y)^T(Y)^t | 3-9-2-1 | 5144.9 | 289 |
| 284 | hp2339-2353-A | G(Y)^T(Y)^A(Y)^a^g^g^t^t^t^t^g^G(Y)^5(Y)^t | 3-9-2-1 | 5142.3 | 290 |
| 285 | hp2341-2355-A | T(Y)^A(Y)^G(Y)^t^g^g^t^t^t^t^t^t^T(Y)^G(Y)^g | 3-9-2-1 | 5151.6 | 291 |

[Table 4-5]

| Ex. | Sequence name | Antisense oligonucleotide sequence (5'-3') | X-Y-Z X-Y-Z-W | Mw (actual) | SEQ ID NO: |
|---|---|---|---|---|---|
| 286 | hp2342-2356-A | 5(Y)^T(Y)^A(Y)^g^t^a^a^g^g^t^t^t^T(Y)^T(Y)^g | 3-9-2-1 | 5126.6 | 292 |
| 287 | hp2386-2400-A | A(Y)^G(Y)^A(Y)^g^a^a^t^a^g^c^a^c^G(Y)^A(Y)^t | 3-9-2-1 | 5133.4 | 293 |
| 288 | hp2406-2420-A | G(Y)^T(Y)^T(Y)^t^g^a^t^a^a^g^t^c^5(Y)^T(Y)^a | 3-9-2-1 | 5095.3 | 294 |
| 289 | hp2538-2552-A | T(Y)^T(Y)^T(Y)^g^t^a^t^c^t^a^c^c^T(Y)^5(Y)^c | 3-9-2-1 | 4982.5 | 295 |
| 290 | hp2540-2554-A | G(Y)^5(Y)^T(Y)^t^t^g^t^a^t^c^t^a^5(Y)^5(Y)^t | 3-9-2-1 | 5050.8 | 296 |
| 291 | hp2541-2555-A | A(Y)^G(Y)^5(Y)^t^t^t^g^t^a^t^c^t^A(Y)^5(Y)^c | 3-9-2-1 | 5046.0 | 297 |
| 292 | hp2585-2599-A | 5(Y)^T(Y)^G(Y)^g^t^t^g^g^a^c^c^t^G(Y)^T(Y)^a | 3-9-2-1 | 5111.9 | 298 |
| 293 | hp2586-2600-A | G(Y)^5(Y)^T(Y)^g^g^t^t^g^g^a^c^c^T(Y)^G(Y)^t | 3-9-2-1 | 5128.0 | 299 |
| 294 | hp2587-2601-A | A(Y)^G(Y)^5(Y)^t^g^g^t^t^g^g^a^c^5(Y)^T(Y)^g | 3-9-2-1 | 5151.6 | 300 |
| 295 | hp2583-2597-A | G(Y)^G(Y)^T(Y)^t^g^g^a^c^c^t^g^t^A(Y)^A(Y)^a | 3-9-2-1 | 5131.8 | 301 |
| 296 | hp2584-2598-A | T(Y)^G(Y)^G(Y)^t^t^g^g^a^c^c^t^g^T(Y)^A(Y)^a | 3-9-2-1 | 5122.4 | 302 |

Herein, symbols or expressions described below may be used to represent corresponding structures.

—

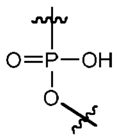

^

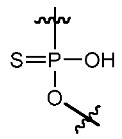

=

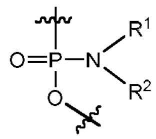

x

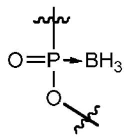

a

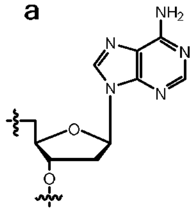

A

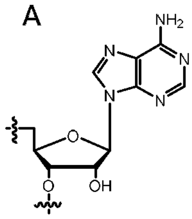

A(M)

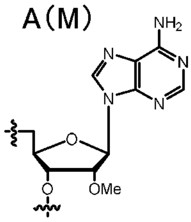

A(m)

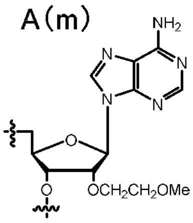

A(L)

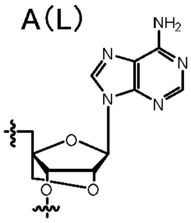

A(Y)

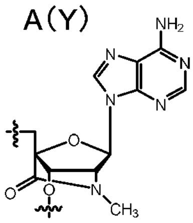

A(Gx)

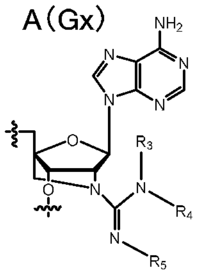

A(S)

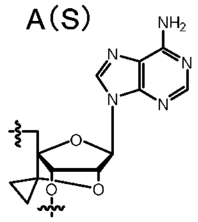

A(5'–CP)

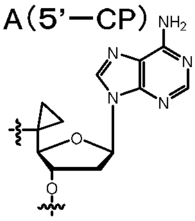

A(Mx)

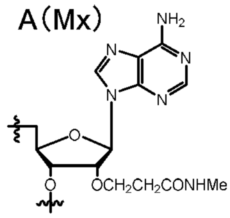

c

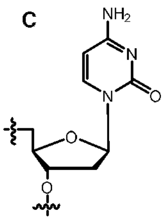

5(x)

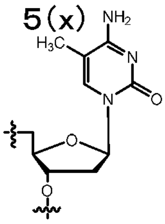

C

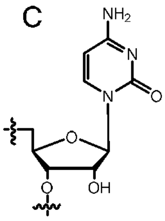

C(M)

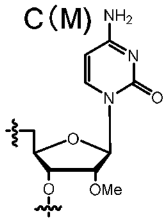

5(m)

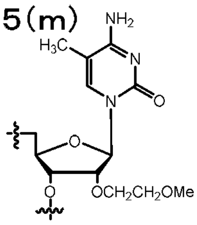

5(L)

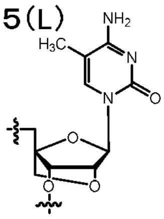

5(Y)

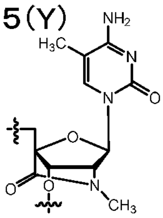

5(Gx)

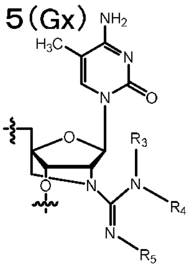

5(S)

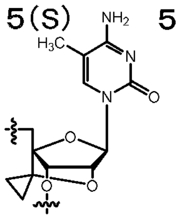

5(5'–CP)

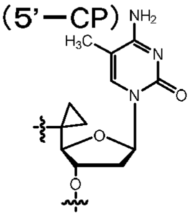

C(Mx)

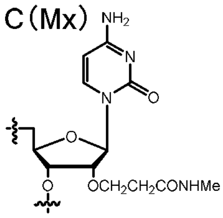

g

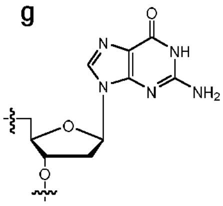

G

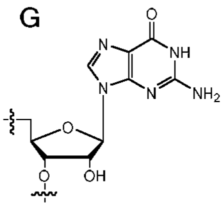

G(M)

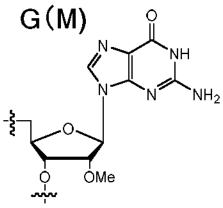

G(m)

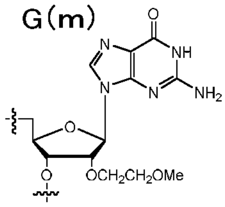

G(L)

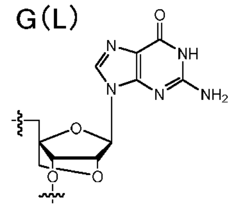

G(Y)

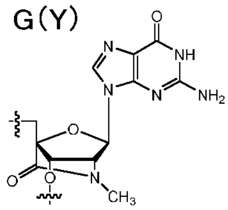

G(Gx)

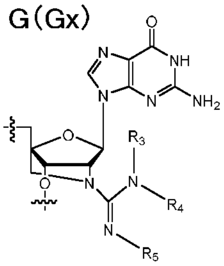

G(S)

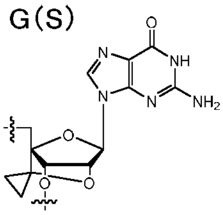

G(5'−CP)

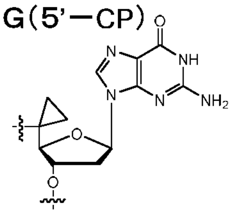

G(Mx)

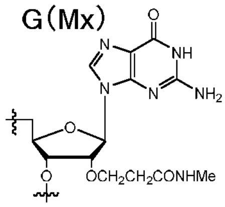

t

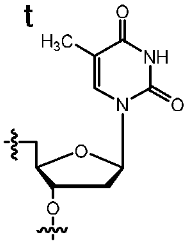

U

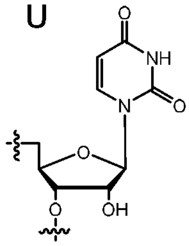

U(M)

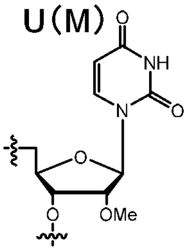

T(m)

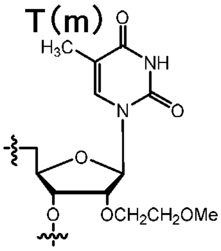

T(L)

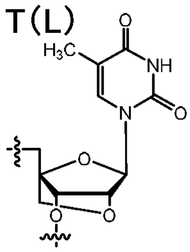

T(Y)

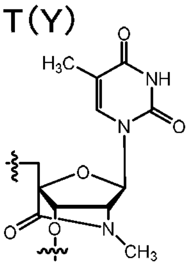

T(Gx)

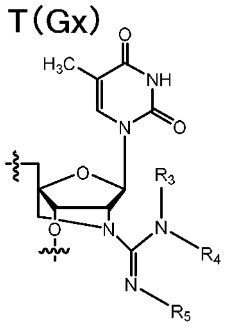

T(S)

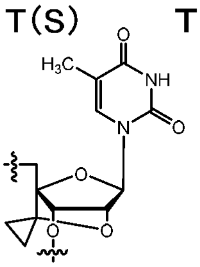

T(5'−CP)

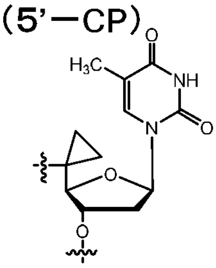

U(Mx)

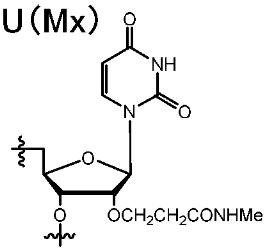

In the above-described structural formulae, $R^1$ and $R^2$ independently represents a hydrogen atom, or a linear or branched alkyl group having 1 to 3 carbon atoms. Each of $R^1$ and $R^2$ involving in a bond represented by "=" above represents a methyl group. $R^3$, $R^4$, and $R^5$ independently represents a hydrogen atom, a linear or branched alkyl group having 1 to 7 carbon atoms, or a cycloalkyl group having 3 to 7 carbon atoms. GuNA represented by "Gx" above is represented by "Gm" when $R^3$ and $R^5$ are both hydrogen atoms and $R^4$ is a methyl group; it is represented by "Gdm" when $R^3$ is a hydrogen atom and $R^4$ and $R^5$ are both methyl groups; and it is represented by "GtB" when $R^3$ and $R^5$ are both hydrogen atoms and $R^4$ is a tert-butyl group.

«Evaluation of RPS25 Gene Expression»

Evaluation of RPS25 gene expression was carried out in two ways depending on the single-stranded antisense oligonucleotide thus produced; expression evaluation with the use of human fetal kidney cells and expression evaluation with the use of mouse primary culture neurons. Evaluation of RPS25 gene expression can also be carried out with the use of neurons derived from human iPS cells. Evaluation of gene expression in the present Examples means evaluation of the amount of mRNA by measurement of the amount of complementary DNA (cDNA) obtained by reverse transcription reaction. In the following, the specific procedure of each expression evaluation will be described.

<Expression Evaluation with Use of Human Fetal Kidney Cells>

Human fetal kidney cells HEK293T (ATCC (registered trademark) CRL-3216 (trademark)) were cultured in a culture medium under the conditions of 37°C and 5% $CO_2$. The culture medium used for HEK293T cells had the composition as described below.

Composition of Culture Medium for HEK293T Cells

Dulbecco modified Eagle medium (DMEM): manufactured by SIGMA, Cat# D6429

10% fetal bovine serum (FBS): manufactured by biowest, Cat# S1820
100-Fold diluted penicillin-streptomycin mixed solution: manufactured by Nacalai Tesque, Cat# 09367-34 (penicillin 10000 units/ml, streptomycin 10000 μg/ml, a stabilizer contained)

Firstly, the day before measuring the amount of expression of human RPS25 gene, HEK293T cells were plated in a 96-well plate (12000 cells/well) and cultured overnight under the conditions of 37°C and 5% COz. Subsequently, the cells were transfected by lipofection with the single-stranded antisense oligonucleotide diluted with phosphate-buffered physiological saline (PBS) (in a final concentration of 0.5 nM, 5 nM, 15 nM, or 50 nM). As the negative control group, cells transfected with PBS that was free of single-stranded antisense oligonucleotide were used. The transfected cells were cultured in a growth medium under the conditions of 37°C and 5% $CO_2$ for 48 hours. Subsequently, the growth medium was removed, and with the use of Taqman Fast Cells-to-CT Kit (manufactured by Thermo Fisher Scientific, Cat# 4399003), reverse transcription reaction was carried out for the extracted total RNA. With the use of the resulting complementary DNA (cDNA) obtained from the reverse transcription reaction, Taqman gene expression assays (manufactured by Applied Biosystems), and a gene-specific probe designed in advance (see below), real-time PCR was carried out (40 cycles of 95°C for 3 seconds and 60°C for 30 seconds).

List of Gene-Specific Probes Used in Evaluation of Human RPS25 Gene Expression

Human RPS25: Hs01568661_g1
Human GAPDH: 4326317E (internal control)

The expression rate of human RPS25 mRNA in the single-stranded antisense oligonucleotides for human RPS25 mRNA, as determined by the above-described method, is shown in Table 5-1 to Table 5-7, Table 6-1, and Table 6-2. At this time, the expression rate of human RPS25 mRNA for the negative control group was defined as 1.00. One with an expression rate of 0.80 or less was judged to be a single-stranded antisense oligonucleotide capable of reducing expression of human RPS25 mRNA. "-" in the tables means that no measurement was performed. Generally, it is expected that when mRNA expression is reduced, subsequent protein translation and the like are also reduced. Hence, it can be judged that one with an expression rate of 0.80 or less is a single-stranded antisense oligonucleotide capable of modulating the function of human RPS25 gene.

[Table 5-1]

| SEQ ID NO: | Sequence name | hRPS25 expression rate | SEQ ID NO: | Sequence name | hRPS25 expression rate |
|---|---|---|---|---|---|
| | | @ 50 nM | | | @ 50 nM |
| 7 | h8-22-A | 0.36 | 29 | h126-140-A | 0.12 |
| 8 | h9-23-A | 0.63 | 30 | h140-154-A | 0.75 |
| 9 | h10-24-A | 0.45 | 31 | h160-174-A | 0.45 |
| 10 | h27-41-A | 0.62 | 32 | h161-175-A | 0.66 |
| 11 | h28-42-A | 0.25 | 33 | h180-194-A | 0.44 |
| 12 | h29-43-A | 0.43 | 34 | h181-195-A | 0.63 |
| 13 | h30-44-A | 0.84 | 35 | h182-196-A | 0.70 |
| 14 | h32-46-A | 0.86 | 36 | h183-197-A | 0.27 |
| 15 | h33-47-A | 0.82 | 37 | h184-198-A | 0.41 |
| 16 | h34-48-A | 0.72 | 38 | h187-201-A | 0.15 |
| 17 | h35-49-A | 0.50 | 39 | h188-202-A | 0.69 |
| 18 | h36-50-A | 0.20 | 40 | h189-203-A | 1.01 |
| 19 | h37-51-A | 0.38 | 41 | h191-205-A | 0.68 |
| 20 | h38-52-A | 0.75 | 42 | h193-207-A | 0.87 |
| 21 | h72-86-A | 0.85 | 43 | h196-210-A | 1.21 |

(continued)

| SEQ ID NO: | Sequence name | hRPS25 expression rate | SEQ ID NO: | Sequence name | hRPS25 expression rate |
|---|---|---|---|---|---|
| | | @ 50 nM | | | @ 50 nM |
| 22 | h79-93-A | 0.67 | 44 | h197-211-A | 1.06 |
| 23 | h101-115-A | 0.28 | 45 | h208-222-A | 0.53 |
| 24 | h102-116-A | 0.02 | 46 | h209-223-A | 0.25 |
| 25 | h103-117-A | 0.08 | 47 | h210-224-A | 0.27 |
| 26 | h122-136-A | 1.36 | 48 | h213-227-A | 0.10 |
| 27 | h124-138-A | 0.24 | 49 | h214-228-A | 0.16 |
| 28 | h125-139-A | 0.07 | 50 | h216-230-A | 0.41 |

[Table 5-2]

| SEQ ID NO: | Sequence name | hRPS25 expression rate | SEQ ID NO: | Sequence name | hRPS25 expression rate |
|---|---|---|---|---|---|
| | | @ 50 nM | | | @ 50 nM |
| 51 | h217-231-A | 0.59 | 73 | h344-358-A | 0.26 |
| 52 | h219-233-A | 0.42 | 74 | h365-379-A | 0.39 |
| 53 | h220-234-A | 0.18 | 75 | h375-389-A | 1.01 |
| 54 | h242-256-A | 0.72 | 76 | h390-404-A | 1.05 |
| 55 | h243-257-A | 0.67 | 77 | h393-407-A | 1.11 |
| 56 | h253-267-A | 0.91 | 78 | h394-408-A | 1.59 |
| 57 | h254-268-A | 0.81 | 79 | h430-444-A | 0.11 |
| 58 | h259-273-A | 0.12 | 80 | h431-445-A | 0.10 |
| 59 | h260-274-A | 0.18 | 81 | h432-446-A | 0.33 |
| 60 | h261-275-A | 0.36 | 82 | h433-447-A | 0.74 |
| 61 | h285-299-A | 0.38 | 83 | h434-448-A | 0.59 |
| 62 | h286-300-A | 0.63 | 84 | h435-449-A | 0.10 |
| 63 | h295-309-A | 0.13 | 85 | h436-450-A | 0.45 |
| 64 | h296-310-A | 0.05 | 86 | h438-452-A | 0.15 |
| 65 | h297-311-A | 0.29 | 87 | h439-453-A | 0.17 |
| 66 | h325-339-A | 0.12 | 88 | h440-454-A | 0.10 |
| 67 | h326-340-A | 0.11 | 89 | h441-455-A | 0.24 |
| 68 | h327-341-A | 0.17 | 90 | h442-456-A | 0.39 |
| 69 | h340-354-A | 0.52 | 91 | h444-458-A | 0.06 |
| 70 | h341-355-A | 0.44 | 92 | h446-460-A | 0.37 |
| 71 | h342-356-A | 0.75 | 93 | h447-461-A | 0.18 |
| 72 | h343-357-A | 0.66 | 94 | h451-465-A | 0.08 |

[Table 5-3]

| SEQ ID NO: | Sequence name | hRPS25 expression rate @ 50 nM | SEQ ID NO: | Sequence name | hRPS25 expression rate @ 50 nM |
|---|---|---|---|---|---|
| 95 | h125-139-B | 0.17 | 117 | h324-342-A | 0.16 |
| 96 | h125-139-C | 0.32 | 118 | h324-342-B | 0.13 |
| 97 | h124-140-A | 0.05 | 119 | h444-458-B | 0.05 |
| 98 | h124-140-B | 0.30 | 120 | h444-458-C | 0.35 |
| 99 | h123-141-A | 0.13 | 121 | h442-458-A | 0.10 |
| 100 | h123-141-B | 0.35 | 122 | h442-458-B | 0.15 |
| 101 | h187-201-B | 0.12 | 123 | h442-460-A | 0.18 |
| 102 | h187-201-C | 0.19 | 124 | h442-460-B | 0.25 |
| 103 | h186-202-A | 0.14 | 125 | h451-465-B | 0.11 |
| 104 | h186-202-B | 0.15 | 126 | h451-465-C | 0.21 |
| 105 | h185-203-A | 0.12 | 127 | h450-466-A | 0.05 |
| 106 | h185-203-B | 0.29 | 128 | h450-466-B | 0.11 |
| 107 | h213-227-B | 0.44 | 129 | h449-467-A | 0.03 |
| 108 | h213-227-C | 1.00 | 130 | h449-467-B | 0.03 |
| 109 | h212-228-A | 0.58 | 131 | h39-53-A | 0.54 |
| 110 | h212-228-B | 0.60 | 132 | h40-54-A | 0.67 |
| 111 | h211-229-A | 0.49 | 133 | h98-112-A | 0.69 |
| 112 | h211-229-B | 0.63 | 134 | h99-113-A | 0.91 |
| 113 | h326-340-B | 0.04 | 135 | h100-114-A | 0.89 |
| 114 | h326-340-C | 0.06 | 136 | h104-118-A | 0.32 |
| 115 | h325-341-A | 0.13 | 137 | h105-119-A | 0.61 |
| 116 | h325-341-B | 0.08 | 138 | h106-120-A | 0.59 |

[Table 5-4]

| SEQ ID NO: | Sequence name | hRPS25 expression rate @ 50 nM | SEQ ID NO: | Sequence name | hRPS25 expression rate @ 50 nM |
|---|---|---|---|---|---|
| 139 | h107-121-A | 0.87 | 166 | h294-308-A | 0.83 |
| 140 | h123-137-C | 0.18 | 167 | h298-312-A | 1.07 |
| 141 | h127-141-A | 0.81 | 168 | h299-313-A | 0.64 |
| 142 | h128-142-A | 0.92 | 169 | h300-314-A | 0.15 |
| 143 | h129-143-A | 0.45 | 170 | h321-335-A | 0.22 |
| 144 | h130-144-A | 0.86 | 171 | h322-336-A | 0.19 |
| 145 | h185-199-C | 0.71 | 172 | h323-337-A | 0.09 |
| 146 | h186-200-C | 0.59 | 173 | h324-338-A | 0.11 |
| 147 | h190-204-A | 0.65 | 174 | h328-342-A | 0.59 |
| 148 | h211-225-A | 0.47 | 175 | h329-343-A | 1.17 |
| 149 | h212-226-A | 0.74 | 176 | h330-344-A | 1.15 |

(continued)

| SEQ ID NO: | Sequence name | hRPS25 expression rate | SEQ ID NO: | Sequence name | hRPS25 expression rate |
|---|---|---|---|---|---|
| | | @ 50 nM | | | @ 50 nM |
| 150 | h215-229-A | 0.43 | 177 | h331-345-A | 0.93 |
| 151 | h218-232-A | 1.09 | 178 | h426-440-A | 1.08 |
| 152 | h221-235-A | 0.71 | 179 | h427-441-A | 0.94 |
| 153 | h222-236-A | 0.82 | 180 | h428-442-A | 0.90 |
| 154 | h223-237-A | 0.93 | 181 | h429-443-A | 1.17 |
| 155 | h224-238-A | 1.01 | 182 | h437-451-A | 0.75 |
| 156 | h255-269-A | 0.74 | 183 | h443-457-A | 0.08 |
| 157 | h256-270-A | 0.79 | 184 | h445-459-A | 0.32 |
| 158 | h257-271-A | 0.55 | 185 | h448-462-A | 0.29 |
| 159 | h258-272-A | 0.49 | 186 | h449-463-A | 0.39 |
| 160 | h262-276-A | 0.71 | 187 | h450-464-A | 0.63 |
| 161 | h263-277-A | 0.50 | 188 | h452-466-A | 0.12 |
| 162 | h264-278-A | 0.04 | 189 | h453-467-A | 0.34 |
| 163 | h291-305-A | 0.91 | 190 | h454-468-A | 0.14 |
| 164 | h292-306-A | 0.78 | 191 | h455-469-A | 0.81 |
| 165 | h293-307-A | 0.78 | | | |

[Table 5-5]

| SEQ ID NO: | Sequence name | hRPS25 expression rate | SEQ ID NO: | Sequence name | hRPS25 expression rate |
|---|---|---|---|---|---|
| | | @ 50 nM | | | @ 50 nM |
| 303 | h125-140-A | 0.22 | 325 | h295-309-C | 0.68 |
| 304 | h125-140-B | 0.18 | 326 | h296-310-B | 0.04 |
| 305 | h124-140-C | 0.07 | 327 | h296-310-C | 0.10 |
| 306 | h187-201-D | 0.06 | 328 | h300-314-B | 0.25 |
| 307 | h187-201-E | 0.21 | 329 | h300-314-C | 0.32 |
| 308 | h186-201-A | 0.40 | 330 | h301-315-A | 0.40 |
| 309 | h186-202-A | 0.05 | 331 | h302-316-A | 0.09 |
| 310 | h186-202-B | 0.18 | 332 | h303-317-A | 0.13 |
| 311 | h186-202-C | 0.47 | 333 | h304-318-A | 0.46 |
| 312 | h186-203-A | 0.08 | 334 | h296-311-A | 0.15 |
| 313 | h214-228-B | 0.12 | 335 | h296-311-B | 0.29 |
| 314 | h213-228-A | 0.51 | 336 | h295-311-A | 0.24 |
| 315 | h214-229-A | 0.64 | 337 | h300-316-A | 0.18 |
| 316 | h259-273-B | 1.00 | 338 | h323-337-B | 0.29 |
| 317 | h265-279-A | 0.10 | 339 | h323-337-C | 0.69 |
| 318 | h266-280-A | 0.97 | 340 | h326-340-D | 0.04 |

(continued)

| SEQ ID NO: | Sequence name | hRPS25 expression rate @ 50 nM | SEQ ID NO: | Sequence name | hRPS25 expression rate @ 50 nM |
|---|---|---|---|---|---|
| 319 | h267-281-A | 0.45 | 341 | h326-340-E | 0.09 |
| 320 | h268-282-A | 0.64 | 342 | h325-340-A | 0.19 |
| 321 | h259-274-A | 0.11 | 343 | h325-340-B | 0.15 |
| 322 | h263-279-A | 0.08 | 344 | h326-341-A | 0.17 |
| 323 | h264-280-A | 0.08 | 345 | h326-341-B | 0.25 |
| 324 | h295-309-B | 0.69 | 346 | h326-341-C | 0.06 |

[Table 5-6]

| SEQ ID NO: | Sequence name | hRPS25 expression rate @ 50 nM | SEQ ID NO: | Sequence name | hRPS25 expression rate @ 50 nM |
|---|---|---|---|---|---|
| 347 | h326-341-D | 0.21 | 369 | h442-457-A | 0.66 |
| 348 | h326-341-E | 0.08 | 370 | h442-457-B | 0.39 |
| 349 | h322-338-A | 0.03 | 371 | h443-458-A | 0.16 |
| 350 | h322-3 3 8-B | 0.57 | 372 | h450-465-A | 0.06 |
| 351 | h325-341-C | 0.02 | 373 | h450-465-B | 0.03 |
| 352 | h325-341-D | 0.28 | 374 | h450-465-C | 0.03 |
| 353 | h325-341-E | 0.14 | 375 | h450-465-D | 0.05 |
| 354 | h325-341-F | 0.32 | 376 | h451-466-A | 0.04 |
| 355 | h325-341-G | 0.06 | 377 | h451-466-B | 0.07 |
| 356 | h439-453-B | 0.14 | 378 | h451-466-C | 0.02 |
| 357 | h440-454-B | 0.11 | 379 | h451-466-D | 0.10 |
| 358 | h440-454-C | 0.08 | 380 | h451-466-E | 0.17 |
| 359 | h440-454-D | 0.03 | 381 | h451-466-F | 0.07 |
| 360 | h440-454-E | 0.10 | 382 | h429-445-A | 0.07 |
| 361 | h451-465-D | 0.06 | 383 | h430-446-A | 0.53 |
| 362 | h451-465-E | 0.17 | 384 | h434-450-A | 1.77 |
| 363 | h451-465-F | 0.07 | 385 | h439-455-A | 0.16 |
| 364 | h451-465-G | 0.19 | 386 | h441-457-A | 0.06 |
| 365 | h451-465-H | 0.22 | 387 | h441-457-B | 0.22 |
| 366 | h439-454-A | 0.09 | 388 | h442-458-C | 0.14 |
| 367 | h439-454-B | 0.06 | 389 | h442-458-D | 0.14 |
| 368 | h439-454-C | 0.09 | 390 | h442-458-E | 0.36 |

[Table 5-7]

| SEQ ID NO: | Sequence name | hRPS25 expression rate @ 50 nM | SEQ ID NO: | Sequence name | hRPS25 expression rate @ 50 nM |
|---|---|---|---|---|---|
| 391 | h443-459-A | 0.18 | 413 | h451-467-A | 0.47 |
| 392 | h449-465-A | 0.38 | 414 | h451-468-A | 0.91 |
| 393 | h449-465-B | 0.22 | 415 | h451-468-B | 0.29 |
| 394 | h449-465-C | 0.09 | 416 | h451-469-A | 0.26 |
| 395 | h449-465-D | 0.23 | 417 | h449-467-D | 0.62 |
| 396 | h449-465-E | 0.17 | 418 | h451-465-I | 0.20 |
| 397 | h449-467-C | 0.05 | 419 | h451-465-J | 0.07 |
| 398 | h442-458-F | 0.38 | 420 | h451-465-K | 0.12 |
| 399 | h442-460-C | 0.58 | 421 | h451-465-L | 0.09 |
| 400 | h442-459-A | 0.46 | 422 | h451-465-M | 0.08 |
| 401 | h442-459-B | 0.29 | 423 | h451-465-N | 0.08 |
| 402 | h443-460-A | 0.40 | 424 | h451-465-O | 0.08 |
| 403 | h443-460-B | 0.42 | 425 | h451-465-P | 0.22 |
| 404 | h447-465-A | 0.33 | 426 | h451-467-B | 0.08 |
| 405 | h448-465-A | 0.26 | 427 | h451-467-C | 0.18 |
| 406 | h448-465-B | 0.35 | 428 | h451-465-Q | 0.86 |
| 407 | h449-466-A | 0.17 | 429 | h451-465-R | 0.62 |
| 408 | h449-466-B | 0.15 | 430 | h451-467-D | 0.24 |
| 409 | h450-467-A | 0.40 | 431 | h451-467-E | 0.10 |
| 410 | h448-466-A | 0.17 | 432 | h451-467-F | 0.08 |
| 411 | h450-467-B | 0.47 | 469 | h451-465-AN | 0.88 |
| 412 | h450-468-A | 0.58 | 470 | h451-465-AO | 0.95 |

[Table 6-1]

| SEQ ID NO: | Sequence name | Final concentration (nM) | hRPS25 expression rate |
|---|---|---|---|
| 24 | h102-116-A | 5 | 1.19 |
| | | 15 | 1.36 |
| | | 50 | 0.26 |
| 25 | h103-117-A | 5 | 0.89 |
| | | 15 | 0.63 |
| | | 50 | 0.05 |
| 28 | h125-139-A | 5 | 0.53 |
| | | 15 | 0.64 |
| | | 50 | 0.17 |
| 64 | h296-310-A | 5 | 1.02 |
| | | 15 | 0.26 |
| | | 50 | 0.04 |

(continued)

| SEQ ID NO: | Sequence name | Final concentration (nM) | hRPS25 expression rate |
|---|---|---|---|
| 80 | h431-445-A | 5 | 1.07 |
| | | 15 | 0.35 |
| | | 50 | 0.11 |
| 91 | h444-458-A | 5 | 0.77 |
| | | 15 | 0.18 |
| | | 50 | 0.07 |
| 94 | h451-465-A | 5 | 0.24 |
| | | 15 | 0.31 |
| | | 50 | 0.10 |

[Table 6-2]

| SEQ ID NO: | Sequence name | Final concentration (nM) | hRPS25 expression rate |
|---|---|---|---|
| 116 | h325-341-B | 0.5 | 0.74 |
| | | 5 | 0.23 |
| | | 50 | 0.02 |
| 126 | h451-465-C | 0.5 | 0.12 |
| | | 5 | 0.03 |
| | | 50 | 0.06 |
| 128 | h450-466-B | 0.5 | 0.19 |
| | | 5 | 0.03 |
| | | 50 | 0.12 |
| 130 | h449-467-B | 0.5 | 0.22 |
| | | 5 | 0.04 |
| | | 50 | 0.03 |
| 409 | h450-467-A | 0.5 | 0.63 |
| | | 5 | 0.13 |
| | | 50 | 0.04 |

Similarly, the expression rate of human RPS25 mRNA in the single-stranded antisense oligonucleotides for human RPS25 mRNA precursor is shown in Table 7-1 to Table 7-5. The single-stranded antisense oligonucleotide used in lipofection was adjusted to have a final concentration of 50 nM or 100 nM.

[Table 7-1]

| SEQ ID NO: | Sequence name | hRPS25 expression rate | |
|---|---|---|---|
| | | @ 50 nM | @ 100 nM |
| 192 | hp1-15-A | 0.84 | 0.38 |
| 193 | hp72-86-A | 0.91 | 1.12 |
| 194 | hp73-87-A | 1.10 | 1.08 |

(continued)

| SEQ ID NO: | Sequence name | hRPS25 expression rate | |
|---|---|---|---|
| | | @ 50 nM | @ 100 nM |
| 195 | hp74-88-A | 0.93 | 1.05 |
| 196 | hp75-89-A | 0.74 | 0.73 |
| 197 | hp76-90-A | 0.83 | 0.86 |
| 198 | hp231-245-A | 1.08 | 0.92 |
| 199 | hp232-246-A | 1.15 | 1.10 |
| 200 | hp233-247-A | 1.26 | 0.71 |
| 201 | hp261-275-A | 0.98 | 0.56 |
| 202 | hp262-276-A | 0.82 | 1.04 |
| 203 | hp278-292-A | 0.46 | 0.30 |
| 204 | hp279-293-A | 0.86 | 0.46 |
| 205 | hp280-294-A | 0.75 | 0.73 |
| 206 | hp390-404-A | 0.88 | 0.73 |
| 207 | hp392-406-A | 0.88 | 0.68 |
| 208 | hp417-431-A | 0.29 | 0.29 |
| 209 | hp418-432-A | 0.22 | 0.20 |
| 210 | hp419-433-A | 0.43 | 0.53 |
| 211 | hp420-434-A | 0.39 | 0.46 |
| 212 | hp421-435-A | 0.45 | 0.59 |
| 213 | hp422-436-A | 0.53 | 0.67 |
| 214 | hp423-437-A | 1.22 | 0.65 |
| 215 | hp445-459-A | 0.81 | 0.77 |
| 216 | hp446-460-A | 0.65 | 0.72 |

[Table 7-2]

| SEQ ID NO: | Sequence name | hRPS25 expression rate | |
|---|---|---|---|
| | | @ 50 nM | @ 100 nM |
| 217 | hp447-461-A | 0.55 | 0.68 |
| 218 | hp448-462-A | 1.22 | 1.12 |
| 219 | hp458-472-A | 0.95 | 1.27 |
| 220 | hp460-474-A | 1.01 | 0.70 |
| 221 | hp461-475-A | 1.02 | 0.62 |
| 222 | hp510-524-A | 0.65 | 0.79 |
| 223 | hp561-575-A | 0.61 | 0.48 |
| 224 | hp562-576-A | 0.77 | 0.67 |
| 225 | hp589-603-A | 1.75 | 0.70 |

(continued)

| SEQ ID NO: | Sequence name | hRPS25 expression rate | |
|---|---|---|---|
| | | @ 50 nM | @ 100 nM |
| 226 | hp605-619-A | 0.58 | 0.40 |
| 227 | hp606-620-A | 0.85 | 0.99 |
| 228 | hp626-640-A | 0.73 | 0.61 |
| 229 | hp627-641-A | 0.38 | 0.17 |
| 230 | hp628-642-A | 0.73 | 0.70 |
| 231 | hp629-643-A | 0.82 | 1.03 |
| 232 | hp632-646-A | 0.40 | 0.27 |
| 233 | hp633-647-A | 0.38 | 0.25 |
| 234 | hp634-648-A | 0.48 | 0.33 |
| 235 | hp654-668-A | 1.02 | 1.03 |
| 236 | hp681-695-A | 1.07 | 0.87 |
| 237 | hp696-710-A | 0.55 | 0.61 |
| 238 | hp697-711-A | 0.47 | 0.39 |
| 239 | hp761-775-A | 0.89 | 1.02 |
| 240 | hp762-776-A | 0.75 | 0.88 |
| 241 | hp764-778-A | 1.10 | 0.82 |

[Table 7-3]

| SEQ ID NO: | Sequence name | hRPS25 expression rate | |
|---|---|---|---|
| | | @ 50 nM | @ 100 nM |
| 242 | hp1034-1048-A | 0.53 | 0.52 |
| 243 | hp1035-1049-A | 0.70 | 0.49 |
| 244 | hp1103-1117-A | 1.05 | 0.63 |
| 245 | hp1104-1118-A | 0.74 | 0.57 |
| 246 | hp1105-1119-A | 0.83 | 0.60 |
| 247 | hp1106-1120-A | 0.96 | 0.78 |
| 248 | hp1107-1121-A | 0.84 | 0.75 |
| 249 | hp1108-1122-A | 1.08 | 0.92 |
| 250 | hp1110-1124-A | 1.31 | 0.97 |
| 251 | hp1128-1142-A | 0.70 | 0.34 |
| 252 | hp1129-1143-A | 0.80 | 0.52 |
| 253 | hp1196-1210-A | 0.74 | 0.29 |
| 254 | hp1197-1211-A | 0.51 | 0.26 |
| 255 | hp1217-1231-A | 1.19 | 1.12 |
| 256 | hp1218-1232-A | 1.04 | 0.93 |

(continued)

| SEQ ID NO: | Sequence name | hRPS25 expression rate | |
|---|---|---|---|
| | | @ 50 nM | @ 100 nM |
| 257 | hp1219-1233-A | 1.14 | 0.91 |
| 258 | hp1398-1412-A | 0.67 | 0.65 |
| 259 | hp1399-1413-A | 0.80 | 0.83 |
| 260 | hp1402-1416-A | 1.14 | 1.45 |
| 261 | hp1408-1422-A | 1.13 | 0.70 |
| 262 | hp1409-1423-A | 0.68 | 0.29 |
| 263 | hp1410-1424-A | 0.75 | 0.31 |
| 264 | hp1411-1425-A | 1.07 | 0.69 |
| 265 | hp1412-1426-A | 0.91 | 0.77 |
| 266 | hp1478-1492-A | 0.73 | 0.35 |

[Table 7-4]

| SEQ ID NO: | Sequence name | hRPS25 expression rate | |
|---|---|---|---|
| | | @ 50 nM | @ 100 nM |
| 267 | hp1480-1494-A | 0.71 | 0.69 |
| 268 | hp1715-1729-A | 0.84 | 0.49 |
| 269 | hp1749-1763-A | 0.99 | 0.50 |
| 270 | hp 1750-1764-A | 0.70 | 0.49 |
| 271 | hp1751-1765-A | 0.99 | 0.76 |
| 272 | hp1763-1777-A | 1.04 | 0.95 |
| 273 | hp1793-1807-A | 0.91 | 0.81 |
| 274 | hp1885-1899-A | 1.08 | 0.90 |
| 275 | hp1887-1901-A | 1.01 | 1.04 |
| 276 | hp2047-2061-A | 0.61 | 0.26 |
| 277 | hp2048-2062-A | 0.95 | 0.51 |
| 278 | hp2049-2063-A | 0.67 | 0.35 |
| 279 | hp2121-2135-A | 0.99 | 0.59 |
| 280 | hp2122-2136-A | 1.01 | 0.93 |
| 281 | hp2123-2137-A | 0.75 | 0.58 |
| 282 | hp2124-2138-A | 0.93 | 1.06 |
| 283 | hp2260-2274-A | 0.99 | 0.79 |
| 284 | hp2261-2275-A | 1.20 | 0.72 |
| 285 | hp2262-2276-A | 1.04 | 0.69 |
| 286 | hp2268-2282-A | 1.00 | 0.56 |
| 287 | hp2269-2283-A | 1.18 | 0.99 |

(continued)

| SEQ ID NO: | Sequence name | hRPS25 expression rate | |
|---|---|---|---|
| | | @ 50 nM | @ 100 nM |
| 288 | hp2271-2285-A | 0.90 | 1.17 |
| 289 | hp2277-2291-A | 0.85 | 0.83 |
| 290 | hp2339-2353-A | 0.97 | 1.27 |
| 291 | hp2341-2355-A | 1.03 | 0.96 |

[Table 7-5]

| SEQ ID NO: | Sequence name | hRPS25 expression rate | |
|---|---|---|---|
| | | @ 50 nM | @ 100 nM |
| 292 | hp2342-2356-A | 0.57 | 0.40 |
| 293 | hp2386-2400-A | 1.05 | 0.97 |
| 294 | hp2406-2420-A | 0.73 | 0.39 |
| 295 | hp2538-2552-A | 1.07 | 1.39 |
| 296 | hp2540-2554-A | 0.88 | 0.87 |
| 297 | hp2541-2555-A | 1.20 | 1.00 |
| 298 | hp2585-2599-A | 0.39 | 0.13 |
| 299 | hp2586-2600-A | 0.39 | 0.14 |
| 300 | hp2587-2601-A | 0.23 | 0.09 |
| 301 | hp2583-2597-A | 0.88 | |
| 302 | hp2584-2598-A | 0.97 | |

<Expression Evaluation with Use of Mouse Primary Culture Neurons>

Mouse primary culture neurons were cultured in a culture medium under the conditions of 37°C and 5% $CO_2$. The culture medium used for mouse primary culture neurons had the composition as described below.

Composition of Culture Medium for Mouse Primary Culture Neurons

B-27 Electrophysiology Kit: manufactured by gibco, Cat# A1413701
100-Fold diluted 200-mM L-glutamine solution: manufactured by Nacalai Tesque, Cat# 16948-04
100-Fold diluted penicillin-streptomycin mixed solution: manufactured by Nacalai Tesque, Cat# 09367-34 (penicillin 10000 units/ml, streptomycin 10000 μg/ml, a stabilizer contained)

Firstly, mouse primary culture neurons (derived from mouse fetal cerebrum) were plated in a 96-well plate at 40000 cells/well, followed by culturing under the conditions of 37°C and 5% $CO_2$ for 5 days. Subsequently, the single-stranded antisense oligonucleotide diluted with phosphate-buffered physiological saline (PBS) (with a final concentration of 0.01 μM, 0.1 μM, or 1 μM) was added to the culture medium. For the negative control group, PBS that was free of single-stranded antisense oligonucleotide was added to the culture medium. The cells were cultured in the culture medium under the conditions of 37°C and 5% COz for 48 hours. Subsequently, the culture medium was removed, and with the use of Taqman Fast Cells-to-CT Kit (manufactured by Thermo Fisher Scientific, Cat# 4399003), reverse transcription reaction was carried out for the extracted total RNA. With the use of the resulting complementary DNA (cDNA)

obtained from the reverse transcription reaction, Taqman gene expression assays (manufactured by Applied Biosystems), and a gene-specific probe designed in advance (see below), real-time PCR was carried out (40 cycles of 95°C for 3 seconds and 60°C for 30 seconds).

List of Gene-Specific Probes Used in Evaluation of Mouse RPS25 Gene Expression

Mouse Rps25: Mm02342783_g1
Mouse GAPDH: 4352339E (internal control)

The expression rate of mouse RPS25 mRNA in the single-stranded antisense oligonucleotides as determined by the above-described method is shown in Table 8. At this time, the expression rate of mouse RPS25 mRNA for the negative control group was defined as 1.00. Generally, it is expected that when mRNA expression is reduced, subsequent protein translation and the like are also reduced; hence, it can be judged that one with an expression rate of 0.80 or less is a single-stranded antisense oligonucleotide capable of modulating the function of mouse RPS25 gene. The target region to which the single-stranded antisense oligonucleotide h451-465-A binds is a region whose sequence is conserved between human RPS25 gene and mouse RPS25 gene.

[Table 8]

| SEQ ID NO: | Sequence name | Final concentration (μM) | mRPS25 expression rate |
|---|---|---|---|
| 94 | h451-465-A | 0.01 | 0.89 |
| | | 0.1 | 0.62 |
| | | 1 | 0.25 |

«Evaluation with Use of Motor Neurons Derived from Human iPS Cells»

Motor neurons were induced and differentiated from human iPS cells and used for evaluation. Maintaining the cells and induced differentiation thereof were carried out in a medium which is described below, under the conditions of 37°C and 5% $CO_2$.

List of Medium Compositions

(SNL cell medium)

DMEM (manufactured by Sigma-Aldrich, Cat# D6429),
100-Fold diluted penicillin-streptomycin mixed solution (manufactured by ThermoFisher Scientific, Cat# 15140-122)
10-Fold diluted fetal bovine serum (manufactured by ThermoFisher Scientific, Cat# 10437-028)

(iPS cell medium)

Medium for primate ES/iPS cells (manufactured by REPROCELL, Cat# RCHEMD001B)
100-Fold diluted penicillin-streptomycin mixed solution (manufactured by ThermoFisher Scientific, cat# 15140-122)

(Mixed medium A)

DMEM/Ham's F12 GlutaMAX (manufactured by ThermoFisher Scientific, Cat# 10565-018)
2 mM L-glutamine (manufactured by ThermoFisher Scientific, Cat# 25030-081)
Non-Essential Amino Acid (NEAA) (manufactured by ThermoFisher Scientific, Cat# 11140-050)

100-Fold diluted penicillin-streptomycin mixed solution (manufactured by ThermoFisher Scientific, cat# 15140-122)
2 μg/mL Heparin (manufactured by Sigma-Aldrich, H-4784)
N2 supplement (manufactured by ThermoFisher Scientific, Cat# 17502-048)

(Mixed medium B)

Neurobasal medium (manufactured by ThermoFisher Scientific, cat# 21103-049)
2 mM L-glutamine (manufactured by ThermoFisher Scientific, Cat# 25030-081)
Non-Essential Amino Acid (NEAA) (manufactured by ThermoFisher Scientific, Cat# 11140-050)
Antibiotic-Antimycotic (manufactured by ThermoFisher Scientific, cat# 15240-062)
2 μg/mL Heparin (manufactured by Sigma-Aldrich, H-4784)
N2 supplement (manufactured by ThermoFisher Scientific, Cat# 17502-048)
10 ng/mL IGF-1 (manufactured by PeproTech, cat# 100-11)
10 ng/mL Human CNTF (manufactured by PeproTech, cat# 450-13)
10 ng/mL Human GDNF (manufactured by R&D Systems, cat# 212-GD-050)
B27 supplement (manufactured by ThermoFisher Scientific, cat# 12587010)
200 μM Ascorbic acid (manufactured by Sigma-Aldrich, Cat# A5960)
10 ng/mL Human BDNF (manufactured by Peprotech, Cat# 450-02)

(Neuron medium)

Neurobasal medium Electro (manufactured by ThermoFisher Scientific, cat# A14098-01)
2 mM L-glutamine (manufactured by ThermoFisher Scientific, Cat# 25030-081)
Non-Essential Amino Acid (NEAA) (manufactured by ThermoFisher Scientific, Cat# 11140-050)
Antibiotic-Antimycotic (manufactured by ThermoFisher Scientific, cat# 15240-062)
2 μg/mL Heparin (manufactured by Sigma-Aldrich, H-4784)
N2 supplement (manufactured by ThermoFisher Scientific, Cat# 17502-048)
10 ng/mL IGF-1 (manufactured by PeproTech, cat# 100-11)
10 ng/mL Human CNTF (manufactured by PeproTech, cat# 450-13)
10 ng/mL Human GDNF (manufactured by R&D Systems, cat# 212-GD-050)
B27 supplement, Electro (manufactured by ThermoFisher Scientific, cat# A14097-01)
200 μM Ascorbic acid (manufactured by Sigma-Aldrich, Cat# A5960)
10 ng/mL Human BDNF (manufactured by PeproTech, Cat# 450-02)
25 μM 2-mercaptoethanol (manufactured by ThermoFisher Scientific, cat# 21985-0123)
0.1% bovine serum albumin (manufactured by Sigma-Aldrich, cat# A9576)

<Mitomycin Treatment of Feeder Cells>

As feeder cells for plating human iPS cells, SNL cells (manufactured by Cell Biolabs, Cat# CBA-316) treated with mitomycin were prepared. The mitomycin treatment of SNL cells was carried out in the manner described below. Firstly, 0.1% gelatin (manufactured by FUJIFILM Wako Pure Chemical, Cat# 190-15805) was added to a 10-cm petri dish (manufactured by IWAKI, Cat# 3020-100), which was then left to stand for at least 1 hour in an incubator under the conditions of 37°C and 5% $CO_2$ (hereinafter, this procedure may also be called "gelatin treatment"). Subsequently, excess gelatin was sucked out of the petri dish, and, with the use of the SNL cell medium, SNL cells which had been thawed were plated in the petri dish at $1\times10^6$ to $2\times10^6$ cells per petri dish. Every 3 to 4 days, the cells were diluted 8 to 16 folds and passaged, until the cells proliferated into the necessary cell count. Then, in a 15-cm petri dish (manufactured by IWAKI, Cat# 3030-150) with 0.1-% gelatin treatment, the SNL cells were plated at $2\times10^6$ to $4\times10^6$ cells per petri dish. After the cells were cultured to 80 to 90% confluency, mitomycin C (manufactured by Kyowa Kirin, YJ code 4231400D1031) diluted to 0.4 mg/mL with the SNL cell medium was added to the petri dish in a final concentration of 6.2 μg/mL. The petri dish was left to stand in an incubator under the conditions of 37°C and 5% COz for 2 hours and 15 minutes. Subsequently, the medium was removed from the petri dish, and the SNL cells were rinsed once with PBS. 2.5% trypsin/EDTA (manufactured by ThermoFisher Scientific, Cat# 15090-046) was diluted with PBS (final concentration, 0.25%), and then added to the SNL cells, which were then left to stand for 1 minute at room temperature. Subsequently, the SNL cells were collected into a tube, centrifuged, suspended in CELLBANKER (R) (manufactured by

ZENOAQ Resource, Cat# CB011), and then cryopreserved.

<Maintaining Human iPS Cells>

0.1% gelatin was added to a 10-cm petri dish, which was then left to stand in an incubator under the conditions of 37°C and 5% $CO_2$ for at least 1 hour. The mitomycin-treated SNL cells were suspended with the use of the SNL cell medium. Subsequently, the SNL cells ($1.5 \times 10^6$ cells) were plated in the 10-cm petri dish, followed by culturing for 2 to 3 days. Then, the SNL cell medium was removed from the petri dish, and the SNL cells were rinsed with PBS. Subsequently, into the petri dish, human iPS cells (strain 201B7, obtained from iPS Academia Japan, AJ-H1-01) suspended in the iPS cell medium containing Y-27632 (manufactured by Tocris, Cat# 1254) in an amount of 1/1000 were plated. The medium was changed every day from two days after the plating, until the start of induced differentiation.

<Induced Differentiation of Human iPS Cells to Motor Neurons>

Y-27632 (final concentration, 10 μM) was added to the cell culture liquid of the human iPS cells, and, in this way, the iPS cells were exposed to the Y-27632 for at least 1 hour. Culture supernatant was removed and the cells were rinsed with PBS, and then Cell dissociation solution (CTK solution) (manufactured by REPROCELL, Cat# RCHETP002) was added thereto for reaction at room temperature for 1 minute. The CTK solution was removed, and the cells were rinsed twice with PBS, followed by addition of 1 mL of the iPS cell medium. The cells were scraped off with the use of a cell scraper, and passed through a cell strainer (manufactured by Becton, Dickinson, Cat# 352350) to disperse the cell clusters to obtain cell suspension. The resulting suspension was transferred to a 6-well plate (manufactured by Corning, Cat# 3471). The medium was changed to the mixed medium A supplemented with LDN193189 (manufactured by Stemgent, Cat# 04-0074) (final concentration, 0.3 μM), SB431542 (manufactured by Tocris, Cat# 1614) (final concentration, 2 μM), CHIR-99021 (manufactured by Stemgent, Cat# 04-0004-10) (final concentration, 3 μM), and Y-27632 (final concentration, 10 μM), and the cells were cultured in an incubator under the conditions of 37°C and 5% COz (Culture Day 0).

On Culture Day 2 and 4, the culture liquid was removed with a pipette, and the medium was changed to a fresh medium, which was the mixed medium A supplemented with LDN193189 (final concentration, 0.3 μM), SB431542 (final concentration, 2 μM), and CHIR-99021 (final concentration, 3 μM).

On Culture Day 7, 9, and 11, the culture liquid was removed with a pipette, and the medium was changed to a fresh medium, which was the mixed medium A supplemented with LDN193189 (final concentration, 0.3 μM), SB431542 (final concentration, 2 μM), CHIR-99021 (final concentration, 3 μM), Purmorphamine (manufactured by FUJIFILM Wako Pure Chemical, Cat# 166-23991) (final concentration, 0.5 μM), and Retinoic Acid (manufactured by Sigma-Aldrich, Cat# R2625) (final concentration, 0.1 μM).

On Culture Day 14 and 16, the culture liquid was removed with a pipette, and the medium was changed to a fresh medium, which was the mixed medium A supplemented with Purmorphamine (final concentration, 0.5 μM), Retinoic Acid (final concentration, 0.1 μM), Human BDNF (final concentration, 10 ng/mL), and Ascorbic Acid (manufactured by Sigma-Aldrich, Cat# A5960) (final concentration, 200 μM).

On Culture Day 18, the medium was changed to a fresh medium, which was the mixed medium B supplemented with Purmorphamine (final concentration, 0.5 μM), Retinoic Acid (final concentration, 0.1 μM), and Compound E (manufactured by Calbiochem, Cat# 565790) (final concentration, 0.1 μM).

On Culture Day 21, cell clusters were rinsed with PBS, followed by centrifugation to remove the supernatant. To the cell clusters, Accutase (manufactured by Innovative Cell Technologies, Cat# AT104) and Y27632 (final concentration, 10 μM) were added, followed by incubation at 37°C for 10 minutes. The cell clusters were cooled with ice, and then the cell clusters were dispersed by pipetting. After centrifugation (300×g, 5 minutes, 4°C), a process of collecting the precipitated cells and suspending them in the mixed medium B was repeated twice. Thus, motor neurons derived from iPS cells were obtained. The resulting motor neurons were suspended in CELLBANKER (R), split into portions, and frozen for storage.

<Culturing of Motor Neurons Derived from Human iPS Cells, and Evaluation of Single-Stranded Antisense Nucleotide>

To a 96 Well Optical Btm Pit Polybase Black w/ Lid Cell Culture Sterile PS (manufactured by ThermoFisher Scientific, Cat# 165305), Poly-L-Ornitine Solution (PLO) solution (manufactured by Sigma-Aldrich, Cat# P4957) diluted 6.66 folds with PBS was added, followed by leaving to stand at room temperature for at least 2 hours. After rinsing with PBS three times, iMatrix diluted with PBS was added to the plate in a concentration of 0.5 μg/$cm^2$, followed by leaving to stand overnight at 4°C. Then, the motor neurons derived from human iPS cells previously cryopreserved were thawed, and suspended in the neuron medium. Subsequently, the supernatant was removed by centrifugation, and the cells were resuspended in the neuron medium containing Culture One Supplement (manufactured by ThermoFisher Scientific,

A3320201) in an amount of 1/100 and Compound E (final concentration, 0.1 μM). These cells were plated in a coated 96-well plate at 30000 cells/well, and cultured in an incubator under the conditions of 37°C and 5% $CO_2$ for 28 days. Every 2 to 3 days, half the amount of the neuron medium was changed. Until Day 7 after the start of the culturing, as the neuron medium, a medium containing Culture One Supplement and Compound E was used.

After plating, on Culture Day 1, 11, 18, 26 (each of them is expressed as D1, D11, D18, D26), the single-stranded antisense oligonucleotide (in a final concentration of 0.01 μM, 0.1 μM, 1 μM) diluted with PBS was added to the culture medium. For cells of the negative control group, PBS that was free of single-stranded antisense oligonucleotide was added to the culture medium. The cells were cultured in the culture medium under the conditions of 37°C and 5% $CO_2$ for 48 hours or 72 hours, and then the medium containing the single-stranded antisense nucleotide was removed, followed by continued culture in the neuron medium (every 2 to 3 days, half the amount of the medium was changed). Subsequently, the culture medium was removed, and with the use of Taqman Fast Cells-to-CT Kit (manufactured by Thermo Fisher Scientific, Cat# 4399003), reverse transcription reaction was carried out for the extracted total RNA. With the use of the resulting complementary DNA (cDNA) obtained from the reverse transcription reaction, Taqman gene expression assays (manufactured by Applied Biosystems), and a gene-specific probe designed in advance (see below), real-time PCR was carried out (40 cycles of 95°C for 3 seconds and 60°C for 30 seconds). Results are given in Table 9. "Expression-reducing rate" in Table 9 refers to the value calculated by Equation (1) below.

$$\text{(Expression-reducing rate)} = 1\text{-(Expression rate)} \quad \text{(Equation (1))}$$

It was judged that, the greater the value of the expression-reducing rate, the more likely the single-stranded antisense oligonucleotide is capable of reducing expression of human RPS25 mRNA.

List of Gene-Specific Probes Used in Evaluation of Human RPS25 Gene Expression

Human RPS25: Hs01568661_g1
Human GAPDH: 4326317E (internal control)

[Table 9]

| SEQ ID NO: | Sequence name | Day of introduction | Final concentration (μM) | mRPS25 expression-reducing rate |
|---|---|---|---|---|
| 94 | h451-465-A | D1 | 0.01 | 0 |
| | | | 0.1 | 0.79 |
| | | | 1 | 0.58 |
| | | D11 | 0.01 | 0.47 |
| | | | 0.1 | 0.57 |
| | | | 1 | 0.82 |
| | | D18 | 0.01 | 0.29 |
| | | | 0.1 | 0.57 |
| | | | 1 | 0.85 |
| | | D26 | 0.01 | 0 |
| | | | 0.1 | 0.24 |
| | | | 1 | 0.68 |

(continued)

| SEQ ID NO: | Sequence name | Day of introduction | Final concentration (μM) | mRPS25 expression-reducing rate |
|---|---|---|---|---|
| 24 | h102-116-A | D11 | 0.01 | 0.65 |
| | | | 0.1 | 0.62 |
| | | | 1 | 0.93 |
| | | D18 | 0.01 | 0.09 |
| | | | 0.1 | 0.52 |
| | | | 1 | 0.87 |
| | | D26 | 0.01 | 0.08 |
| | | | 0.1 | 0.5 |
| | | | 1 | 0.64 |

«Evaluation of RPS25 Protein Expression»

Evaluation of RPS25 protein expression was carried out with the use of human fetal kidney cells, depending on the single-stranded antisense oligonucleotide thus produced. The evaluation of the amount of protein expression according to the present example means evaluation of the amount of protein translated from mRNA. In the following, the specific procedure of the expression evaluation will be described.

<Expression Evaluation with Use of Human Fetal Kidney Cells>

Human fetal kidney cells HEK293T (ATCC (registered trademark) CRL-3216 (trademark)) were cultured in a culture medium under the conditions of 37°C and 5% $CO_2$. The culture medium used for HEK293T cells had the composition as described below.

Composition of Culture Medium for HEK293T Cells

Dulbecco modified Eagle medium (DMEM): manufactured by SIGMA, Cat# D6429
10% fetal bovine serum (FBS): manufactured by biowest, Cat# S1820
100-Fold diluted penicillin-streptomycin mixed solution: manufactured by Nacalai Tesque, Cat# 09367-34 (penicillin 10000 units/ml, streptomycin 10000 μg/ml, a stabilizer contained)

<Quantitative Protein Analysis by Western Blotting>

Firstly, HEK293T cells were plated in a 6-well plate (500000 cells/well), and cultured overnight under the conditions of 37°C and 5% COz. Subsequently, the cells were transfected by lipofection with the single-stranded antisense oligonucleotide diluted with phosphate-buffered physiological saline (PBS) (final concentration, 50 nM). As the negative control group, cells transfected with PBS that was free of single-stranded antisense oligonucleotide were used. The transfected cells were cultured in a growth medium under the conditions of 37°C and 5% $CO_2$ for 48 hours. Subsequently, the growth medium was removed, followed by rinsing with PBS, and then the cells were collected with the use of a cell scraper. The liquid thus collected was centrifuged under the conditions of 2700×g, 5 minutes, and 4°C to precipitate the cells. After the supernatant was removed, 1 mL of RIPA Lysis and Extraction buffer (manufactured by ThemoFisher Scientific, Cat# 89900) containing Protease Inhibitor (manufactured by ThermoFisher Scientific, Cat# 1860932) in an amount of 1/100 was added, followed by disrupting the cells with the use of an ultrasonic homogenizer. Subsequently, centrifugation was carried out under the conditions of 15000×g, 10 minutes, and 4°C, and the supernatant was to be used as a sample. The sample thus collected was subjected to protein quantification with the use of Pierce (trademark) BCA Protein Assay kit (manufactured by ThermoScientific, Cat# 23225). All samples were adjusted to have the same concentration, and then Pierce (trademark) Lane Marker Reducing Sample Buffer (manufactured by ThermoFisher Scientifier, Cat# 39000) was added, followed by heat treatment at 95°C for 5 minutes. The sample thus prepared was loaded in an amount of protein of 10 μg to 20 μg/lane, followed by electrophoresis. The electrophoresis was carried out

with the use of Criterion (trademark) TDX (trademark) Precast Gel 4-15% (manufactured by BIO-RAD, Cat# 5671085J10) under the conditions of constant voltage of 200 V for 30 minutes. As the electrophoresis buffer, Running Buffer Solution (10×) for SDS-PAGE (manufactured by Nacalai Tesque, Cat# 30329-61) diluted to 1× concentration was used.

After electrophoresis, transfer was carried out in the semi-dry mode. Trans-Blot Turbo Transfer Pack (manufactured by BIO-RAD, Cat# 1704157) was used as the membrane, and BIO-RAD Trans-Blot Turbo Transfer System in the Standard protocol (30 minutes) was used as the transfer apparatus. After the transfer, the membrane was rinsed with TBST. The composition of TBST was as follows: tris buffer physiological saline (pH7.4) (manufactured by Nacalai Tesque, Cat# 35438-81) diluted to 1 × concentration containing 0.06% polyoxyethylene sorbitan monolaurate (Tween-20) (manufactured by Nacalai Tesque, Cat# 28353-85). After rinsing, blocking was carried out by shaking in Blocking One (manufactured by Nacalai Tesque, Cat# 03953-95) or PVDF Blocking Reagent (manufactured by Toyobo, Cat# NYPBR01) under the conditions of 1 hour at room temperature. After blocking, rinsing with TBST was carried out, followed by adding a diluted primary antibody and then shaking overnight under the conditions of 4°C. The primary antibody and the solvent for dilution are as follows.

·RPS25

Antibody: Anti-RPS25 antibody (manufactured by Abeam, Cat# ab102940)
Solvent: Canget signal Solution 1 (manufactured by Toyobo, Cat# NKB-101)

·β-actin

Antibody: β-Actin (13E5) Rabbit mAb (HRP conjugate) (manufactured by Cell Signaling, Cat# 5125)
Solvent: Blocking One

After shaking with the primary antibody, rinsing with TBST was carried out, followed by adding a diluted secondary antibody and then shaking at room temperature for 1 hour. The secondary antibody and the solvent for dilution are as follows.

·RPS25

Antibody: Rabbit IgG (H+L) Cross-Adsorbed Secondary Antibody (manufactured by Invitrogen, Cat# A24537)
Solvent: Canget Signal Solution 2 (manufactured by Toyobo, Cat# NKB-101)

After shaking with the secondary antibody, rinsing with TBST was carried out, followed by detection with the use of ECL prime (manufactured by Amersham, Cat# RPN2232). For the detection and analysis, Amersham Imager 680 was used.

The expression rate of human RPS25 protein in the single-stranded antisense oligonucleotide determined by the above-described method is shown in Table 10. At this time, the expression rate of human RPS25 protein for the negative control group was defined as 1.00. The expression rate of protein was lower than 0.80, and, therefore, it can be judged that this is a single-stranded antisense oligonucleotide capable of modulating the function of RPS25 gene.

[Table 10]

| SEQ ID NO: | Sequence name | Final concentration (μM) | hRPS25 expression rate |
|---|---|---|---|
| 94 | h451-465-A | 0.05 | 0.56 |

«Evaluation of Cytotoxicity of Single-Stranded Antisense Oligonucleotide»

Human cervical cancer cells, HeLa-S3 cells, were cultured in a growth medium under the conditions of 37°C and 5% $CO_2$. The growth medium had the composition as described below.

Composition of Growth Medium Used for Cytotoxicity Evaluation

10% fetal bovine serum (FBS): manufactured by GIBCO, CAT# 10437028
1% non-essential amino acid (NEAA): manufactured by GIBCO, Cat# 11140050
Dulbecco's Modified Eagle Medium Low Glucose (containing L-glutamine, phenol red) (manufactured by FUJIFILM Wako Pure Chemical, Cat# 041-29775)

The day before the experiment, the cells were plated in a 96-well plate ($1.0\times10^4$ cells/well). The plated cells were cultured overnight under the conditions of 37°C and 5% $CO_2$, and then, to Opti-Minimum Essential Medium (manufactured by Thermo Fisher Scientific, Cat# 31985070), the single-stranded antisense oligonucleotide (final concentration, 1 to 200 nM) in the form of a complex with Lipofectamine 3000 (manufactured by Thermo Fisher Scientific, Cat# L3000-015) was added, followed by culturing the cells under the conditions of 37°C and 5% COz for 24 hours. Subsequently, to the growth medium, Caspase-Glo 3/7 Assay System (manufactured by Promega, Cat# G8093) or Celltiter-Glo 2.0 Assay (manufactured by Promega, Cat# G9242) was added to evaluate caspase activity and cell viability. Results of cytotoxicity evaluation of the single-stranded antisense oligonucleotide determined by the above-described method (cell viability) are shown in Table 11-1 to Table 11-3.

[Table 11-1]

| SEQ ID NO: | Sequence name | Cell viability* (n=1) | | | SEQ ID NO: | Sequence name | Cell viability* (n=1) | | |
|---|---|---|---|---|---|---|---|---|---|
| | | 200 nM | 100 nM | 50 nM | | | 200 nM | 100 nM | 50 nM |
| 18 | h36-50-A | 0.65 | 0.70 | 0.90 | 95 | h125-139-B | 1.01 | 1.01 | 0.99 |
| 24 | h102-116-A | 0.74 | 0.85 | 1.00 | 96 | h125-139-C | 1.04 | 1.02 | 1.00 |
| 25 | h103-117-A | 0.80 | 0.88 | 0.96 | 97 | h124-140-A | 1.02 | 1.03 | 1.02 |
| 28 | h125-139-A | 0.96 | 1.00 | 1.00 | 98 | h124-140-B | 1.03 | 1.01 | 0.98 |
| 29 | h126-140-A | 1.02 | 1.06 | 1.04 | 99 | h123-141-A | 0.77 | 0.91 | 1.02 |
| 38 | h187-201-A | 0.92 | 0.99 | 1.00 | 100 | h123-141-B | 0.73 | 0.83 | 1.02 |
| 48 | h213-227-A | 1.02 | 0.99 | 1.02 | 101 | h187-201-B | 1.05 | 1.05 | 1.08 |
| 49 | h214-228-A | 0.99 | 1.02 | 1.06 | 102 | h187-201-C | 0.92 | 1.04 | 0.99 |
| 53 | h220-234-A | 0.99 | 1.05 | 1.08 | 103 | h186-202-A | 0.97 | 1.05 | 1.09 |
| 58 | h259-273-A | 0.86 | 0.93 | 1.06 | 104 | h186-202-B | 1.04 | 0.99 | 1.07 |
| 59 | h260-274-A | 0.94 | 0.98 | 1.01 | 105 | h185-203-A | 1.04 | 0.97 | 1.00 |
| 63 | h295-309-A | 0.88 | 0.84 | 0.99 | 106 | h185-203-B | 0.92 | 0.95 | 1.01 |
| 64 | h296-310-A | 0.91 | 0.92 | 1.03 | 107 | h213-227-B | 1.10 | 1.10 | 1.07 |
| 66 | h325-339-A | 1.01 | 1.02 | 1.06 | 108 | h213-227-C | 1.12 | 1.10 | 1.10 |
| 67 | h326-340-A | 0.95 | 0.95 | 0.95 | 109 | h212-228-A | 1.08 | 1.11 | 1.10 |
| 68 | h327-341-A | 1.05 | 1.02 | 1.03 | 110 | h212-228-B | 1.16 | 1.13 | 1.14 |
| 79 | h430-444-A | 0.96 | 0.96 | 1.01 | 111 | h211-229-A | 1.00 | 1.02 | 1.05 |
| 80 | h431-445-A | 0.91 | 1.00 | 1.02 | 112 | h211-229-B | 1.05 | 1.12 | 1.07 |
| 84 | h435-449-A | 0.83 | 0.91 | 0.95 | 113 | h326-340-B | 1.00 | 1.06 | 1.13 |
| 86 | h438-452-A | 0.87 | 0.93 | 1.06 | 114 | h326-340-C | 0.80 | 0.94 | 1.03 |
| 87 | h439-453-A | 0.99 | 1.06 | 1.04 | 115 | h325-341-A | 0.94 | 0.99 | 1.01 |
| 88 | h440-454-A | 0.96 | 1.04 | 1.05 | 116 | h325-341-B | 0.91 | 1.06 | 1.08 |
| 91 | h444-458-A | 0.68 | 0.91 | 0.89 | 117 | h324-342-A | 1.00 | 1.01 | 1.04 |
| 93 | h447-461-A | 1.01 | 1.00 | 0.93 | 118 | h324-342-B | 1.12 | 1.05 | 1.07 |

(continued)

| SEQ ID NO: | Sequence name | Cell viability* (n=1) | | | SEQ ID NO: | Sequence name | Cell viability* (n=1) | | |
|---|---|---|---|---|---|---|---|---|---|
| | | 200 nM | 100 nM | 50 nM | | | 200 nM | 100 nM | 50 nM |
| 94 | h451-465-A | 1.00 | 0.99 | 1.03 | 119 | h444-458-B | 0.65 | 0.95 | 1.02 |
| * ratio of transfection control | | | | | | | | | |

[Table 11-2]

| SEQ ID NO: | Sequence name | Cell viability* (n=1) | | | SEQ ID NO: | Sequence name | Cell viability* (n=1) | | |
|---|---|---|---|---|---|---|---|---|---|
| | | 200 nM | 100 nM | 50 nM | | | 200 nM | 100 nM | 50 nM |
| 120 | h444-458-C | 0.54 | 0.94 | 1.08 | 145 | h185-199-C | 0.84 | 0.96 | 0.91 |
| 121 | h442-458-A | 0.97 | 1.07 | 1.10 | 146 | h186-200-C | 0.91 | 0.86 | 0.92 |
| 122 | h442-458-B | 0.95 | 0.86 | 1.12 | 147 | h190-204-A | 0.84 | 0.83 | 0.82 |
| 123 | h442-460-A | 0.87 | 0.94 | 0.89 | 148 | h211-225-A | 1.02 | 0.85 | 0.84 |
| 124 | h442-460-B | 0.94 | 0.90 | 0.96 | 149 | h212-226-A | 0.93 | 0.91 | 0.83 |
| 125 | h451-465-B | 0.99 | 0.99 | 1.01 | 150 | h215-229-A | 0.87 | 0.81 | 0.79 |
| 126 | h451-465-C | 0.99 | 1.02 | 1.11 | 151 | h218-232-A | 0.81 | 0.82 | 0.76 |
| 127 | h450-466-A | 0.81 | 0.86 | 1.04 | 152 | h221-235-A | 0.66 | 0.72 | 0.76 |
| 128 | h450-466-B | 0.90 | 0.83 | 1.01 | 153 | h222-236-A | 0.85 | 0.89 | 0.87 |
| 129 | h449-467-A | 0.94 | 0.88 | 1.03 | 154 | h223-237-A | 0.94 | 0.90 | 0.95 |
| 130 | h449-467-B | 0.82 | 0.85 | 0.96 | 155 | h224-238-A | 0.84 | 0.76 | 0.85 |
| 131 | h39-53-A | 0.89 | 0.83 | 0.95 | 156 | h255-269-A | 0.75 | 0.81 | 0.83 |
| 132 | h40-54-A | 0.80 | 0.79 | 0.94 | 157 | h256-270-A | 0.61 | 0.65 | 0.71 |
| 133 | h98-112-A | 0.82 | 0.81 | 0.93 | 158 | h257-271-A | 0.63 | 0.59 | 0.62 |
| 134 | h99-113-A | 0.94 | 0.88 | 0.91 | 159 | h258-272-A | 0.66 | 0.63 | 0.64 |
| 135 | h100-114-A | 0.90 | 0.84 | 0.88 | 160 | h262-276-A | 0.61 | 0.60 | 0.75 |
| 136 | h104-118-A | 0.68 | 0.66 | 0.76 | 161 | h263-277-A | 0.79 | 0.81 | 0.90 |
| 137 | h105-119-A | 0.94 | 0.89 | 0.98 | 162 | h264-278-A | 0.84 | 0.82 | 0.92 |
| 138 | h106-120-A | 0.92 | 0.91 | 0.93 | 163 | h291-305-A | 0.93 | 0.98 | 1.01 |
| 139 | h107-121-A | 0.94 | 0.95 | 0.92 | 164 | h292-306-A | 0.88 | 0.92 | 0.99 |
| 140 | h123-137-C | 0.68 | 0.80 | 0.86 | 165 | h293-307-A | 0.81 | 0.93 | 1.00 |
| 141 | h127-141-A | 0.89 | 0.94 | 0.85 | 166 | h294-308-A | 0.74 | 0.84 | 0.94 |
| 142 | h128-142-A | 0.90 | 0.92 | 0.80 | 167 | h298-312-A | 0.92 | 1.01 | 1.01 |
| 143 | h129-143-A | 0.77 | 0.83 | 0.76 | 168 | h299-313-A | 0.92 | 0.94 | 1.01 |
| 144 | h130-144-A | 0.80 | 0.87 | 0.82 | 169 | h300-314-A | 0.87 | 0.95 | 0.99 |
| * ratio of transfection control | | | | | | | | | |

[Table 11-3]

| SEQ ID NO: | Sequence name | Cell viability* (n=1) | | | SEQ ID NO: | Sequence name | Cell viability* (n=1) | | |
|---|---|---|---|---|---|---|---|---|---|
| | | 200 nM | 100 nM | 50 nM | | | 200 nM | 100 nM | 50 nM |
| 170 | h321-335-A | 1.00 | 1.02 | 1.05 | 183 | h443-457-A | 0.92 | 0.94 | 0.95 |
| 171 | h322-336-A | 0.93 | 0.96 | 1.03 | 184 | h445-459-A | 0.89 | 0.87 | 1.00 |
| 172 | h323-337-A | 0.97 | 1.00 | 0.99 | 185 | h448-462-A | 0.73 | 0.79 | 0.93 |
| 173 | h324-338-A | 1.03 | 0.95 | 1.04 | 186 | h449-463-A | 0.96 | 0.93 | 0.97 |
| 174 | h328-342-A | 1.01 | 1.02 | 1.00 | 187 | h450-464-A | 0.87 | 0.96 | 0.89 |
| 175 | h329-343-A | 1.03 | 1.06 | 1.01 | 188 | h452-466-A | 0.82 | 0.82 | 0.88 |
| 176 | h330-344-A | 1.06 | 1.06 | 0.99 | 189 | h453-467-A | 0.86 | 0.90 | 0.90 |
| 177 | h331-345-A | 1.02 | 1.04 | 1.03 | 190 | h454-468-A | 0.82 | 0.90 | 0.87 |
| 178 | h426-440-A | 1.07 | 1.06 | 1.06 | 191 | h455-469-A | 0.91 | 0.95 | 0.92 |
| 179 | h427-441-A | 1.00 | 0.96 | 0.99 | 300 | hp2587-2601-A | 1.03 | 1.02 | 0.99 |
| 180 | h428-442-A | 1.07 | 0.99 | 1.01 | 301 | hp2583-2597-A | 0.93 | 0.94 | 1.01 |
| 181 | h429-443-A | 1.07 | 1.03 | 1.00 | 302 | hp2584-2598-A | 0.98 | 0.94 | 0.93 |
| 182 | h437-451-A | 0.82 | 0.84 | 0.96 | | | | | |
| * ratio of transfection control | | | | | | | | | |

«Evaluation of Serum Stability of Single-Stranded Antisense Oligonucleotide»

To Tris-EDTA buffer (pH=8.0) solution (4 μL) containing 400 pmol of the single-stranded antisense oligonucleotide, mouse serum (20 μL) or human serum (20 μL) was mixed, followed by addition of mineral oil (15 μL). The resulting solution was incubated at 37°C, and, then, an 8-mol/L urea solution (10 μL) was mixed thereto for inactivating nuclease in the serum. After ultrapure water (10 μL) was added, centrifugation was carried out to separate the resultant into an aqueous layer containing the single-stranded antisense oligonucleotide and a mineral oil layer, and the aqueous layer was subjected to analysis by LC-MS (manufactured by Waters), where, from the integrated intensity of the UV-chromatogram for the obtained single-stranded antisense oligonucleotide, the residual single-stranded antisense oligonucleotide was calculated. "Residual oligonucleotide (%)" refers to the rate, relative to the non-degraded single-stranded antisense oligonucleotide at the time of analysis which was carried out immediately after mixing with the serum, of the residual non-degraded single-stranded antisense oligonucleotide remaining 72 hours later.

One with a residual rate of 50% or more after 72 hours is judged to be a stable single-stranded antisense oligonucleotide.

«Evaluation of In Vivo RPS25 Gene Expression»

Evaluation of RPS25 gene expression was carried out by performing intraventricular administration for mice

and measuring the amount of mRNA in different parts of the prefrontal cortex. Evaluation of gene expression according to the present example means measuring the amount of complementary DNA (cDNA) obtained from reverse transcription reaction to assess the amount of mRNA. In the following, the specific procedure of the expression evaluation will be described.

FVB mice (CLEA Japan) were anesthetized with isoflurane (manufactured by Pfizer, Cat# 114133403). Then, the FVB mice under anesthesia were administered with the antisense oligonucleotide dissolved in artificial cerebrospinal fluid (manufactured by Tocris Bioscience, Cat# 3525 /25 mL) (10 μL/individual) with the use of a double needle (manufactured by TOP, medical instrument approval number 15800BZZ01460000) inserted in a 50-μL Hamilton syringe (manufactured by Hamilton, Cat# 705LT). To the mice in the negative control group, artificial cerebrospinal fluid alone was administered (10 μL/individual).

After a certain period of time following the administration, the FVB mice were euthanized and three parts, namely the brain, the cervical cord, and the lumbar cord, were sampled. The sampled tissue was immersed in RNA later (manufactured by Applied Biosystems, Cat# AM7024) and left to stand overnight, followed by stored at - 80°C. From the tissue sample thus stored, RNA was extracted with the use of RNeasy Mini Kit (manufactured by QIAGEN, Cat# 74106). The mRNA thus extracted was subjected to reverse transcription reaction with the use of High-Capacity cDNA Reverse Transcription Kit (manufactured by Applied Biosystem, Cat# 4368814). For the reverse transcription reaction, 1 μg of mRNA diluted to 20 μL was used. With the use of the complementary DNA (cDNA) obtained from the reverse transcription reaction, Taqman expression assays (manufactured by Applied Biosystems), and a gene-specific probe designed in advance (see below), real-time PCR was carried out (40 cycles of 95°C for 3 seconds and 60°C for 30 seconds).

List of Gene-Specific Probes Used in Evaluation of Mouse RPS25 Gene Expression

Mouse Rps25: Mm02342783_g1
Mouse GAPDH: 4352339E (internal control)

The expression rate of mouse RPS25 mRNA in the single-stranded antisense oligonucleotide as determined by the above-described method is shown in Table 12. At this time, the expression rate of mouse RPS25 RNA for the negative control group was defined as 1.00. Generally, it is expected that when mRNA expression is reduced, subsequent protein translation and the like are also reduced; hence, it can be judged that one with an expression rate of 0.80 or less is a single-stranded antisense oligonucleotide capable of modulating the function of mouse RPS25 gene. The target region to which the single-stranded antisense oligonucleotide listed in Table 12 binds is a region whose sequence is conserved between human RPS25 gene and mouse RPS25 gene.

[Table 12]

| SEQ ID NO: | Sequence name | Dose | Sampling site | Post-administration days | mRPS25 expression rate |
|---|---|---|---|---|---|
| 114 | h326-340-C | 100 μg | Prefrontal cortex | 3 days | 0.78 |
| 116 | h325-341-B | 100 μg | Prefrontal cortex | 3 days | 0.66 |
| 126 | h451-465-C | 100 μg | Prefrontal cortex | 3 days | 0.69 |
| 128 | h450-466-B | 100 μg | Prefrontal cortex | 3 days | 0.76 |
| 130 | h449-467-B | 100 μg | Prefrontal cortex | 3 days | 0.62 |
| 362 | h451-465-E | 100 μg | Prefrontal cortex | 3 days | 0.75 |
| 374 | h450-465-C | 100 μg | Prefrontal cortex | 3 days | 0.67 |

<<Evaluation of RAN Translation-Reducing Action in Neurons Expressing CAG Repeat>>

Into pan-neurons induced differentiation from healthy iPS cells, a lentiviral vector harboring a CAG102 repeat was introduced, and thereby CAG-repeat-expressing neurons were prepared. Conditions were designed so as to allow for detection of RAN translation product with the use of the CAG-repeat-expressing neurons thus prepared, and the amount of RAN translation product in the CAG-repeat-expressing neurons at the time of treatment with the antisense oligonucleotide was measured for evaluating RAN translation-reducing activity. In the following, the procedure of the evaluation is described.

The composition of the medium used is described below.

(StemFit AK03N): manufactured by Ajinomoto
(Neural Induction Medium)
Neurobasal Medium (manufactured by ThermoFisher Scientific, Cat# 21103-49)
Neural Induction Supplement (manufactured by ThermoFisher Scientific, Cat# A1647801): 1/50 amount

(Neural Expansion Medium)

Neurobasal Medium (manufactured by ThermoFisher Scientific, Cat# 21103-49)
Advanced DMEM (manufactured by ThermoFisher Scientific, Cat# 12634)
Neural Induction Supplement (manufactured by ThermoFisher Scientific, Cat# A1647801), 1/50 amount

(NB Medium)

Neurobasal Medium (manufactured by ThermoFisher Scientific, Cat# 21103049)
1/50 SM1 Neural Supplement (manufactured by StemCell, Cat# 05711)
1/100 N2 Neural Supplement (manufactured by StemCell, Cat# 07152)
10 ng/mL Human BDNF (manufactured by Peprotech, Cat# 450-02)
10 ng/mL Human GDNF (manufactured by R&D Systems, cat# 212-GD-050)
100 μM Ascorbic Acid (manufactured by Tokyo Chemical Industry, Cat# A0537)
100 μM N6,2'-0-Dibutyryladenosine-3',5'-cyclic Monophosphate Sodium Salt (manufactured by Nacalai, Cat# 11540-61)
100-Fold diluted penicillin-streptomycin mixed solution (manufactured by ThermoFisher Scientific, cat# 15140-122)
0.1 μM Compound E (manufactured by Calbiochem, Cat# 56790)

(BP Medium)

BrainPhys Neuronal Medium (manufactured by StemCell, Cat# 05790)
1/50 SM1 Neural Supplement (manufactured by StemCell, Cat# 05711)
1/100 N2 Neural Supplement (manufactured by StemCell, Cat# 07152)
10 ng/mL Human BDNF (manufactured by Peprotech, Cat# 450-02)
10 ng/mL Human GDNF (manufactured by R&D Systems, cat# 212-GD-050)
100 μM Ascorbic Acid (manufactured by Tokyo Chemical Industry, Cat# A0537)
100 μM N6,2'-O-Dibutyryladenosine-3',5'-cyclic Monophosphate Sodium Salt (manufactured by Nacalai, Cat# 11540-61)
100-Fold diluted penicillin-streptomycin mixed solution (manufactured by ThermoFisher Scientific, cat# 15140-122)
0.1 μM Compound E (manufactured by Calbiochem, Cat# 56790)

<Maintaining Human iPS Cells>

iMAtrix-511 (manufactured by Nippi, Cat# 892012) diluted 100 folds with PBS was added to a 6-well plate to coat the plate. In the plate, iPS cells (strain 201B7, obtained from iPS Academia Japan, AJ-H1-01) thawed with the use of StemFitAK-03N medium (manufactured by Ajinomoto) containing 10 μM Y-27632 (manufactured by Tocris, Cat# 1254) were plated at 200,000 cells/plate. The day after the plating, the medium was changed to Y-27632-free StemFitAK-03N, and, after this, every 2 to 3 days, the medium was changed to Y-27632-free AK-03 medium. The cell culture was passaged once a week. The passage was carried out by the procedure described below. Firstly, to the plate from which the medium was removed, Accutase (manufactured by Funakoshi, Cat# AT104) was added at 350 μL/well, followed by treatment at 37°C for 5 minutes, to peel the cells off. Then, the cells were dispersed with the use of PBS, followed by counting the cells and subsequently plating the cells at 5000 to 15000 cells/well in StemFitAK03N medium containing 10 μM Y-27632 and 11150 amount of iMAtrix.

<Induced Differentiation from Human iPS Cells into Pan-Neurons>

iPS cells were passaged in the manner described above, and plated in a 6-well plate at 300000 cells/well. The day after plating, 15 to 25% confluency was observed, and the medium was changed to PSC Neural Induction Medium. Every 2 days, the medium was changed to PSC Neural Induction Medium. Seven days after the first medium change to PSC Neural Induction Medium, the cells were passaged. Specifically, firstly, Geltrex (trademark) hESC-Qualified, Ready-To-Use, Reduced Growth Factor Basement Membrane Matrix was added to a 10-cm petri dish, followed by leaving to stand at 37°C for at least 1 hour for coating. The medium was removed from the cultured cells, followed by rinsing with PBS, and then adding Accutase for peeling the cells off. The cells thus peeled off were collected through a cell strainer (manufactured by Falcon, Cat# 352360), followed by centrifugation in himac CF7D2 manufactured by Hitachi at 900 rpm for 4 minutes. After resuspended in PBS, the cells were counted, and a necessary number of the cells were taken for another round of centrifugation at 900 rpm for 4 minutes. To the precipitated cells, Neural Expansion Medium containing 5 μM Y-27632 was added for resuspension, and then the cells were plated at $3\times10^6$ to $6\times10^6$ cells/dish. The day after plating, the medium was changed to Y-27632-free Neural Expansion Medium, followed by continued culturing of the cells. Cell stocks were prepared from the second-passaged cells or later. Specifically, the cells were peeled off by the same procedure as for passaging, and the cells were suspended at $1\times10^7$ cells/mL with the use of Bambanker (manufactured by Nippon Genetics, Cat# CS-04-001), followed by freezing them.

<Culture for Maintaining Pan-Neurons>

Frozen pan-neuron stock (frozen stock) was thawed. Specifically, firstly, a 6-well plate was coated with geltrex at 37°C for at least 1 hour. Then, to the frozen stock, Neural Expansion Medium (containing 5 μM Y-27632) warmed to 37°C was added for melting the cells, followed by plating all the cells in one well. Next day, the medium was changed to Y-27632-free Neural Expansion Medium. Three days after the cells were melted, the medium was removed, followed by rinsing with PBS, adding Accutase, and leaving to stand at room temperature for 1 minute. Accutase was removed and the cells were peeled off with the use of PBS, followed by cell counting. A necessary number of the cells were taken, centrifuged at 300×g for 4 minutes for supernatant removal, and then resuspended at $1\times10^5$ cells/mL in NB medium containing 5 μM Y-27632. After the suspension, the cells were plated in a 96-well U-shaped plate (manufactured by ThermoFisher Scientific, Cat# 174929) at 100 μL/well, and then centrifuged at 1000 rpm for 4 minutes, followed by culturing under the conditions of 37°C and 5% $CO_2$. At the same time as the cell plating, a lentivirus solution containing CAG102 repeat (5 μL) and a solution of the antisense oligonucleotide (final concentration, 1 μM) were added. Two days after the cell plating, Y-27632-free BP medium was added at 100 μL/well, followed by changing half the amount of the medium with Y-27632-free BP medium every 2 to 3 days. On Culture Day 7 and 14, the antisense oligonucleotide was added in a final concentration of 1 μM.

<Method for Preparing Lentivirus>

Between NheI-SwaI of pCDH-EF1-MCS plasmid vector (manufactured by System Biosciences, Cat# CD502A-1-SBI), (CAG)x120 repeat-containing HTT gene partial sequence + 3x epitope tag (Myc, Flag, V5) (Table 13, SEQ ID NO: 788) was inserted into. This plasmid vector, together with a lentivirus packaging plasmid vector (manufactured by Invitrogen, Cat# ViraPower Packaging mix K497500), was transfected into HEK293T cells (manufactured by Takara Bio, Cat# TransIT-293 V2700). The conditions for introducing the plasmid vector were as described in the manual provided from the manufacturer. The transfected cells were cultured for 3 days, and then the culture supernatant was collected. The lentivirus in the culture supernatant was concentrated with the use of PEG-it (System Biosciences LV825A-1), and suspended in PBS. The lentivirus thus prepared was infected to HEK293T, followed by immunostaining to check the production of the desired molecule.

[Table 13]

| Mvc, Flag, V5 base sequence |
|---|
| GCTAGCCACCATGGCGACCCTGGAAAAGaTGATGAAGGCCTTCGAGTCCCTCAAGTCCTTCC<br>AGCAGCAGCAGCAGCAGCAGCAGCAGCAGCAGCAGCAGCAGCAGCAGCAGCAGCAGCAGCAG<br>CAGCAGCAGCAGCAGCAGCAGCAGCAGCAGCAGCAGCAGCAGCAGCAGCAGCAGCAGCAGCA<br>GCAGCAGCAGCAGCAGCAGCAGCAGCAGCAGCAGCAGCAGCAGCAGCAGCAGCAGCAGCAGC<br>AGCAGCAGCAGCAGCAGCAGCAGCAGCAGCAGCAGCAGCAGCAGCAGCAGCAGCAGCAGCAG<br>CAGCAGCAGCAGCAGCAGCAGCAGCAGCAGCAGCAGCAGCAGCAGCAGCAGCAGCAGCAGCA<br>GCAGCAGCAGCAGCAGCAGCAGCAGCAGCAGCAGCAGCAGCAGCAGCAGCAGCAGCAGCAGC<br>AGCAGCCGCCACCGCCGCCGCCGCCGCCGCCTCCTCAGCTTCCTCAGCCGCCGCCGCA<br>GGCACAGCCGCTGCTGCCTCAGCCGCAGCCGCCCCCGCCGCCGCCCCCGCCGCCACCCGGC<br>CCGGCTGTGGCTGTGGAGCCGCAAGAATTCGGAGGAGATATCGAACAAAAACTCATCTCAG<br>AAGAGGATCTGTGACTACAAAGACGACGACGACAAGCGGTAAGCCTATCCCTAACCCTCTC<br>CTCGGTCTCGATTCTACGTAGCTCGAGTCTAGAGGGCCCGTTT |

<Immunostaining and Observation>

On Cell Culture Day 23, the cells were fixed with 4% paraformaldehyde (left to stand at room temperature for 15 minutes) for immunostaining. After the fixation, the cells were rinsed with PBS (left to stand for 5 minutes after addition, repeated 3 times), followed by adding a blocking buffer (3% BSA [manufactured by Sigma, Cat# A9576-50ML], 0.3% Triton X-100 [manufactured by Nacalai Tesque, Cat# 35501-02] in PBS) and leaving the cells to stand at room temperature for 1 hour for blocking. After the blocking, the following primary antibody diluted with the blocking buffer was added to the cells, followed by leaving them to stand overnight at 4°C.

·Anti-cMyc antibody (manufactured by Abcom, Cat# ab9106), 1190-fold diluted

·Anti-V5 antibody (manufactured by ThermoFisher Scientific, Cat# R960-25), 1000-fold diluted

Next day, the cells were rinsed with PBS-T (3% BSA, 0.3% TritonX-100 in PBS) (left to stand for 5 minutes after addition, repeated 3 times), and the following secondary antibody diluted with the blocking buffer was added, followed by leaving them to stand overnight at 4°C.

·Donkey anti-Mouse IgG (H+L) Highly Cross-Adsorbed Secondary Antibody, Alexa Fluor Plus (manufactured by Invitrogen, Cat# A32766), 1000-fold diluted
·Donkey anti-Rabbit IgG (H+L) Highly Cross-Adsorbed Secondary Antibody, Alexa Fluor 594 (manufactured by Invitrogen, Cat# A32766), 1000-fold diluted
·Hoechst 33342 solution (1 mg/mL) (WAKO code: 346-07951)

Next day, the cells were rinsed with PBS-T (3% BSA, 0.3% TritonX-100 in PBS) (left to stand for 5 minutes after addition, repeated 3 times), and the cells examined with the use of a confocal quantification imaging cytometer CellVoyager CQ1 (manufactured by Yokogawa Electric). Peptides produced by ordinary translation (detected with Myc tag) were compared between the antisense oligonucleotide treated cell group and the untreated cell group, but there was no difference in the amount of peptide production. On the other hand, the peptides produced by RAN translation (detected with V5 tag) specifically decreased in the antisense oligonucleotide treated cell group, as compared to the untreated cell group.

The embodiment and Examples of the present invention are described above, and the configurations of the above embodiment and Examples may be combined as appropriate.

The embodiment and Examples disclosed herein are illustrative and non-restrictive in any respect. The scope of the present invention is defined by the terms of the claims, not by the embodiment and Examples, and intended to encompass all modifications and variations equivalent in meaning and scope to the claims.

[Sequence Listing]

## Claims

1. A single-stranded antisense oligonucleotide, or a pharmaceutically acceptable salt thereof, capable of modulating expression and/or function of RPS25 gene, wherein

   nucleotides of the single-stranded antisense oligonucleotide are bonded to each other via a phosphate group and/or a modified phosphate group,
   the single-stranded antisense oligonucleotide includes a gap region, a 3' wing region bonded to a 3' end of the gap region, and a 5' wing region bonded to a 5' end of the gap region,
   the gap region is a deoxyribose-based nucleic acid optionally including a nucleic acid having a modified sugar moiety,
   each of the 3' wing region and the 5' wing region is a modified nucleic acid,
   the single-stranded antisense oligonucleotide has a base length of 12- to 30-mer, and
   a base sequence of the single-stranded antisense oligonucleotide is:

      a base sequence with a sequence identity of 90% to 100% to a base sequence complementary to at least one target region of the same base length as the single-stranded antisense oligonucleotide present in a base sequence as set forth in SEQ ID NO: 1 or SEQ ID NO: 2;
      a base sequence complementary to a base sequence of the target region with deletion, substitution, insertion, or addition of one or several bases; or
      a base sequence capable of hybridizing under stringent conditions with an oligonucleotide having the target region.

2. The single-stranded antisense oligonucleotide or a pharmaceutically acceptable salt thereof according to claim 1, wherein the base sequence of the single-stranded antisense oligonucleotide is:
   a base sequence with a sequence identity of 95% to 100% to a base sequence complementary to at least one target region of the same base length as the single-stranded antisense oligonucleotide present in the base sequence as set forth in SEQ ID NO: 1 or SEQ ID NO: 2.

3. The single-stranded antisense oligonucleotide or a pharmaceutically acceptable salt thereof according to claim 1, wherein the base sequence of the single-stranded antisense oligonucleotide is:
   a base sequence complementary to at least one target region of the same base length as the single-stranded antisense oligonucleotide present in the base sequence as set forth in SEQ ID NO: 1 or SEQ ID NO: 2.

4. The single-stranded antisense oligonucleotide or a pharmaceutically acceptable salt thereof according to any one of claims 1 to 3, wherein

   the gap region has a base count of 5- to 20-mer,
   the 3' wing region is a 1- to 5-mer modified nucleic acid, and
   the 5' wing region is a 1- to 5-mer modified nucleic acid.

5. The single-stranded antisense oligonucleotide or a pharmaceutically acceptable salt thereof according to any one of claims 1 to 4, wherein the single-stranded antisense oligonucleotide has a base length of 14- to 22-mer.

6. The single-stranded antisense oligonucleotide or a pharmaceutically acceptable salt thereof according to any one of claims 1 to 5, wherein

   the modified nucleic acid of the 3' wing region includes at least one selected from the group consisting of 2'-MOE nucleic acid, LNA, AmNA, GuNA, and scpBNA, and
   the modified nucleic acid of the 5' wing region includes at least one selected from the group consisting of 2'-MOE nucleic acid, LNA, AmNA, GuNA, and scpBNA.

7. The single-stranded antisense oligonucleotide or a pharmaceutically acceptable salt thereof according to any one of claims 1 to 6, wherein at least one bond between nucleotides of the single-stranded antisense oligonucleotide is

a phosphorothioate bond.

8. The single-stranded antisense oligonucleotide or a pharmaceutically acceptable salt thereof according to any one of claims 1 to 7, wherein at least one bond between nucleotides of the single-stranded antisense oligonucleotide is a phosphodiester bond.

9. The single-stranded antisense oligonucleotide or a pharmaceutically acceptable salt thereof according to any one of claims 1 to 8, wherein
the base sequence of the single-stranded antisense oligonucleotide is:

a base sequence with a sequence identity of 90% to 100% to a base sequence complementary to a target region of 14- to 22-mer present in the base sequence as set forth in SEQ ID NO: 1 following a base located at one of position 8 to position 10, position 27 to position 29, position 34 to position 40, position 79, position 98, position 101 to position 106, position 123 to position 129, position 140, position 160 to position 161, position 180 to position 191, position 208 to position 221, position 242 to position 243, position 255 to position 268, position 285 to position 286, position 292 to position 304, position 321 to position 328, position 340 to position 344, position 365, and position 429 to position 454 counted from a 5' end of the base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region.

10. The single-stranded antisense oligonucleotide or a pharmaceutically acceptable salt thereof according to any one of claims 1 to 9, wherein

the base sequence of the single-stranded antisense oligonucleotide is a base sequence with a sequence identity of 90% to 100% to a base sequence complementary to a target region of 14- to 22-mer present in the base sequence as set forth in SEQ ID NO: 1 following a base located at one of position 8, position 10, position 28 to position 29, position 35 to position 37, position 101 to position 104, position 123 to position 126, position 129, position 160, position 180 to position 187, position 209 to position 220, position 258 to position 267, position 285, position 295 to position 297, position 300 to position 304, position 321 to position 327, position 341, position 344, position 365, and position 429 to position 454 counted from a 5' end of the base sequence as set forth in SEQ ID NO: 1,
the 3' wing region is a 2- to 5-mer, and
the 5' wing region is a 2- to 5-mer.

11. The single-stranded antisense oligonucleotide or a pharmaceutically acceptable salt thereof according to any one of claims 1 to 10, wherein the base sequence of the single-stranded antisense oligonucleotide is a base sequence with a sequence identity of 90% to 100% to a base sequence complementary to a target region of 14- to 22-mer present in the base sequence as set forth in SEQ ID NO: 1 following a base located at one of position 36, position 102 to position 103, position 123 to position 126, position 185 to position 187, position 213 to position 214, position 220, position 259 to position 260, position 263 to position 265, position 295 to position 296, position 300, position 302 to position 303, position 322 to position 327, position 429 to position 431, position 435, and position 438 to position 454 counted from a 5' end of the base sequence as set forth in SEQ ID NO: 1.

12. The single-stranded antisense oligonucleotide or a pharmaceutically acceptable salt thereof according to any one of claims 1 to 11, wherein the base sequence of the single-stranded antisense oligonucleotide is a base sequence selected from the group consisting of base sequences as set forth in SEQ ID NOs: 18, 24 to 25, 28 to 29, 38, 48 to 49, 53, 58 to 59, 63 to 64, 66 to 68, 79 to 80, 84, 86 to 91, 93 to 95, 97, 99 to 105, 113 to 119, 121 to 123, 125, 127 to 130, 140, 162, 169, 171 to 173, 183, 188, 190, 304 to 306, 309, 310, 312, 313, 317, 321 to 323, 326, 327, 331, 332, 334, 337, 340 to 344, 346, 348, 349, 351, 353, 355 to 364, 366, 367, 371 to 382, 385, 386, 388, 389, 391, 394, 396, 397, 407, 408, 410, 418 to 424, 426, 427, 431, and 432.

13. The single-stranded antisense oligonucleotide or a pharmaceutically acceptable salt thereof according to any one of claims 1 to 8, wherein
the base sequence of the single-stranded antisense oligonucleotide is:

a base sequence with a sequence identity of 90% to 100% to a base sequence complementary to a target region of 14- to 22-mer present in the base sequence as set forth in SEQ ID NO: 2 following a base located at one of position 1, position 75, position 233, position 261, position 278 to position 280, position 390 to position 392, position 417 to position 423, position 445 to position 447, position 460 to position 461, position 510, position 561 to position 562, position 589, position 605, position 626 to position 628, position 632 to position 634, position 696 to position 697, position 1034 to position 1035, position 1103 to position 1107, position 1128 to position 1129, position 1196 to position 1197, position 1398, position 1408 to position 1412, position 1478 to position 1480, position 1715, position 1749 to position 1751, position 2047 to position 2049, position 2121 to position 2123, position 2260 to position 2268, position 2342, position 2406, and position 2585 to position 2587 counted from a 5' end of the base sequence as set forth in SEQ ID NO: 2;
a base sequence complementary to a base sequence of the target region with deletion, substitution, insertion, or addition of one or several bases; or
a base sequence capable of hybridizing under stringent conditions with an oligonucleotide having the target region.

**14.** The single-stranded antisense oligonucleotide or a pharmaceutically acceptable salt thereof according to any one of claims 1 to 8 and 13, wherein

the base sequence of the single-stranded antisense oligonucleotide is a base sequence with a sequence identity of 90% to 100% to a base sequence complementary to a target region of 14- to 22-mer present in the base sequence as set forth in SEQ ID NO: 2 following a base located at one of position 1, position 278 to position 279, position 417 to position 420, position 561, position 605, position 627, position 632 to position 634, position 697, position 1035, position 1128, position 1196 to position 1197, position 1409 to position 1410, position 1478, position 1715, position 1750, position 2047 to position 2049, position 2342, position 2406, and position 2585 to position 2587 counted from a 5' end of the base sequence as set forth in SEQ ID NO: 2,
the 3' wing region is a 2- to 5-mer, and
the 5' wing region is a 2- to 5-mer.

**15.** A double-stranded antisense oligonucleotide or a pharmaceutically acceptable salt thereof comprising:

the single-stranded antisense oligonucleotide according to any one of claims 1 to 14; and
a second oligonucleotide hybridized to the single-stranded antisense oligonucleotide, wherein
a base sequence of the second oligonucleotide is a base sequence with a sequence identity of 90% to 100% to a base sequence complementary to the base sequence of the single-stranded antisense oligonucleotide.

**16.** An antisense oligonucleotide complex or a pharmaceutically acceptable salt thereof comprising:

the single-stranded antisense oligonucleotide or a pharmaceutically acceptable salt thereof according to any one of claims 1 to 14, or the double-stranded antisense oligonucleotide or a pharmaceutically acceptable salt thereof according to claim 15; and
an additional substance bonded to the single-stranded antisense oligonucleotide or to the second oligonucleotide, wherein
the additional substance is selected from the group consisting of polyethylene glycol, peptide, alkyl chain, ligand compound, antibody, protein, and sugar chain.

**17.** A pharmaceutical comprising the single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, as an active ingredient.

**18.** An agent for modulating expression and/or function of RPS25 gene, comprising the single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, as an active ingredient.

**19.** An agent for inhibiting dipeptide repeat production attributable to RAN translation, comprising the single-stranded antisense oligonucleotide or a pharmaceutically acceptable salt thereof according to any one of claims 1 to 14, the

double-stranded antisense oligonucleotide or a pharmaceutically acceptable salt thereof according to claim 15, or the antisense oligonucleotide complex or a pharmaceutically acceptable salt thereof according to claim 16, as an active ingredient.

**20.** An agent for treating a repeat disease, comprising the single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, as an active ingredient.

**21.** An agent for preventing a repeat disease, comprising the single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, as an active ingredient.

**22.** The agent for treating a repeat disease according to claim 20, or the agent for preventing a repeat disease according to claim 21, wherein the repeat disease is at least one selected from the group consisting of C9orf12 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

**23.** A method of treating or preventing a repeat disease, comprising administering the single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, to an individual.

**24.** The method of treating or preventing a repeat disease according to claim 23, wherein the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

**25.** The single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, for use for treating or preventing C9orf72 ALS.

**26.** The single-stranded antisense oligonucleotide or a pharmaceutically acceptable salt thereof according to any one of claims 1 to 14, the double-stranded antisense oligonucleotide or a pharmaceutically acceptable salt thereof according to claim 15, or the antisense oligonucleotide complex or a pharmaceutically acceptable salt thereof according to claim 16, for use for producing an agent for treating or preventing C9orf72 ALS.

FIG.1

STRUCTURE OF GAPMER-TYPE ASO

(EXAMPLE) 3-12-3 GAPMER

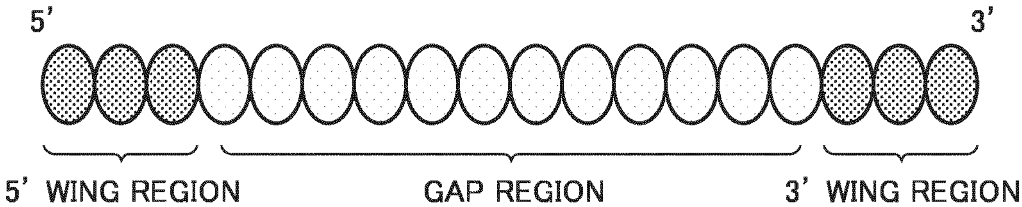

FIG.2

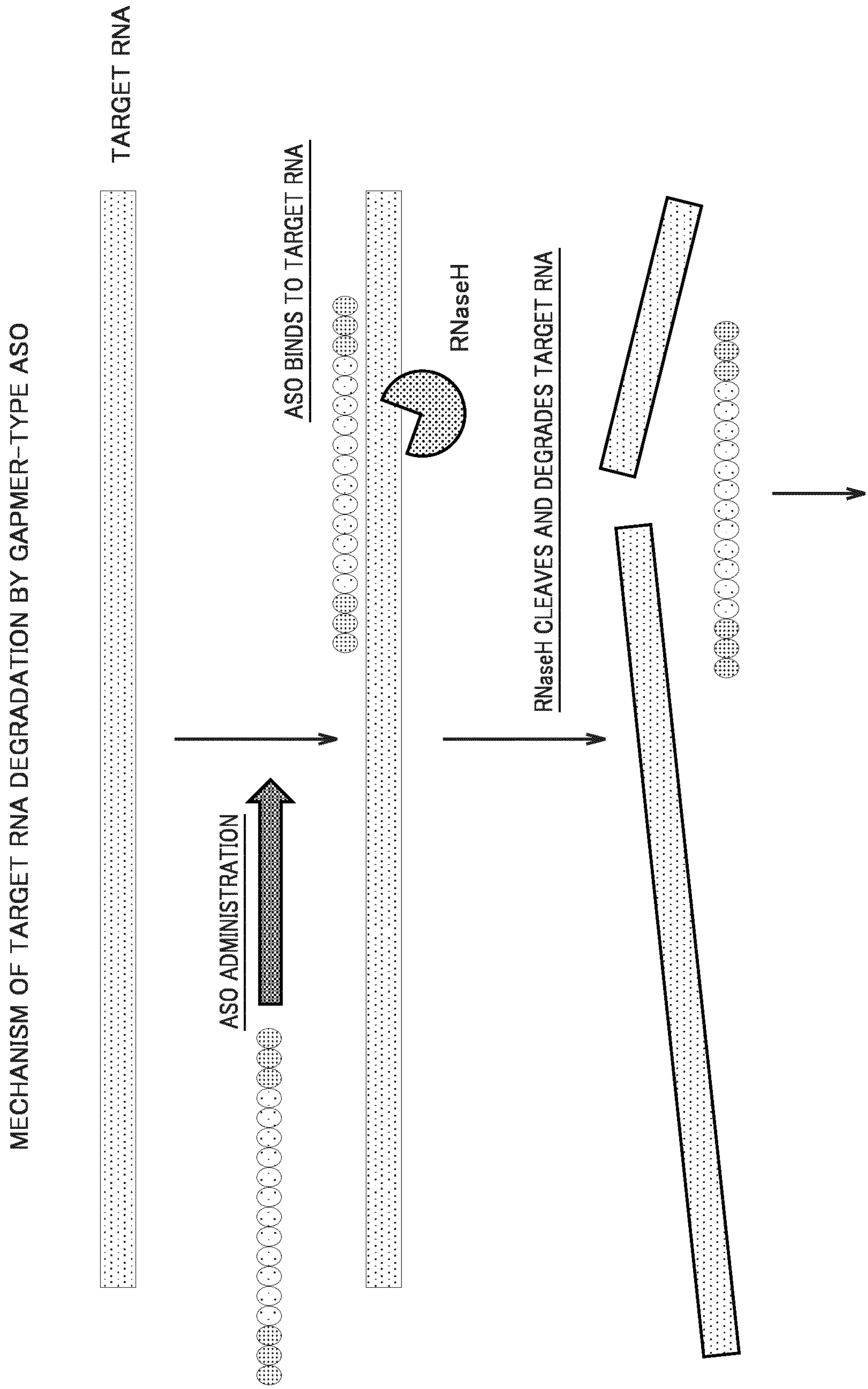
MECHANISM OF TARGET RNA DEGRADATION BY GAPMER-TYPE ASO
TARGET RNA
ASO ADMINISTRATION
ASO BINDS TO TARGET RNA
RNaseH
RNaseH CLEAVES AND DEGRADES TARGET RNA

FIG.3

H451-465-I

THEORETICAL MOLECULAR WEIGHT : 5752.8

STRUCTURAL FORMULA: $C_{199}H_{263}N_{60}O_{92}P_{15}S_{10}$

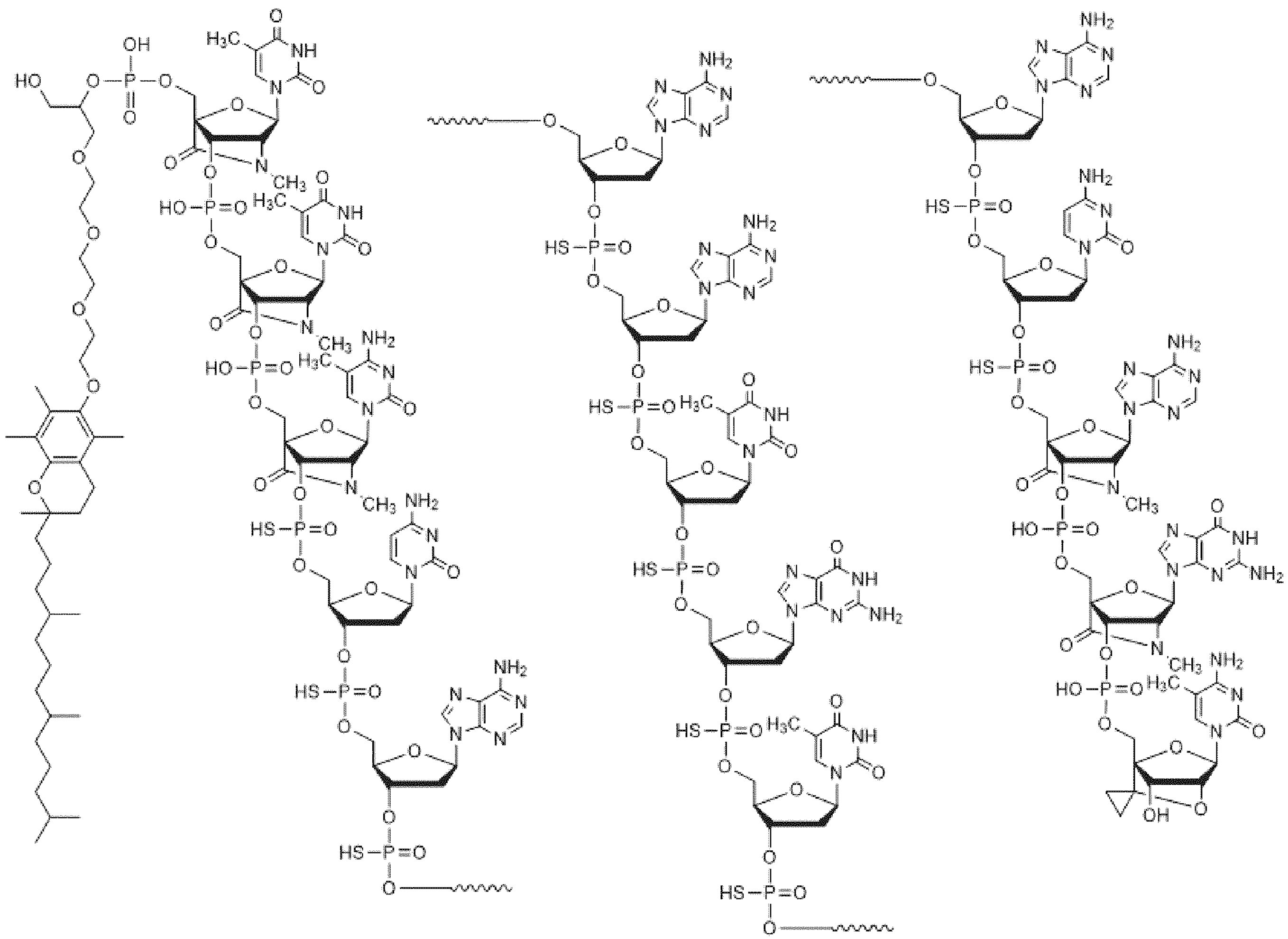

FIG.4

H451-465-J

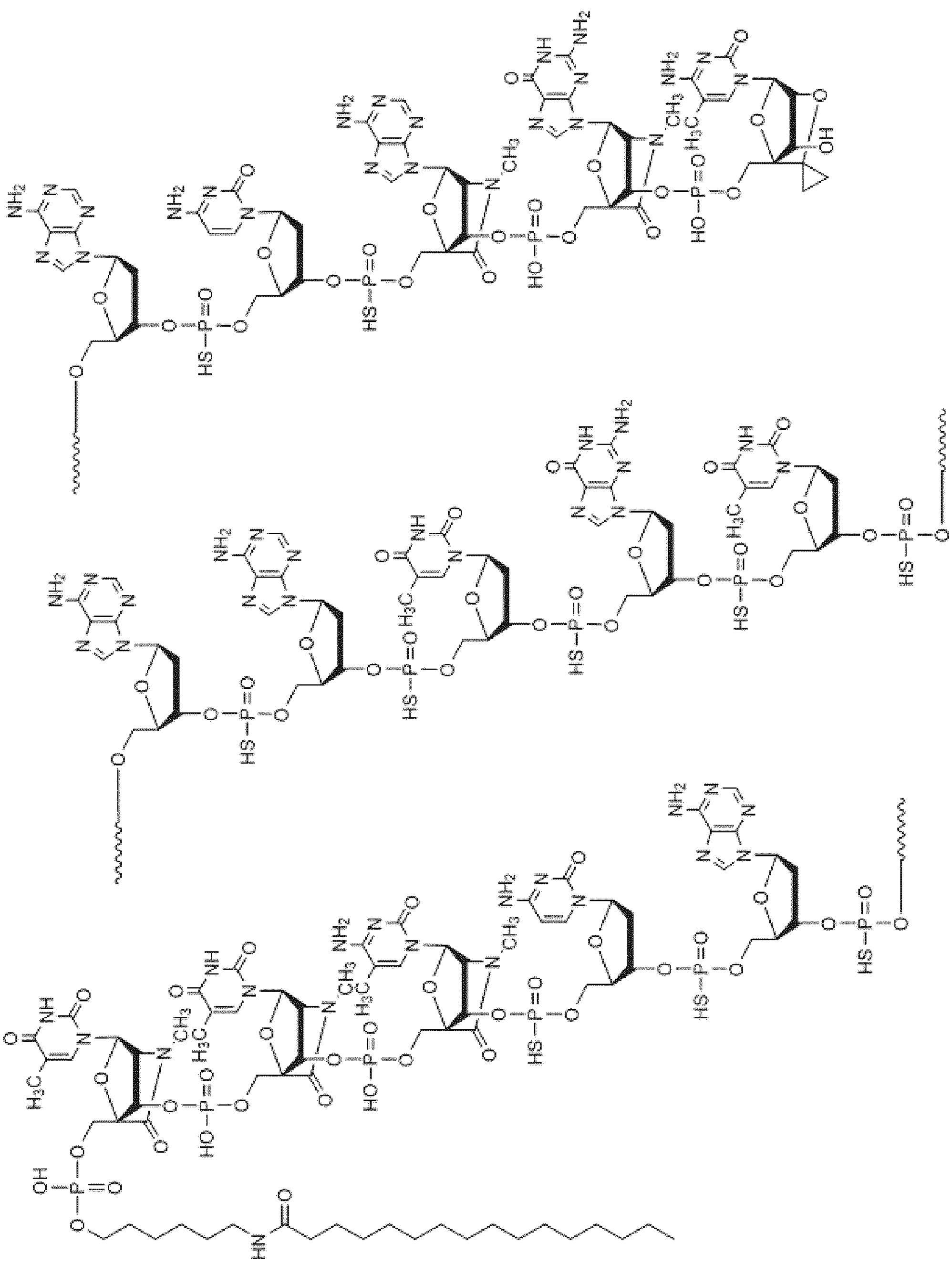

THEORETICAL MOLECULAR WEIGHT : 5471.5

STRUCTURAL FORMULA : $C_{183}H_{240}N_{61}O_{87}P_{15}S_{10}$

FIG.5

H451-465-K

THEORETICAL MOLECULAR WEIGHT: 6378.1

STRUCTURAL FORMULA: $C_{212}H_{277}N_{71}O_{105}P_{18}S_{10}$

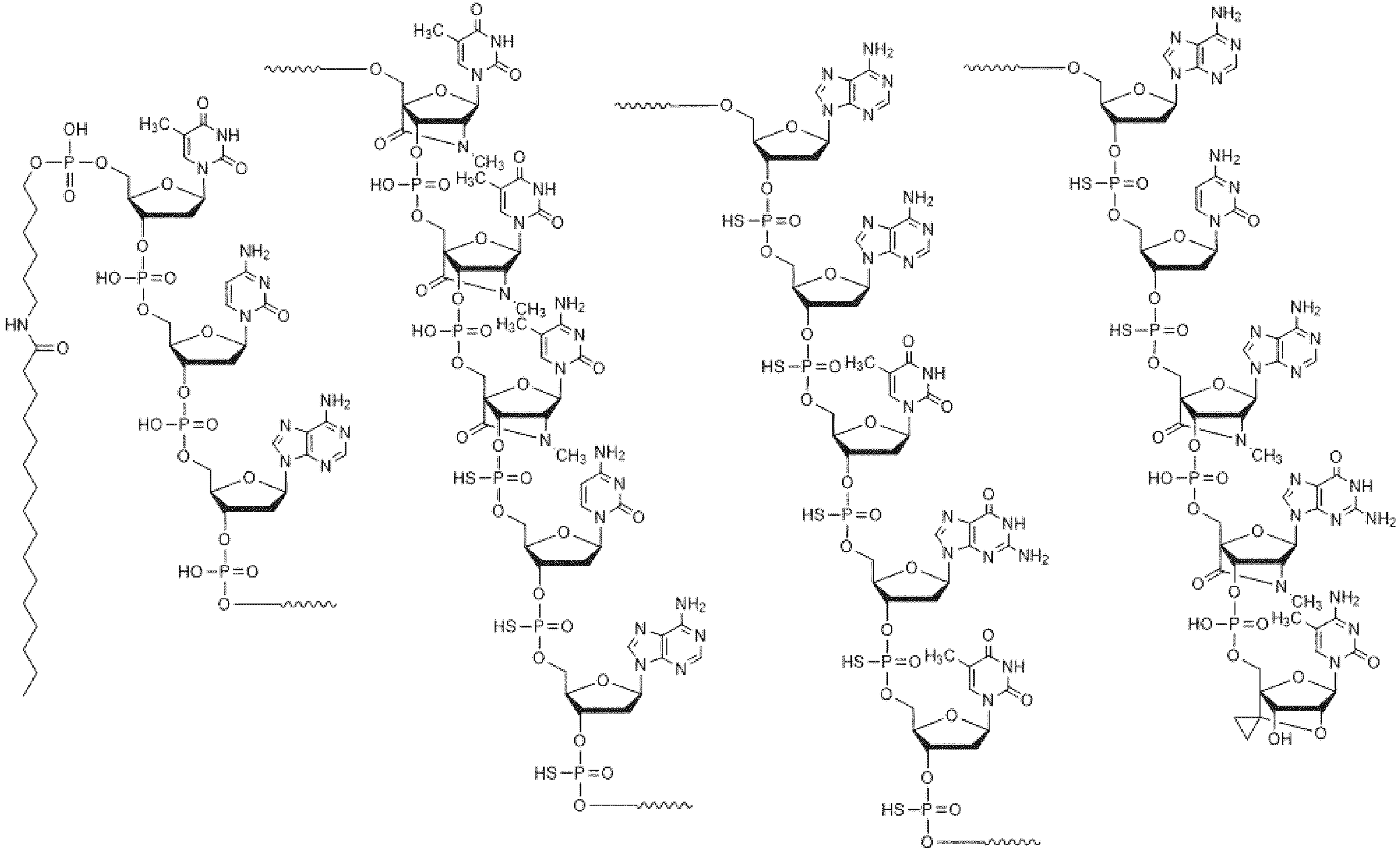

FIG.6

H451-465-L

THEORETICAL MOLECULAR WEIGHT : 5663.4

STRUCTURAL FORMULA: $C_{191}H_{235}Cl_2N_{62}O_{88}P_{15}S_{10}$

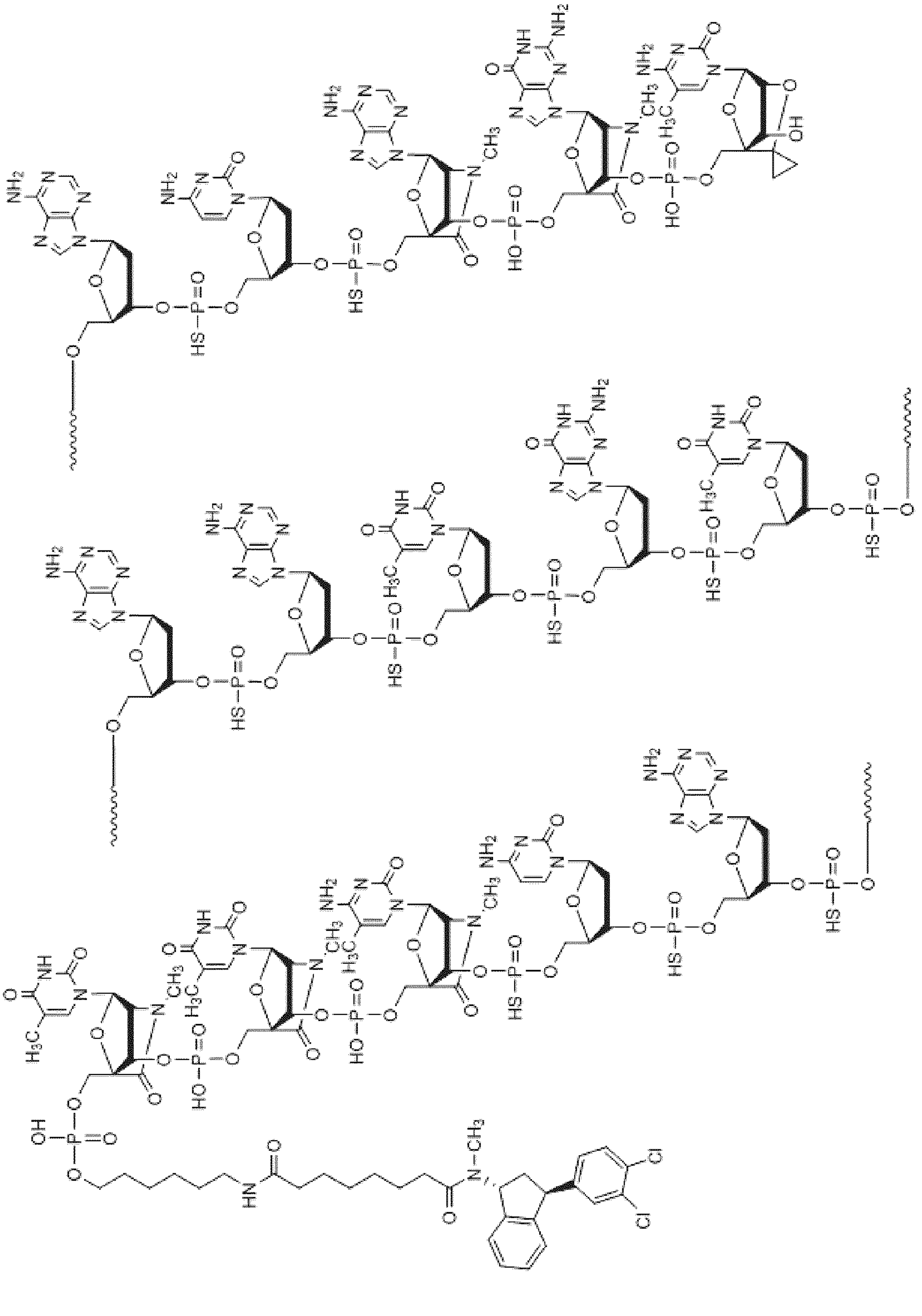

FIG.7

H451-465-M

THEORETICAL MOLECULAR WEIGHT : 5543.5

STRUCTURAL FORMULA : $C_{189}H_{240}N_{61}O_{87}P_{15}S_{10}$

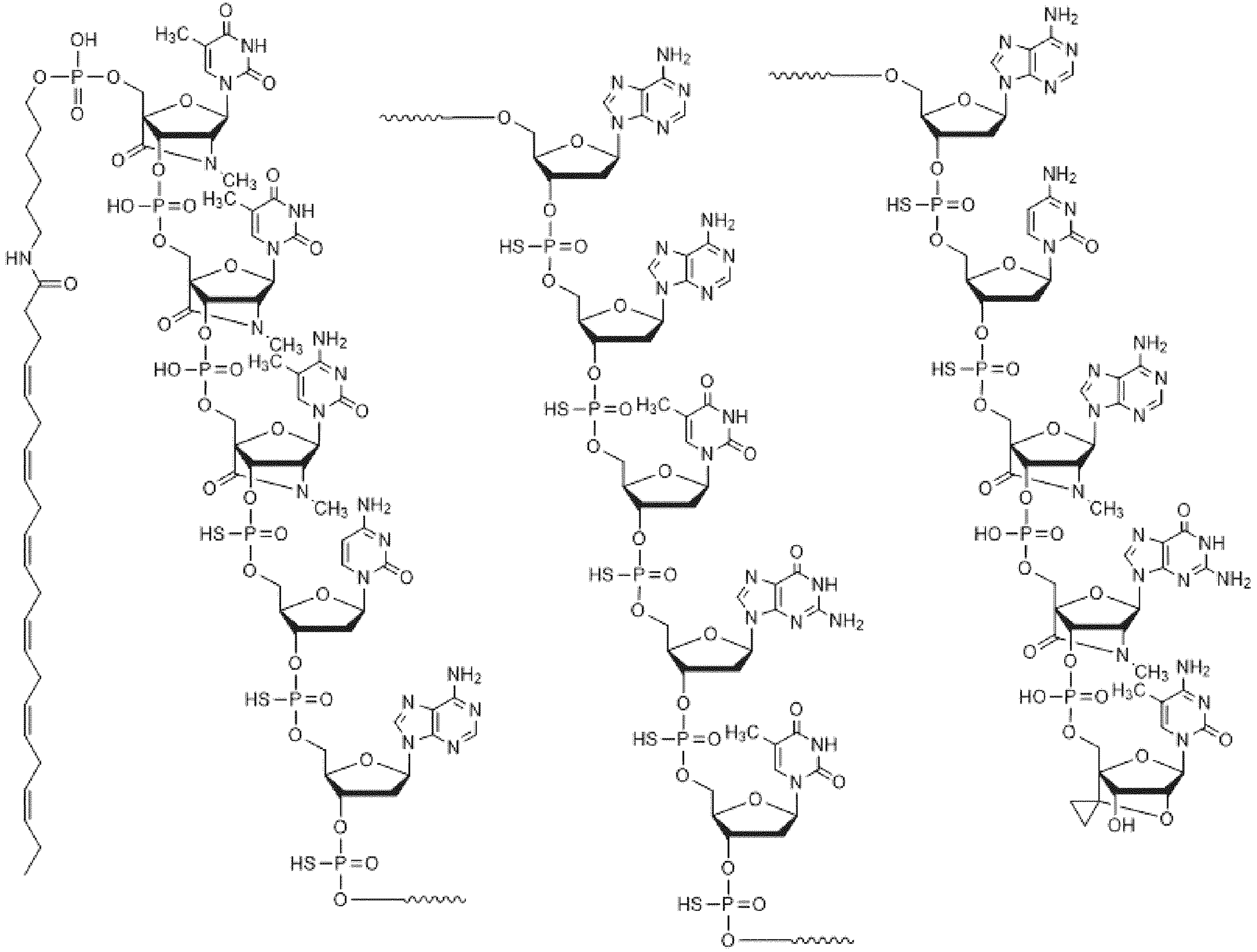

**INTERNATIONAL SEARCH REPORT**

International application No.
**PCT/JP2021/040853**

**A. CLASSIFICATION OF SUBJECT MATTER**

***A61P 21/04***(2006.01)i; ***A61P 25/00***(2006.01)i; ***A61P 43/00***(2006.01)i; ***C12N 15/113***(2010.01)i; ***A61K 31/712***(2006.01)i; ***A61K 31/7125***(2006.01)i; ***A61K 31/713***(2006.01)i
FI: C12N15/113 Z ZNA; A61K31/712; A61K31/7125; A61K31/713; A61P21/04; A61P25/00; A61P43/00 105

According to International Patent Classification (IPC) or to both national classification and IPC

**B. FIELDS SEARCHED**

Minimum documentation searched (classification system followed by classification symbols)

A61P21/04; A61P25/00; A61P43/00; C12N15/113; A61K31/712; A61K31/7125; A61K31/713

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | YAMADA, S. B. et al. RPS25 is required for efficient RAN translation of C9orf72 and other neurodegenerative disease-associated nucleotide repeats. Nature Neuroscience (Author manuscript). 2019, vol. 22, no. 9, pp. 1383-1388 (pp. 1-21), supplementary information<br>abstract, p. 4, third paragraph, pp. 12, 13, section of 7F iMN survival assay | 1-26 |
| Y | BC004986. Homo sapiens ribosomal protein S25, mRNA (cDNA clone MGC:4211 IMAGE:2905996). complete cds. [online], 2006, [retrieved 14 January 2022], Retrieved from the Internet: <URL:https://www.ncbi.nlm.nih.gov/nuccore/BC004986?report=GenBank><br>entire text | 1-26 |
| Y | 小比賀 聡 他, アンチセンス核酸医薬のデザイン戦略, 日本薬理学雑誌, 2016, vol. 148, pp. 100-104, (OBIKA, Satoshi et al. Folia Pharmacol. Jpn.), non-official translation (Antisense nucleic acid drug design strategy.)<br>section 1., 2., table 1, fig. 1 | 1-26 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
**PCT/JP2021/040853**

C. DOCUMENTS CONSIDERED TO BE RELEVANT

| Category* | Citation of document, with indication, where appropriate, of the relevant passages | Relevant to claim No. |
|---|---|---|
| Y | 實吉 尚郎 他, プロドラッグ開発を目的とする合成核酸の保護と修飾, Drug Delivery System. 2015, vol. 30, no. 5, pp. 465-472, (SANEYOSHI, Hisao et al. Protection and modification of synthetic oligonucleotides for the development of pro-drug type oligonucleotides.) section of 3., 7., 8., fig. 5 | 1-26 |
| Y | 松田 彰, 核酸医薬開発の現状と展望, ファルマシア, 2015, vol. 51, no. 5, pp. 429-433, (MATSUDA, Akira. Farumashia.), non-official translation (Current status and prospects of nucleic acid drug development.) section of 2, fig. 1 | 1-26 |
| Y | WO 2014/046212 A1 (OSAKA UNIV.) 27 March 2014 (2014-03-27) claims, examples | 1-26 |
| Y | YAMAGUCHI, T. et al., Synthesis and properties of 2'-O,4'-C-spirocyclopropylene bridged nucleic acid (scpBNA), an analogue of 2',4'-BNA/LNA bearing a cyclopropane ring, Chemical Communications, 2015, vol. 51, pp. 9737-9740 abstract, fig. 1 | 1-26 |

Form PCT/ISA/210 (second sheet) (January 2015)

**INTERNATIONAL SEARCH REPORT**

International application No.
**PCT/JP2021/040853**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/040853**

| Patent document cited in search report | Publication date (day/month/year) | Patent family member(s) | Publication date (day/month/year) |
|---|---|---|---|
| WO 2014/046212 A1 | 27 March 2014 | US 2015/0266917 A1<br>claims, examples<br>EP 2899197 A1<br>KR 10-2015-0056647 A<br>CN 104768964 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019084068 A
- WO 2011052436 A
- WO 2014046212 A
- WO 2015125783 A
- WO 2020158910 A
- WO 9914226 A


### Non-patent literature cited in the description


- *Science,* 2011, vol. 331 (6018), 730-736
- *Genes Dev,* 2009, vol. 23 (23), 2753-2764
- *Proc. Natl. Acad. Sci. USA,* 2011, vol. 108 (1), 260-265
- *Neuron,* 2015, vol. 88, 667-677
- *Neuron,* 2020, vol. 105, 645-662
- *Nat. Neurosci,* 2019, vol. 22 (9), 1383-1388
- *Mol. Gen. Genet,* 1979, vol. 169, 1-6
- *Curr. Opin. Struct. Biol,* 2014, vol. 24, 165-169
- *J. Med. Chem.,* 2016, vol. 59, 9645-9667
- *Nature,* 2015, vol. 518 (7539), 409-412
- *Mol. Gen. Genet.,* 1979, vol. 169, 1-6
- *Curr. Opin. Struct. Biol.,* 2014, vol. 24, 165-169
- **W. BRAD WAN.** *Al. J. Med. Chem.,* 2016, vol. 59, 9645-9667
- *Nature,* 2015, vol. 518 (7539), 409-12